(19) Europäisches Patentamt
European Patent Office
Office européen des brevets

(11) EP 1 833 972 B1

(12) **EUROPEAN PATENT SPECIFICATION**

(45) Date of publication and mention of the grant of the patent:
**13.10.2010 Bulletin 2010/41**

(21) Application number: **05823644.9**

(22) Date of filing: **23.12.2005**

(51) Int Cl.:
***C12N 15/82*** *(2006.01)* ***C12N 5/10*** *(2006.01)*
***C07K 14/415*** *(2006.01)*

(86) International application number:
**PCT/EP2005/057154**

(87) International publication number:
**WO 2006/067232 (29.06.2006 Gazette 2006/26)**

# (54) PLANTS HAVING INCREASED SEED YIELD AND METHOD FOR MAKING THE SAME

PFLANZEN MIT ERHÖHTEM SAATGUTERTRAG UND VERFAHREN ZUR ERZEUGUNG DAVON

PLANTES A RENDEMENT EN SEMENCES ET PROCEDE DE FABRICATION DESDITES PLANTES

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.12.2004 EP 04106984**
**06.01.2005 US 641657 P**

(43) Date of publication of application:
**19.09.2007 Bulletin 2007/38**

(73) Proprietor: **CropDesign N.V.**
**9052 Zwijnaarde-Gent (BE)**

(72) Inventor: **REUZEAU, Christophe**
**F-24350 La Chappelle Gonaguet (FR)**

(74) Representative: **Mistry, Meeta**
**BASF SE**
**Global Intellectual Property**
**GVX - C6**
**67056 Ludwigshafen (DE)**

(56) References cited:
**WO-A-00/04761** **WO-A-98/59039**
**WO-A-03/072763** **WO-A-03/085115**
**US-A1- 2003 044 972**

- **LOPEZ-VALENZUELA JOSE A ET AL: "Cytoskeletal proteins are coordinately increased in maize genotypes with high levels of eEF1A" PLANT PHYSIOLOGY (ROCKVILLE), vol. 135, no. 3, July 2004 (2004-07), pages 1784-1797, XP002330224 ISSN: 0032-0889**
- **BHADULA SHAILENDRA K ET AL: "Heat-stress induced synthesis of chloroplast protein synthesis elongation factor (EF-Tu) in a heat-tolerant maize line" February 2001 (2001-02), PLANTA (BERLIN), VOL. 212, NR. 3, PAGE(S) 359-366 , XP002330227 ISSN: 0032-0935 the whole document**
- **WANG XUELU ET AL: "Quantitative trait locus mapping of loci influencing elongation factor 1alpha content in maize endosperm" PLANT PHYSIOLOGY (ROCKVILLE), vol. 125, no. 3, March 2001 (2001-03), pages 1271-1282, XP002330225 ISSN: 0032-0889**
- **DATABASE EMBL [Online] EBI.CO.UK; 8 June 1992 (1992-06-08), "N. sylvestris tufB gene for chloroplast elongation factor TuB (EF-TuB), partial sequence." XP002330228 retrieved from EBI, HINXTON accession no. EBI.CO.UK Database accession no. D11376 -& MURAYAMA YUHKO ET AL: "Purification of chloroplast elongation factor Tu and cDNA analysis in tobacco: The existence of two chloroplast elongation factor Tu species" PLANT MOLECULAR BIOLOGY, vol. 22, no. 5, 1993, pages 767-774, XP002330226 ISSN: 0167-4412**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> CropDesign N.V.

<120> Plants having increased yield and method for making the same

<130> CD-127-PCT

<150> EP 04106984.0
<151> 2004-01-24

<150> US 60/641,657
<151> 2005-01-06

<160> 29

<170> PatentIn version 3.3

<210> 1
<211> 1860
<212> DNA
<213> Nicotiana tabacum

<400> 1

```
ccacgcgttc gggataacat cattatcctt ctctcctctt cttccttctt tcaaccacaa      60
ttctcactcc cctctttcgt ctctcttctc caacttcaat cccattttca ggcaaaaagc     120
tgtcatggct tcaatttcag cagcttctgc cacagctaca gcttctacaa agcttgcata     180
cccttattcc ccttcttcct caagcagcag cagcaacact gctgctgtat tcccttcaaa     240
ttcctcaaag cttatccttt cctcttcttt tacacccacc ccttcaaccc ttttcctcca     300
ctcaccaaca actactcctt ccaccaccca cccccgtcgg ttcactgtcc gcgctgcacg     360
tggcaaattc gagcgtaaaa aacctcacgt caacattggt acaattggcc acgttgacca     420
tggaaagacc acactcacag ctgctttgac catggcgctt gcctctatgg gcaactccgc     480
cccaagaaa tatgacgaaa ttgatgctgc cctgaagaa agggcgcgtg gtattactat     540
caacactgcc actgtggaat atgaaacgga aaacagacat tatgcacacg tggactgccc     600
ggggcatgct gattatgtca agaacatgat tactggtgct gcccaaatgg atggggcaat     660
tcttgttgtg tcaggtgctg atggcccaat gccacagact aaagagcata ttttgttagc     720
taagcaagtt ggggtcccta atatggttgt tttcttgaac aaacaagacc aagttgatga     780
tgaggagtta cttgagcttg ttgagttgga ggtaagagaa ttattgtcaa gttatgagtt     840
ccctggtgat gaaattccta ttatttctgg ttctgcactt ttagctttag aggctttgat     900
ggctaatcct agtattaaaa ggggtgaaaa tcaatgggtt gataagattt atcaattgat     960
ggataatgtt gatgaatata tccctatccc acaaagacaa actgaattgc ctttcttgat    1020
ggctattgag gatgttttct cgattaccgg tagaggtact gtggcgacgg ggagagtaga    1080
gagagggact gttaaggttg gggaaattgt tgatatagtt ggattgaagg atactaggaa    1140
tactacagtg acaggggttg agatgtttca gaagattttg gatgaagcga tggcgggaga    1200
taatgtggga ttgttgttga gaggtattca gaagattgat attcagagag ggatggtgtt    1260
ggcgaagccc ggaacaatta ctccgcacac aaagtttgaa gctttggtgt atgtgttgaa    1320
gaaggaagag ggaggaaggc attccccgtt ctttgcgggt tataggcctc aattttacat    1380
gaggacaact gatgtgactg gaaaggttac tgtgattatg agtgacaaag gagaggaatc    1440
taagatggtc atgcctggcg atcgtgtaaa catggtggtt gagcttatca tgccggttgc    1500
atgtgagcaa gggatgaggt ttgctatcag ggaaggagga aagactgttg gagctggtgt    1560
tattcagaaa atcttagaat gatgaacttg cagctgagca tctcttttca catgatcggc    1620
actttccatt gaagttactt aatccattgt catatatgca acttcttggt tacttttatt    1680
atgtcttaga atcttacttt agtagaagta tcctgtttta aacaccaaat tctactgaac    1740
ttttgggatt tttcctcagt ctcctctttc atttttcctt tgcttgaaag gaatgagaac    1800
atttgatttc atgcacttta tttaatttag aacaaatgtg cgactctgtt taaaattaag    1860
```

<210> 2
<211> 485
<212> PRT
<213> Nicotiana tabacum

<400> 2

```
Met Ala Ser Ile Ser Ala Ala Ser Ala Thr Ala Thr Ala Ser Thr Lys
1               5                   10                  15

Leu Ala Tyr Pro Tyr Ser Pro Ser Ser Ser Ser Ser Ser Ser Asn Thr
            20                  25                  30

Ala Ala Val Phe Pro Ser Asn Ser Ser Lys Leu Ile Leu Ser Ser Ser
        35                  40                  45

Phe Thr Pro Thr Pro Ser Thr Leu Phe Leu His Ser Pro Thr Thr Thr
    50                  55                  60

Pro Ser Thr Thr His Pro Arg Arg Phe Thr Val Arg Ala Ala Arg Gly
65                  70                  75                  80

Lys Phe Glu Arg Lys Lys Pro His Val Asn Ile Gly Thr Ile Gly His
                85                  90                  95

Val Asp His Gly Lys Thr Thr Leu Thr Ala Ala Leu Thr Met Ala Leu
            100                 105                 110

Ala Ser Met Gly Asn Ser Ala Pro Lys Lys Tyr Asp Glu Ile Asp Ala
        115                 120                 125

Ala Pro Glu Glu Arg Ala Arg Gly Ile Thr Ile Asn Thr Ala Thr Val
    130                 135                 140

Glu Tyr Glu Thr Glu Asn Arg His Tyr Ala His Val Asp Cys Pro Gly
145                 150                 155                 160

His Ala Asp Tyr Val Lys Asn Met Ile Thr Gly Ala Ala Gln Met Asp
                165                 170                 175

Gly Ala Ile Leu Val Val Ser Gly Ala Asp Gly Pro Met Pro Gln Thr
            180                 185                 190

Lys Glu His Ile Leu Leu Ala Lys Gln Val Gly Val Pro Asn Met Val
        195                 200                 205

Val Phe Leu Asn Lys Gln Asp Gln Val Asp Asp Glu Glu Leu Leu Glu
    210                 215                 220

Leu Val Glu Leu Glu Val Arg Glu Leu Leu Ser Ser Tyr Glu Phe Pro
225                 230                 235                 240

Gly Asp Glu Ile Pro Ile Ile Ser Gly Ser Ala Leu Leu Ala Leu Glu
                245                 250                 255

Ala Leu Met Ala Asn Pro Ser Ile Lys Arg Gly Glu Asn Gln Trp Val
            260                 265                 270

Asp Lys Ile Tyr Gln Leu Met Asp Asn Val Asp Glu Tyr Ile Pro Ile
        275                 280                 285

Pro Gln Arg Gln Thr Glu Leu Pro Phe Leu Met Ala Ile Glu Asp Val
    290                 295                 300
```

```
Phe Ser Ile Thr Gly Arg Gly Thr Val Ala Thr Gly Arg Val Glu Arg
305                 310                 315                 320

Gly Thr Val Lys Val Gly Glu Ile Val Asp Ile Val Gly Leu Lys Asp
                325                 330                 335

Thr Arg Asn Thr Thr Val Thr Gly Val Glu Met Phe Gln Lys Ile Leu
            340                 345                 350

Asp Glu Ala Met Ala Gly Asp Asn Val Gly Leu Leu Leu Arg Gly Ile
        355                 360                 365

Gln Lys Ile Asp Ile Gln Arg Gly Met Val Leu Ala Lys Pro Gly Thr
    370                 375                 380

Ile Thr Pro His Thr Lys Phe Glu Ala Leu Val Tyr Val Leu Lys Lys
385                 390                 395                 400

Glu Glu Gly Gly Arg His Ser Pro Phe Phe Ala Gly Tyr Arg Pro Gln
                405                 410                 415

Phe Tyr Met Arg Thr Thr Asp Val Thr Gly Lys Val Thr Val Ile Met
            420                 425                 430

Ser Asp Lys Gly Glu Glu Ser Lys Met Val Met Pro Gly Asp Arg Val
        435                 440                 445

Asn Met Val Val Glu Leu Ile Met Pro Val Ala Cys Glu Gln Gly Met
    450                 455                 460

Arg Phe Ala Ile Arg Glu Gly Gly Lys Thr Val Gly Ala Gly Val Ile
465                 470                 475                 480

Gln Lys Ile Leu Glu
                485
```

<210> 3
<211> 2342
<212> DNA
<213> Nicotiana tabacum

<400> 3

```
gaattcttat aatttaacgt aaaccaaata attaaaaaat cgatcttcat attgcagaaa      60
aacagggatt ttatatatgt agttttaaaa aaagaaaaag aaaaagaaaa aatgtttccc     120
tgcccctaat tcccaagagg aaagttgttg ccgcagcgta gtgttatcca aataggtaga     180
aatggataga aaaatccccc caaaatgagt atatgagcgg ccctattttt tccttccaag     240
ggatataaca ctattatccc cttctcctca ttttctttca aatcccaatt ctcactcgct     300
ctatccttaa ctctcttttc tctcccaact tcaatctccg gccatggctt caatttcagc     360
agccaccgcc acctcctcca ctaagcttgt atcatccaac tccaccaatc ctcttcttcc     420
ttcctccact aaaccctcta agcttatcct atcctcttcc tttaccccta attgttccac     480
ccttttcctc cactcacccg ccaccccttc ttctactgcc acccatcgcc accgccggtt     540
tactgtccgc gctgcccgtg gcaaattcga gcggaaaaaa ccccatgtca acatcggtac     600
tattggccat gttgaccacg gaaagactac tctcaccgct gctttaacca tggctcttgc     660
ttctatggga aactccgccc ctaagaaata cgacgaaatt gatgccgccc cagaagagcg     720
tgctcgtggt attactatta acactgctac tgttgagtac gagactgaga accgtcacta     780
cgcccacgtg gattgccctg gtcacgctga ttatgtcaag aatatgatta ccggtgctgc     840
ccaaatggac ggcgctattc ttgtttgttc aggtgctgat ggtcccatgc cacagactaa     900
ggaacacatt ttgcttgcta agcaagtagg tgtccccaac atggttgtgt tcttgaacaa     960

acaagatcag gttgatgatg aggagctgct tcagcttgtt gagttggagg taagagagtt    1020
attgtcaagt tatgagtttc ctggtgatga tattcctatt atttctggct ctgctctttt    1080
ggctttagag gctttgatgg ctaatcctag tattaagaga ggtgaaaatc aatgggttga    1140
taagatatat gagttgatgg acgctgttga tagttatatt cctattccag ttaggcaaac    1200
tgaattgcct ttcttgatgg ctattgaaga tgtgttttcc attactggta gaggtactgt    1260
ggcgacgggg agggtagaga gagggactgt taggattgga gacactgttg atattgtagg    1320
tttgaaggac actaggagta ctaccgtgac gggtgttgag atgtttcaaa agattttgga    1380
tgaagcaatg gctggagaca atgtggggtt gttgttgaga ggtattcaaa agattgatat    1440
tcagagagga atggtgttgg caaaacccgg aacaattacc cctcacacca agtttgaagc    1500
tattgtgtat gtgttgaaga aggaggaggg tggtaggcat tccccccttct tttcagggta    1560
caggcctcag ttttacatga ggactactga tgtgaccgga aaggttactt ccattacgac    1620
tgataaagga gaggaatcta agatggtcat gcctggtgat cgtgtgaact tggtggttga    1680
gctcattatg ccggtggctt gtgagcaagg gatgagattt gccatcaggg aaggaggaaa    1740
gactgttgga gctggtgtca ttcagaaaat tatcgagtga tcaaccaata gcggagcaat    1800
ttcccttcaa atggtctggt ccctgtcaca cagtattttt aatccattgt catatatgca    1860
gctttttggt tacttttttc ttatctttta gaatcctagt tcagttggag aaatcttgtt    1920
ataagtcaat cctagtggac ctttgcgagt ttgcttcagt ttcgtgcttt gttttttcat    1980
ttgtttgcaa ggatggagaa cattttgtct cttgtgcatc ttactttctg ctagaacaaa    2040
ttcatattgt gtttgaaact cgagtttaac atgctaatga aattaggtgc atttgatatt    2100
tgttttgacg aattgatgct gctaggaaac acccaaaaaa aagattactg agatgttttc    2160
tttaccagca tatgtcgttt aaaaactaaa attgatggac atgagctgac cctgtaagga    2220
aaatattgta acagaacctc ataagactta agtttagtga tgcttaattg ttattctcaa    2280
ttctggcttt aatccctgag tagaactatc tgaaatgatc agttggttct gtcttggaat    2340
tc                                                                   2342
```

<210> 4
<211> 478
<212> PRT
<213> Nicotiana tabacum

<400> 4

```
Met Ala Ser Ile Ser Ala Ala Thr Ala Thr Ser Ser Thr Lys Leu Val
1               5                   10                  15

Ser Ser Asn Ser Thr Asn Pro Leu Leu Pro Ser Ser Thr Lys Pro Ser
            20                  25                  30

Lys Leu Ile Leu Ser Ser Ser Phe Thr Pro Asn Cys Ser Thr Leu Phe
        35                  40                  45

Leu His Ser Pro Ala Thr Pro Ser Ser Thr Ala Thr His Arg His Arg
    50                  55                  60

Arg Phe Thr Val Arg Ala Ala Arg Gly Lys Phe Glu Arg Lys Lys Pro
65                  70                  75                  80

His Val Asn Ile Gly Thr Ile Gly His Val Asp His Gly Lys Thr Thr
                85                  90                  95

Leu Thr Ala Ala Leu Thr Met Ala Leu Ala Ser Met Gly Asn Ser Ala
            100                 105                 110

Pro Lys Lys Tyr Asp Glu Ile Asp Ala Ala Pro Glu Glu Arg Ala Arg
        115                 120                 125

Gly Ile Thr Ile Asn Thr Ala Thr Val Glu Tyr Glu Thr Glu Asn Arg
    130                 135                 140

His Tyr Ala His Val Asp Cys Pro Gly His Ala Asp Tyr Val Lys Asn
```

```
145                 150                 155                 160

Met Ile Thr Gly Ala Ala Gln Met Asp Gly Ala Ile Leu Val Cys Ser
                165                 170                 175

Gly Ala Asp Gly Pro Met Pro Gln Thr Lys Glu His Ile Leu Leu Ala
            180                 185                 190

Lys Gln Val Gly Val Pro Asn Met Val Val Phe Leu Asn Lys Gln Asp
        195                 200                 205

Gln Val Asp Asp Glu Glu Leu Leu Gln Leu Val Glu Leu Glu Val Arg
    210                 215                 220

Glu Leu Leu Ser Ser Tyr Glu Phe Pro Gly Asp Asp Ile Pro Ile Ile
225                 230                 235                 240

Ser Gly Ser Ala Leu Leu Ala Leu Glu Ala Leu Met Ala Asn Pro Ser
                245                 250                 255

Ile Lys Arg Gly Glu Asn Gln Trp Val Asp Lys Ile Tyr Glu Leu Met
            260                 265                 270

Asp Ala Val Asp Ser Tyr Ile Pro Ile Pro Val Arg Gln Thr Glu Leu
        275                 280                 285

Pro Phe Leu Met Ala Ile Glu Asp Val Phe Ser Ile Thr Gly Arg Gly
    290                 295                 300

Thr Val Ala Thr Gly Arg Val Glu Arg Gly Thr Val Arg Ile Gly Asp
305                 310                 315                 320

Thr Val Asp Ile Val Gly Leu Lys Asp Thr Arg Ser Thr Thr Val Thr
                325                 330                 335

Gly Val Glu Met Phe Gln Lys Ile Leu Asp Glu Ala Met Ala Gly Asp
            340                 345                 350

Asn Val Gly Leu Leu Leu Arg Gly Ile Gln Lys Ile Asp Ile Gln Arg
        355                 360                 365

Gly Met Val Leu Ala Lys Pro Gly Thr Ile Thr Pro His Thr Lys Phe
    370                 375                 380

Glu Ala Ile Val Tyr Val Leu Lys Lys Glu Glu Gly Gly Arg His Ser
385                 390                 395                 400

Pro Phe Phe Ser Gly Tyr Arg Pro Gln Phe Tyr Met Arg Thr Thr Asp
                405                 410                 415

Val Thr Gly Lys Val Thr Ser Ile Thr Thr Asp Lys Gly Glu Glu Ser
            420                 425                 430

Lys Met Val Met Pro Gly Asp Arg Val Asn Leu Val Val Glu Leu Ile
        435                 440                 445

Met Pro Val Ala Cys Glu Gln Gly Met Arg Phe Ala Ile Arg Glu Gly
    450                 455                 460
```

Gly Lys Thr Val Gly Ala Gly Val Ile Gln Lys Ile Ile Glu
465 470 475

<210> 5
<211> 1758
<212> DNA
<213> Arabidopsis thaliana

<400> 5

```
atccccaaat cttctccttc tccaaaaaaa tttcaaaccc tagcttctcg atttctctcc      60
tctgctctcc aattccatct tcccatggcg atttcggctc cagccgcttg ttcttcctcc     120
tctagaattc tctgttccta ctcctctcct tctccttctc tctgtcccgc catttccacc     180
tctggtaaac tcaaaaccct aactctctct tcctccttcc tcccttctta ctcattaacc     240
accacctccg cttctcaatc cactcgtcgc tccttcaccg tccgcgccgc tcgtggaaag     300
ttcgagagga agaagcctca tgtcaacatc ggaaccatcg gtcatgttga ccatgggaaa     360
actactttaa ccgcagctct aaccatggct ctcgcttcca ttggttccag cgtcgctaaa     420
aagtacgacg agattgacgc tgcgccggag gagagagctc gtggtatcac aatcaacact     480
gctactgttg agtacgagac tgagaatcgt cactacgctc acgttgattg tcctggtcac     540
gctgattacg ttaagaatat gattaccgga gctgcacaga tggacggagc tatcctcgtt     600
gtttccggcg ccgatggtcc tatgcctcag actaaagagc atatcctttt ggctaagcag     660
gttggtgttc ctgatatggt tgtgtttctt aacaaagagg atcaagtaga tgatgcagag     720
ttgctagagc tcgttgagct tgaggttcgt gagcttctct cgtcttatga atttaacggt     780
gatgatattc cgattatctc tggttctgct cttttagccg ttgagactct tactgagaat     840
cctaaggtta agagaggtga taacaaatgg gtagataaga tttatgaact tatggatgct     900
gttgatgatt acatccctat ccctcagaga caaactgaat tgccattctt gttagctgtt     960
gaggatgtgt tctctatcac tggacgtggt acggtggcta cagggcgtgt cgagagaggt    1020
acggttaagg taggagagac tgtagattta gtgggtttga gggagactag gagttacact    1080
gtcactgggg ttgaaatgtt tcagaagatt cttgatgagg ctttagctgg tgacaatgta    1140
gggttgttgc ttaggggtat tcaaaaggct gatattcaga gaggtatggt tttagctaag    1200
ccgggatcga ttactccaca taccaagttt gaagcaatta tctatgtgtt gaagaaagag    1260
gaaggtggaa ggcattctcc attctttgca gggtacaggc ctcagttcta catgaggacg    1320
actgatgtta cgggtaaagt gacgaagatc atgaacgaca aagatgaaga gtcgaagatg    1380
gttatgcccg gtgatcgagt gaagattgtt gttgagctta ttgtgccggt ggcttgtgaa    1440
caagggatga ggtttgctat cagagaagga ggaaagactg ttggtgctgg agttattggg    1500
acgatcctcg aatgattata aggttttaga gagtttcata tagtgagcat ctctacttgc    1560
tttctttttg tttcactctt gattctcaaa ctctttgagt tttacctttt ttttatttat    1620
ttttgtgcca ttttccataa gaacttgaaa tcaaaatgtc atatatttct tgattttctg    1680
atccttgtta ttcatctttg tctcgctcca tatgattctg atgtggattt ggtactcaac    1740
gaccaatcac ttaatttg                                                  1758
```

<210> 6
<211> 476
<212> PRT
<213> Arabidopsis thaliana

<400> 6

```
Met Ala Ile Ser Ala Pro Ala Ala Cys Ser Ser Ser Ser Arg Ile Leu
1               5                   10                  15

Cys Ser Tyr Ser Ser Pro Ser Pro Ser Leu Cys Pro Ala Ile Ser Thr
            20                  25                  30

Ser Gly Lys Leu Lys Thr Leu Thr Leu Ser Ser Ser Phe Leu Pro Ser
        35                  40                  45

Tyr Ser Leu Thr Thr Thr Ser Ala Ser Gln Ser Thr Arg Arg Ser Phe
    50                  55                  60

Thr Val Arg Ala Ala Arg Gly Lys Phe Glu Arg Lys Lys Pro His Val
```

```
65                  70                  75                  80

Asn Ile Gly Thr Ile Gly His Val Asp His Gly Lys Thr Thr Leu Thr
                85                  90                  95

Ala Ala Leu Thr Met Ala Leu Ala Ser Ile Gly Ser Ser Val Ala Lys
            100                 105                 110

Lys Tyr Asp Glu Ile Asp Ala Ala Pro Glu Glu Arg Ala Arg Gly Ile
        115                 120                 125

Thr Ile Asn Thr Ala Thr Val Glu Tyr Glu Thr Glu Asn Arg His Tyr
    130                 135                 140

Ala His Val Asp Cys Pro Gly His Ala Asp Tyr Val Lys Asn Met Ile
145                 150                 155                 160

Thr Gly Ala Ala Gln Met Asp Gly Ala Ile Leu Val Val Ser Gly Ala
                165                 170                 175

Asp Gly Pro Met Pro Gln Thr Lys Glu His Ile Leu Leu Ala Lys Gln
            180                 185                 190

Val Gly Val Pro Asp Met Val Val Phe Leu Asn Lys Glu Asp Gln Val
        195                 200                 205

Asp Asp Ala Glu Leu Leu Glu Leu Val Glu Leu Glu Val Arg Glu Leu
    210                 215                 220

Leu Ser Ser Tyr Glu Phe Asn Gly Asp Asp Ile Pro Ile Ile Ser Gly
225                 230                 235                 240

Ser Ala Leu Leu Ala Val Glu Thr Leu Thr Glu Asn Pro Lys Val Lys
                245                 250                 255

Arg Gly Asp Asn Lys Trp Val Asp Lys Ile Tyr Glu Leu Met Asp Ala
            260                 265                 270

Val Asp Asp Tyr Ile Pro Ile Pro Gln Arg Gln Thr Glu Leu Pro Phe
        275                 280                 285

Leu Leu Ala Val Glu Asp Val Phe Ser Ile Thr Gly Arg Gly Thr Val
    290                 295                 300

Ala Thr Gly Arg Val Glu Arg Gly Thr Val Lys Val Gly Glu Thr Val
305                 310                 315                 320

Asp Leu Val Gly Leu Arg Glu Thr Arg Ser Tyr Thr Val Thr Gly Val
                325                 330                 335

Glu Met Phe Gln Lys Ile Leu Asp Glu Ala Leu Ala Gly Asp Asn Val
            340                 345                 350

Gly Leu Leu Leu Arg Gly Ile Gln Lys Ala Asp Ile Gln Arg Gly Met
        355                 360                 365

Val Leu Ala Lys Pro Gly Ser Ile Thr Pro His Thr Lys Phe Glu Ala
    370                 375                 380
```

```
Ile Ile Tyr Val Leu Lys Lys Glu Glu Gly Gly Arg His Ser Pro Phe
385                 390                 395                 400

Phe Ala Gly Tyr Arg Pro Gln Phe Tyr Met Arg Thr Thr Asp Val Thr
                405                 410                 415

Gly Lys Val Thr Lys Ile Met Asn Asp Lys Asp Glu Glu Ser Lys Met
            420                 425                 430

Val Met Pro Gly Asp Arg Val Lys Ile Val Val Glu Leu Ile Val Pro
        435                 440                 445

Val Ala Cys Glu Gln Gly Met Arg Phe Ala Ile Arg Glu Gly Gly Lys
    450                 455                 460

Thr Val Gly Ala Gly Val Ile Gly Thr Ile Leu Glu
465                 470                 475
```

<210> 7
<211> 3675
<212> DNA
<213> Nicotiana sylvestris

<400> 7

```
tgatcattct gcggatttta gttatgcaag tgggcctaat tcatcagagg aaagaaataa     60
catgatcagc aacaactacc cagctgatgt cagacaaatt gtcaagaaac tcgaggagct    120
gcttggtcag cagcaaaagg agctaaatga tctcaggaaa aaacatgact cggctatatc    180
agatattcta agcaagcttc ctcctgagat ccgtggcatt tatggtttaa aaatatcttc    240
caataatttg tagtggggat gaggctgcta ttctatggat cctgaagact gaagtttctc    300
tgtgaagatg taagttactg tgcggccttt tgccactcaa ctgcagatag tcttggattg    360
caccaattat ctgactaccc ttgaaaggtt tcaagatatt cgagaatagc tgaaagatta    420
attttgccga caatggtcac atgcagatca aaagctagaa gggaaaagca tggacctatc    480
attagataca gattcacttc tgtcaaggat accatcattt cagggccagg aattacagta    540
atacaggttt ctcattacac gcatattgtg cgtctgcaag tcatgttgct gctaaaaatg    600
agtcatggca tgtatacttg tacatattta catgagtatt taactattgg aaataccaat    660
tggttgaaat aatgccagtc aatatataac ttcttaagtt tctttaattt tatcttggtg    720
ttcactagat ccgtaaaaaa ggaaaacaat atatctgaat gattctacta tttgctgttg    780
tccggttttt cttcacaaag tatgtttttt catatgtgct tataaacgcc cccgtttttt    840
ttaaaactct gcttttacct atcctaaagc tctttataag ctccatacag atcattggtg    900
taaactcacg gtttaaactt ttgtgtatac cctattgtcc tggcactagt caatcctttt    960
tactttagta tacatactag tatttgacat gaacattttt ctatagcaca cgttaccaga   1020
tttttcttgg cactctattg tatacatccc actccatgtt aatttttacc aatctttata   1080
gcaaatatac agccattgtt gcagatttgg catgtacaac catgaggggc ggagctagaa   1140
tattccgagc gggttcggcc aaatccagaa cgactagaat tccgaaccca taagcttgaa   1200
atccttgcta tgcctgtgat aatcattaaa ttaaacacta ccgggagaaa aagaaggctt   1260
tggaacaaaa agatttttat ttatttatgt gaagttaaaa gaaaaggcag ggtaaaaatt   1320
tagactattt tcttacgtgt cacattttgg gaacagaatt cacagaaatc ccatacccct   1380
aatccccaca gttgcaagtt gcagcccagt gtgtcatcct agggaggtaa gtattagata   1440
aaaccccaca tatatgtgag gccctcttta ttcctaccaa ggataacatc attatccttc   1500
tctcctcttc ttccttcttt caaccacaat tctcactccc ctctttcgtc tctcttctcc   1560
aacttcaatc ccattttcag gcaaaaagct gtcatggctt caatttcagc agcttctgcc   1620
acagctacag cttctacaaa gcttgcatac ccttattccc cttcttcctc aagcagcagc   1680
agcaacactg ctgctgtatt cccttcaaat tcctcaaagc ttatcctttc ctcttctttt   1740
acacccaccc cttcaaccct tttcctccac tcaccaacaa ctactccttc caccacccac   1800
ccccgtcggt tcactgtccg cgctgcacgt ggcaaattcg agcgtaaaaa acctcacgtc   1860
aacattggta caattggcca cgttgaccat ggaaagacca cactcacagc tgctttgacc   1920
atggcgcttg cctctatggg caactccgcc cccaagaaat atgacgaaat tgatgctgcc   1980
cctgaagaaa gggcgcgtgg tattactatc aacactgcca ctgtggaata tgaaacggaa   2040
aacagacatt atgcacacgt ggactgcccg gggcatgctg attatgtcaa gaacatgatt   2100
```

```
actggtgctg cccaaatgga tggggcaatt cttgttgtgt caggtgctga tggcccaatg   2160
ccacagacta aagagcatat tttgttagct aagcaagttg gggtccctaa tatggttgtt   2220
ttcttgaaca aacaagacca agttgatgat gaggagttac ttgagcttgt tgagttggag   2280
gtaagagaat tattgtcaag ttatgagttc cctggtgatg aaattcctat tatttctggt   2340
tctgcacttt tagctttaga ggctttgatg gctaatccta gtattaaaag gggtgaaaat   2400
caatgggttg ataagattta tcaattgatg gataatgttg atgaatatat ccctatccca   2460
caaagacaaa ctgaattgcc tttcttgatg gctattgagg atgttttctc gattaccggt   2520
agaggtactg tggcgacggg gagagtagag agagggactg ttaaggttgg ggaaattgtt   2580
gatatagttg gattgaagga tactaggaat actacagtga caggggttga gatgtttcag   2640
aagattttgg atgaagcgat ggcgggagat aatgtgggat tgttgttgag aggtattcag   2700
aagattgata ttcagagagg gatggtgttg gcgaagcccg gaacaattac tccgcacaca   2760
aagtttgaag ctttggtgta tgtgttgaag aaggaagagg gaggaaggca ttccccgttc   2820
tttgcgggtt ataggcctca attttacatg aggacaactg atgtgactgg aaaggttact   2880
gtgattatga gtgacaaagg agaggaatct aagatggtca tgcctggcga tcgtgtaaac   2940
atggtggttg agcttatcat gccggttgca tgtgagcaag ggatgaggtt tgctatcagg   3000
gaaggaggaa agactgttgg agctggtgtt attcagaaaa tcttagaatg atgaacttgc   3060
agctgagcat ctcttttcac atgatcggca ctttccattg aagttactta atccattgtc   3120
atatatgcaa cttcttggtt acttttatta tgtcttagaa tcttacttta gtagaagtat   3180
cctgttttaa acaccaaatt ctactgaact tttgggattt ttcggcagtc tcctctttca   3240
tttttccttt gcttgaaagg aatagaacat ttgatttcat gcactttatt taatttagaa   3300
caaatgtgcg actctgtttg aaaatttaag tgcagatttg ctaatttgag attcactgct   3360
tcggcttttt gtttcagcaa aatgatgctg atagaaaagc acaaaagaat aaataatttg   3420
tttgtaaact atggataata atttgtcagc tggccaatga ctgaattttt tatgtccatg   3480
taactaaaaa ctatggtact cgaagcagat atgtatttat ctgtattgtt taaaatcgac   3540
aagaggggtt gctctgatgg taagcaaccc ccacttccaa ctaagaggct gtgagttcaa   3600
gtttccccaa gagcaaggtg ggaagttctt ggagggaagg atgccgaggg tctaattgga   3660
aacagccttt ctacc                                                    3675
```

<210> 8
<211> 485
<212> PRT
<213> Nicotiana sylvestris

<400> 8

```
Met Ala Ser Ile Ser Ala Ala Ser Ala Thr Ala Thr Ala Ser Thr Lys
1               5                   10                  15

Leu Ala Tyr Pro Tyr Ser Pro Ser Ser Ser Ser Ser Ser Ser Asn Thr
            20                  25                  30

Ala Ala Val Phe Pro Ser Asn Ser Ser Lys Leu Ile Leu Ser Ser Ser
        35                  40                  45

Phe Thr Pro Thr Pro Ser Thr Leu Phe Leu His Ser Pro Thr Thr Thr
    50                  55                  60

Pro Ser Thr Thr His Pro Arg Arg Phe Thr Val Arg Ala Ala Arg Gly
65                  70                  75                  80

Lys Phe Glu Arg Lys Lys Pro His Val Asn Ile Gly Thr Ile Gly His
                85                  90                  95

Val Asp His Gly Lys Thr Thr Leu Thr Ala Ala Leu Thr Met Ala Leu
            100                 105                 110

Ala Ser Met Gly Asn Ser Ala Pro Lys Lys Tyr Asp Glu Ile Asp Ala
        115                 120                 125

Ala Pro Glu Glu Arg Ala Arg Gly Ile Thr Ile Asn Thr Ala Thr Val
```

```
        130                 135                 140

Glu Tyr Glu Thr Glu Asn Arg His Tyr Ala His Val Asp Cys Pro Gly
145                 150                 155                 160

His Ala Asp Tyr Val Lys Asn Met Ile Thr Gly Ala Ala Gln Met Asp
                165                 170                 175

Gly Ala Ile Leu Val Val Ser Gly Ala Asp Gly Pro Met Pro Gln Thr
            180                 185                 190

Lys Glu His Ile Leu Leu Ala Lys Gln Val Gly Val Pro Asn Met Val
        195                 200                 205

Val Phe Leu Asn Lys Gln Asp Gln Val Asp Asp Glu Glu Leu Leu Glu
    210                 215                 220

Leu Val Glu Leu Glu Val Arg Glu Leu Leu Ser Ser Tyr Glu Phe Pro
225                 230                 235                 240

Gly Asp Glu Ile Pro Ile Ile Ser Gly Ser Ala Leu Leu Ala Leu Glu
                245                 250                 255

Ala Leu Met Ala Asn Pro Ser Ile Lys Arg Gly Glu Asn Gln Trp Val
            260                 265                 270

Asp Lys Ile Tyr Gln Leu Met Asp Asn Val Asp Glu Tyr Ile Pro Ile
        275                 280                 285

Pro Gln Arg Gln Thr Glu Leu Pro Phe Leu Met Ala Ile Glu Asp Val
    290                 295                 300

Phe Ser Ile Thr Gly Arg Gly Thr Val Ala Thr Gly Arg Val Glu Arg
305                 310                 315                 320

Gly Thr Val Lys Val Gly Glu Ile Val Asp Ile Val Gly Leu Lys Asp
                325                 330                 335

Thr Arg Asn Thr Thr Val Thr Gly Val Glu Met Phe Gln Lys Ile Leu
            340                 345                 350

Asp Glu Ala Met Ala Gly Asp Asn Val Gly Leu Leu Leu Arg Gly Ile
        355                 360                 365

Gln Lys Ile Asp Ile Gln Arg Gly Met Val Leu Ala Lys Pro Gly Thr
    370                 375                 380

Ile Thr Pro His Thr Lys Phe Glu Ala Leu Val Tyr Val Leu Lys Lys
385                 390                 395                 400

Glu Glu Gly Gly Arg His Ser Pro Phe Phe Ala Gly Tyr Arg Pro Gln
                405                 410                 415

Phe Tyr Met Arg Thr Thr Asp Val Thr Gly Lys Val Thr Val Ile Met
            420                 425                 430

Ser Asp Lys Gly Glu Glu Ser Lys Met Val Met Pro Gly Asp Arg Val
        435                 440                 445
```

```
Asn Met Val Val Glu Leu Ile Met Pro Val Ala Cys Glu Gln Gly Met
    450                 455                 460

Arg Phe Ala Ile Arg Glu Gly Gly Lys Thr Val Gly Ala Gly Val Ile
465                 470                 475                 480

Gln Lys Ile Leu Glu
                485
```

<210> 9
<211> 1678
<212> DNA
<213> Oryza sativa

<400> 9

```
caaatccgaa cctccccccc ccccccactc ctctgcgcct ccctcctccg cggcctccgc      60
catggcctcc ctcgcctccg cctccgcatc cacctccctg gtcttctcca cctcctcctc     120
caagccgcgc ctcggctcct ccgtcggatt ctcctcgccc gcgcggttcc ggcgcacggc     180
ggcggcggcg gcgtccaagg gcacggggcg gcgcgcgggg ctgctggtaa tgcgcgcggc     240
gagggggaag ttcgagcgga ccaagccgca cgtcaacatc ggcaccatcg gccacgtcga     300
ccacgggaag actacgctga cggcggcgct caccatggtg ctcgcctccg tgggcgggag     360
cgcccccaag aagtacgacg agatcgacgc ggcgcccgag gagcgcgccc gcggcatcac     420
catcaacacc gccaccgtcg agtacgagac cgagacccgc cactacgccc acgtcgactg     480
ccccggccac gccgactacg tcaagaacat gattaccggc gccgcgcaga tggacggcgc     540
catcctcgtc gtctccggcg ccgacgggcc catgccgcag accaaggagc acatcctgct     600
cgccaagcag gtcggtgtcc ccaagattgt tgtcttcctc aacaagaagg accaggtcga     660
cgacgaggag ctgctccagc tcgtcgagct cgaggtccgc gaattgctct cctcctacga     720
gtacgatggc gacgaagtgc ccatcgtcgc tggctccgcg ctcaaggcgc tcgagaacct     780
catggccaac cctgccatta agcgcggcga tgatgagtgg gtggacggga tcttctcgtt     840
gattgattcc gtggataact acatccctgt cccacagcgc cagaccgacc tcccgttctt     900
gcttgctgtt gaggatgtgt tctccatcac cggtcgtggt accgttgcca ctggccgtat     960
tgagcgtggc accgtcaagg ttggggacac ggtcgatatc gtcggtatcc gggagactcg    1020
caactgcacg gtgactggtg ttgagatgtt ccagaagacc atggatgatg cgatggctgg    1080
ggacaatgtc ggcctgcttc tccgaggtat gcagaaggat gatatcgaga gaggcatggt    1140
gcttgcgaag cctgcttcca tcacgccaca caccaagttt gatgcggttg tgtatgtcct    1200
gaagaaagac gaaggtggac ggcactcacc gtttttccct ggttaccgcc ctcagttcta    1260
catgcggact accgatgtga cggggaatgt cccaaagatt atgaacgaca aggacgagga    1320
ggcgaagatg tgcatgcctg gtgaccgtgt caagatggtt gtggagctca tccagcccgt    1380
cgcttgtgag cagggaatga ggtttgccat ccctgagggt ggaaagaccg tcggtgccgg    1440
cgtcatcaat acgattttga agtaaagtgg atggagcata tccaccgtga gaattttcct    1500
tgtttacttt ttgcgaaatg ctctgtagtt gttattacgc gttgagtttt aggggttgat    1560
catgtgaaat tgtagtatgg cacttttgtt ttcaagtgaa tttgcatact ttgtgatatt    1620
cacgacaaag atacatgtgt ttgcaactaa tttggctatg cagtgccatt tactgtcc      1678
```

<210> 10
<211> 467
<212> PRT
<213> Oryza sativa

<400> 10

```
Met Ala Ser Leu Ala Ser Ala Ser Ala Ser Thr Ser Leu Val Phe Ser
1               5                   10                  15

Thr Ser Ser Ser Lys Pro Arg Leu Gly Ser Ser Val Gly Phe Ser Ser
            20                  25                  30

Pro Ala Arg Phe Arg Arg Thr Ala Ala Ala Ala Ala Ser Lys Gly Thr
        35                  40                  45
```

```
Gly Arg Arg Ala Gly Leu Leu Val Met Arg Ala Ala Arg Gly Lys Phe
    50                  55                  60

Glu Arg Thr Lys Pro His Val Asn Ile Gly Thr Ile Gly His Val Asp
65                  70                  75                  80

His Gly Lys Thr Thr Leu Thr Ala Ala Leu Thr Met Val Leu Ala Ser
                85                  90                  95

Val Gly Gly Ser Ala Pro Lys Lys Tyr Asp Glu Ile Asp Ala Ala Pro
            100                 105                 110

Glu Glu Arg Ala Arg Gly Ile Thr Ile Asn Thr Ala Thr Val Glu Tyr
        115                 120                 125

Glu Thr Glu Thr Arg His Tyr Ala His Val Asp Cys Pro Gly His Ala
    130                 135                 140

Asp Tyr Val Lys Asn Met Ile Thr Gly Ala Ala Gln Met Asp Gly Ala
145                 150                 155                 160

Ile Leu Val Val Ser Gly Ala Asp Gly Pro Met Pro Gln Thr Lys Glu
                165                 170                 175

His Ile Leu Leu Ala Lys Gln Val Gly Val Pro Lys Ile Val Val Phe
            180                 185                 190

Leu Asn Lys Lys Asp Gln Val Asp Asp Glu Glu Leu Leu Gln Leu Val
        195                 200                 205

Glu Leu Glu Val Arg Glu Leu Leu Ser Ser Tyr Glu Tyr Asp Gly Asp
    210                 215                 220

Glu Val Pro Ile Val Ala Gly Ser Ala Leu Lys Ala Leu Glu Asn Leu
225                 230                 235                 240

Met Ala Asn Pro Ala Ile Lys Arg Gly Asp Asp Glu Trp Val Asp Gly
                245                 250                 255

Ile Phe Ser Leu Ile Asp Ser Val Asp Asn Tyr Ile Pro Val Pro Gln
            260                 265                 270

Arg Gln Thr Asp Leu Pro Phe Leu Leu Ala Val Glu Asp Val Phe Ser
        275                 280                 285

Ile Thr Gly Arg Gly Thr Val Ala Thr Gly Arg Ile Glu Arg Gly Thr
    290                 295                 300

Val Lys Val Gly Asp Thr Val Asp Ile Val Gly Ile Arg Glu Thr Arg
305                 310                 315                 320

Asn Cys Thr Val Thr Gly Val Glu Met Phe Gln Lys Thr Met Asp Asp
                325                 330                 335

Ala Met Ala Gly Asp Asn Val Gly Leu Leu Leu Arg Gly Met Gln Lys
            340                 345                 350

Asp Asp Ile Glu Arg Gly Met Val Leu Ala Lys Pro Ala Ser Ile Thr
        355                 360                 365
```

```
Pro His Thr Lys Phe Asp Ala Val Val Tyr Val Leu Lys Lys Asp Glu
    370                 375                 380

Gly Gly Arg His Ser Pro Phe Phe Pro Gly Tyr Arg Pro Gln Phe Tyr
385                 390                 395                 400

Met Arg Thr Thr Asp Val Thr Gly Asn Val Pro Lys Ile Met Asn Asp
                405                 410                 415

Lys Asp Glu Glu Ala Lys Met Cys Met Pro Gly Asp Arg Val Lys Met
            420                 425                 430

Val Val Glu Leu Ile Gln Pro Val Ala Cys Glu Gln Gly Met Arg Phe
        435                 440                 445

Ala Ile Pro Glu Gly Gly Lys Thr Val Gly Ala Gly Val Ile Asn Thr
    450                 455                 460

Ile Leu Lys
465
```

<210> 11
<211> 1365
<212> DNA
<213> Arabidopsis thaliana

<400> 11

```
atggcgtccg ttgttcttcg aaaccctagc tcgaagcgcc ttgttccatt ctcttcccag      60
atctactctc gctgtggtgc ttccgttact tcctcttact cgatctctca ttctatcggt     120
ggagatgatc tctcttcctc taccttcgga acctcctcct tctggagatc catggccact     180
tttactcgaa ataaacctca tgtaaatgtt ggaactattg ggcatgttga tcatggcaag     240
accactttaa ctgctgcaat cacaaaggtt cttgctgagg agggcaaagc taaagctatt     300
gcctttgatg aaattgataa agctcctgaa gagaagaaga gaggaattac tattgccacg     360
gctcatgtgg agtatgaaac tgcaaagcgt cactatgctc atgtggattg ccctggacac     420
gcagattatg ttaaaaatat gattactgga gctgcgcaaa tggatggcgg aattcttgtg     480
gtttcaggac cagatggacc catgccgcag acaaaggaac atattctact tgcacgccag     540
gttggtgttc cctcacttgt gtgcttcttg aacaaagttg atgtggtgga tgaccctgag     600
ctcttggagc ttgtcgagat ggaactacgt gagctcctca gcttctacaa gtttcctggg     660
gatgatattc ccatcatccg aggatctgct ctgtccgcat tacagggcac caatgatgaa     720
attggaaggc aagctatatt aaagctgatg gatgctgttg atgaatatat acctgacccct    780
gttcgcgtcc ttgacaagcc tttcttgatg ccaattgaag atgttttctc aattcaagga     840
cgtggaactg ttgcaaccgg tcgtatcgaa cagggagtca ttaaagtggg tgaagaagtt     900
gagatattgg gtttacgtga gggggggtgtt ccactgaaat cgactgtaac tggggttgag    960
atgttcaaga agattttgga taatggacag gctggtgata atgtaggact tcttctgcgt    1020
gggctaaaga gagaagacat tcagcgtgga atggtgattg ctaagcctgg ttcatgcaag    1080
acatacaaga agtttgaagc agagatttac gtgctcacaa aggatgaagg tggacgtcac    1140
actgcatttt tctctaacta caggcctcag ttttacttga ggactgcaga tatcactggc    1200
aaagtggaat tacccgaaaa cgtgaagatg gttatgcctg gtgacaatgt cacagctgtt    1260
ttcgagttaa tcatgcctgt cccactcgaa acaggtcaaa gatttgcctt aagggaagga    1320
ggtagaacag ttggagctgg tgttgtatca aaagtgatga cctaa                    1365
```

<210> 12
<211> 454
<212> PRT
<213> Arabidopsis thaliana

<400> 12

Met Ala Ser Val Val Leu Arg Asn Pro Ser Ser Lys Arg Leu Val Pro

```
1               5                   10                  15

Phe Ser Ser Gln Ile Tyr Ser Arg Cys Gly Ala Ser Val Thr Ser Ser
            20                  25                  30

Tyr Ser Ile Ser His Ser Ile Gly Gly Asp Asp Leu Ser Ser Ser Thr
        35                  40                  45

Phe Gly Thr Ser Ser Phe Trp Arg Ser Met Ala Thr Phe Thr Arg Asn
    50                  55                  60

Lys Pro His Val Asn Val Gly Thr Ile Gly His Val Asp His Gly Lys
65                  70                  75                  80

Thr Thr Leu Thr Ala Ala Ile Thr Lys Val Leu Ala Glu Glu Gly Lys
                85                  90                  95

Ala Lys Ala Ile Ala Phe Asp Glu Ile Asp Lys Ala Pro Glu Glu Lys
            100                 105                 110

Lys Arg Gly Ile Thr Ile Ala Thr Ala His Val Glu Tyr Glu Thr Ala
        115                 120                 125

Lys Arg His Tyr Ala His Val Asp Cys Pro Gly His Ala Asp Tyr Val
    130                 135                 140

Lys Asn Met Ile Thr Gly Ala Ala Gln Met Asp Gly Gly Ile Leu Val
145                 150                 155                 160

Val Ser Gly Pro Asp Gly Pro Met Pro Gln Thr Lys Glu His Ile Leu
                165                 170                 175

Leu Ala Arg Gln Val Gly Val Pro Ser Leu Val Cys Phe Leu Asn Lys
            180                 185                 190

Val Asp Val Val Asp Asp Pro Glu Leu Leu Glu Leu Val Glu Met Glu
        195                 200                 205

Leu Arg Glu Leu Leu Ser Phe Tyr Lys Phe Pro Gly Asp Asp Ile Pro
    210                 215                 220

Ile Ile Arg Gly Ser Ala Leu Ser Ala Leu Gln Gly Thr Asn Asp Glu
225                 230                 235                 240

Ile Gly Arg Gln Ala Ile Leu Lys Leu Met Asp Ala Val Asp Glu Tyr
                245                 250                 255

Ile Pro Asp Pro Val Arg Val Leu Asp Lys Pro Phe Leu Met Pro Ile
            260                 265                 270

Glu Asp Val Phe Ser Ile Gln Gly Arg Gly Thr Val Ala Thr Gly Arg
        275                 280                 285

Ile Glu Gln Gly Val Ile Lys Val Gly Glu Glu Val Glu Ile Leu Gly
    290                 295                 300

Leu Arg Glu Gly Gly Val Pro Leu Lys Ser Thr Val Thr Gly Val Glu
305                 310                 315                 320
```

```
Met Phe Lys Lys Ile Leu Asp Asn Gly Gln Ala Gly Asp Asn Val Gly
                325                     330                 335

Leu Leu Leu Arg Gly Leu Lys Arg Glu Asp Ile Gln Arg Gly Met Val
            340                 345                 350

Ile Ala Lys Pro Gly Ser Cys Lys Thr Tyr Lys Lys Phe Glu Ala Glu
        355                 360                 365

Ile Tyr Val Leu Thr Lys Asp Glu Gly Gly Arg His Thr Ala Phe Phe
    370                 375                 380

Ser Asn Tyr Arg Pro Gln Phe Tyr Leu Arg Thr Ala Asp Ile Thr Gly
385                 390                 395                 400

Lys Val Glu Leu Pro Glu Asn Val Lys Met Val Met Pro Gly Asp Asn
                405                 410                 415

Val Thr Ala Val Phe Glu Leu Ile Met Pro Val Pro Leu Glu Thr Gly
            420                 425                 430

Gln Arg Phe Ala Leu Arg Glu Gly Gly Arg Thr Val Gly Ala Gly Val
        435                 440                 445

Val Ser Lys Val Met Thr
    450
```

<210> 13
<211> 1425
<212> DNA
<213> Arabidopsis thaliana

<400> 13

```
tagtttgaga tggaagaacc agtagaccga gaagatatcg atttaagggt ttcatccaca     60
gatataggct ggaacgaatt cgcggccgcc tcgaagcgcc ttgttccatt ctcttcccag    120
atctactctc gctgtggtgc ttccgttact tcctcttact cgatctctca ttctatcggt    180
ggagatgatc tctcttcctc taccttcgga acctcctcct tctggagatc catggccact    240
tttactcgaa ataaacctca tgtaaatgtt ggaactattg ggcatgttga tcatggcaag    300
accactttaa ctgctgcaat cacaaaggtt cttgctgagg agggcaaagc taaagctatt    360
gcctttgatg aaattgataa agctcctgaa gagaagaaga gaggaattac tattgccacg    420
gctcatgtgg agtatgaaac tgcaaagcgt cactatgctc atgtggattg ccctggacac    480
gcagattatg ttaaaaatat gattactgga gctgcgcaaa tggatggcgg aattcttgtg    540
gtttcaggac cagatggacc catgccgcag acaaaggaac atattctact tgcacgccag    600
gttggtgttc cctcacttgt gtgcttcttg aacaaagttg atgtggtgga tgaccctgag    660
ctcttggagc ttgtcgagat ggaactacgt gagctcctca gcttctacaa gtttcctggg    720
gatgatattc ccatcatccg aggatctgct ctgtccgcat tacagggcac caatgatgaa    780
attggaaggc aagctatatt aaagctgatg gatgctgttg atgaatatat acctgaccct    840
gttcgcgtcc ttgacaagcc tttcttgatg ccaattgaag atgtttctc aattcaagga     900
cgtggaactg ttgcaaccgg tcgtatcgaa cagggagtca ttaaagtggg tgaagaagtt    960
gagatattgg gtttacgtga agggggtgt tccactaaat cgactgtaac tggggttgag    1020
atgttcaaga agattttgga taatggacag gctggtgata atgtaggact tcttctgcgt   1080
gggctaaaga gagaagacat tcagcgtgga atggtgattg ctaagcctgg ttcatgcaag   1140
acatacaaga agtttgaagc agagatttac gtgctcacaa aggatgaagg tggacgtcac   1200
actgcatttt tctctaacta caggcctcag ttttacttga ggactgcaga tatcactggc   1260
aaagtggaat tacccgaaaa cgtgaagatg gttatgcctg gtgacaatgt cacagctgtt   1320
ttcgagttaa tcatgcctgt cccactcgaa acaggtcaaa gatttgcctt aagggaagga   1380
ggtagaacag ttggagctgg tgttgtatca aaagtgatga cctaa                   1425
```

<210> 14
<211> 471
<212> PRT
<213> Arabidopsis thaliana

<400> 14

```
Met Glu Glu Pro Val Asp Arg Glu Asp Ile Asp Leu Arg Val Ser Ser
1               5                   10                  15

Thr Asp Ile Gly Trp Asn Glu Phe Ala Ala Ala Ser Lys Arg Leu Val
            20                  25                  30

Pro Phe Ser Ser Gln Ile Tyr Ser Arg Cys Gly Ala Ser Val Thr Ser
        35                  40                  45

Ser Tyr Ser Ile Ser His Ser Ile Gly Gly Asp Asp Leu Ser Ser Ser
    50                  55                  60

Thr Phe Gly Thr Ser Ser Phe Trp Arg Ser Met Ala Thr Phe Thr Arg
65                  70                  75                  80

Asn Lys Pro His Val Asn Val Gly Thr Ile Gly His Val Asp His Gly
                85                  90                  95

Lys Thr Thr Leu Thr Ala Ala Ile Thr Lys Val Leu Ala Glu Glu Gly
            100                 105                 110

Lys Ala Lys Ala Ile Ala Phe Asp Glu Ile Asp Lys Ala Pro Glu Glu
        115                 120                 125

Lys Lys Arg Gly Ile Thr Ile Ala Thr Ala His Val Glu Tyr Glu Thr
    130                 135                 140

Ala Lys Arg His Tyr Ala His Val Asp Cys Pro Gly His Ala Asp Tyr
145                 150                 155                 160

Val Lys Asn Met Ile Thr Gly Ala Ala Gln Met Asp Gly Gly Ile Leu
                165                 170                 175

Val Val Ser Gly Pro Asp Gly Pro Met Pro Gln Thr Lys Glu His Ile
            180                 185                 190

Leu Leu Ala Arg Gln Val Gly Val Pro Ser Leu Val Cys Phe Leu Asn
        195                 200                 205

Lys Val Asp Val Val Asp Asp Pro Glu Leu Leu Glu Leu Val Glu Met
    210                 215                 220

Glu Leu Arg Glu Leu Leu Ser Phe Tyr Lys Phe Pro Gly Asp Asp Ile
225                 230                 235                 240

Pro Ile Ile Arg Gly Ser Ala Leu Ser Ala Leu Gln Gly Thr Asn Asp
                245                 250                 255

Glu Ile Gly Arg Gln Ala Ile Leu Lys Leu Met Asp Ala Val Asp Glu
            260                 265                 270

Tyr Ile Pro Asp Pro Val Arg Val Leu Asp Lys Pro Phe Leu Met Pro
        275                 280                 285
```

```
Ile Glu Asp Val Phe Ser Ile Gln Gly Arg Gly Thr Val Ala Thr Gly
    290                 295                 300

Arg Ile Glu Gln Gly Val Ile Lys Val Gly Glu Glu Val Glu Ile Leu
305                 310                 315                 320

Gly Leu Arg Glu Gly Gly Cys Ser Thr Lys Ser Thr Val Thr Gly Val
                325                 330                 335

Glu Met Phe Lys Lys Ile Leu Asp Asn Gly Gln Ala Gly Asp Asn Val
            340                 345                 350

Gly Leu Leu Leu Arg Gly Leu Lys Arg Glu Asp Ile Gln Arg Gly Met
        355                 360                 365

Val Ile Ala Lys Pro Gly Ser Cys Lys Thr Tyr Lys Lys Phe Glu Ala
    370                 375                 380

Glu Ile Tyr Val Leu Thr Lys Asp Glu Gly Gly Arg His Thr Ala Phe
385                 390                 395                 400

Phe Ser Asn Tyr Arg Pro Gln Phe Tyr Leu Arg Thr Ala Asp Ile Thr
                405                 410                 415

Gly Lys Val Glu Leu Pro Glu Asn Val Lys Met Val Met Pro Gly Asp
            420                 425                 430

Asn Val Thr Ala Val Phe Glu Leu Ile Met Pro Val Pro Leu Glu Thr
        435                 440                 445

Gly Gln Arg Phe Ala Leu Arg Glu Gly Gly Arg Thr Val Gly Ala Gly
    450                 455                 460

Val Val Ser Lys Val Met Thr
465                 470
```

<210> 15
<211> 1725
<212> DNA
<213> Oryza sativa

<400> 15

```
cccgtctctc tcctcctctc acctagcgcc gccgccgccg ccgccgcgaa cgcgaacgcg     60
atggccgcag ccgcagtgct ccggagccat ggcgcccgcc gcatcctctc ctaccccacc    120
ctccgcgccg ccgtgatctc gggccccacg gcgctaccgg atgcgtcggc ggctgcggcg    180
gcggcgcccc agcagccacc gcctctggct ggaaccctgt gggcgaggtc catggccacc    240
ttcacccgca cgaagcctca tgtgaatgtt ggcaccattg ggcacgtcga tcatggcaaa    300
accacactca ctgctgcgat taccaaggtg ttagctgagg cagggaaggc taaagctgtt    360
gcttttgacg agatcgacaa ggccccggag gagaaagcaa gaggaatcac cattgcaacg    420
gctcatgtgg agtacgagac tgctaaaagg cattatgctc atgtggactg cccagggcat    480
gctgattatg tcaagaatat gatcactgga gctgctcaaa tggatggtgg tattcttgtc    540
gtatcagctc ctgatggccc catgccacaa acgaaagagc atattcttct ggcccgacag    600
gttggtgtgc catcacttgt atgtttctta aacaaagttg atgctgttga tgaccccgag    660
ctactagagc ttgtagagat ggagcttcgt gagctcctca gtttctacaa gttccctggt    720
gatgagattc caatcatccg tggatccgcc ttgtcagcct tacaggggac taatgatgag    780
attggaaaga atgccatttt gaaactaatg gatgcagttg acgagtatat ccctgatcct    840
gtgaggcagc ttgataagtc tttcttgatg ccgatagaag atgttttctc tatccagggc    900
cgtggaactg ttgtgacagg gcgtgttgag caggggacaa tcaaaactgg tgaagatgtt    960
gagatcctag gtttgactcc aagtggtccc ttgaagacta cagttactgg tgttgagatg   1020

ttcaaaaaga tactggatca tggagaggct ggtgacaatg ttggtcttct tcttcgtggt   1080
cttaagcgtg gtgatgtaca gcgtggccag gtggtgtgca aacctggtac cgttaagacg   1140
taccaaaagt ttgaggcgga aatttacgtc cttacaaagg atgaaggtgg tcgccacaca   1200
gccttcttgt caaattacag tccacagttc tactttagga ctgccgatgt cactggaaaa   1260
gttgtgttac ctgatggcgt ggaaatggtt atgcctggtg ataatgtaac tgcaggattt   1320
gagttgatat cacctgttcc ccttgagcca ggccagagat ttgcgctgag ggaaggaggg   1380
aggacagttg gtgccggagt tgtttccaaa gtttacagct gatctgtttc cagttttttc   1440
tgcgtagcga atttagtaac aaatgaatgt tcatagggaa tcagatctat ccgaccggca   1500
atatgagagc aaaagagcga gggactcgca gagcatcttc tctgaattca tgagatgcag   1560
ggatcgttgg ctcggccacc attccgaact agttattgct tttctttttc catgtctggt   1620
tgttgctgta taacaacgcg tttagtcatc ttaacacgtt tttttgccca tagcttggtg   1680
actaaataag aaagattcat cataagcatc ttaatggttg atgtt                   1725
```

<210> 16
<211> 453
<212> PRT
<213> Oryza sativa

<400> 16

```
Met Ala Ala Ala Ala Val Leu Arg Ser His Gly Ala Arg Arg Ile Leu
1               5                   10                  15

Ser Tyr Pro Thr Leu Arg Ala Ala Val Ile Ser Gly Pro Thr Ala Leu
            20                  25                  30

Pro Asp Ala Ser Ala Ala Ala Ala Ala Ala Pro Gln Gln Pro Pro Pro
        35                  40                  45

Leu Ala Gly Thr Leu Trp Ala Arg Ser Met Ala Thr Phe Thr Arg Thr
    50                  55                  60

Lys Pro His Val Asn Val Gly Thr Ile Gly His Val Asp His Gly Lys
65                  70                  75                  80

Thr Thr Leu Thr Ala Ala Ile Thr Lys Val Leu Ala Glu Ala Gly Lys
                85                  90                  95

Ala Lys Ala Val Ala Phe Asp Glu Ile Asp Lys Ala Pro Glu Glu Lys
            100                 105                 110

Ala Arg Gly Ile Thr Ile Ala Thr Ala His Val Glu Tyr Glu Thr Ala
        115                 120                 125

Lys Arg His Tyr Ala His Val Asp Cys Pro Gly His Ala Asp Tyr Val
    130                 135                 140

Lys Asn Met Ile Thr Gly Ala Ala Gln Met Asp Gly Gly Ile Leu Val
145                 150                 155                 160

Val Ser Ala Pro Asp Gly Pro Met Pro Gln Thr Lys Glu His Ile Leu
                165                 170                 175

Leu Ala Arg Gln Val Gly Val Pro Ser Leu Val Cys Phe Leu Asn Lys
            180                 185                 190

Val Asp Ala Val Asp Asp Pro Glu Leu Leu Glu Leu Val Glu Met Glu
        195                 200                 205

Leu Arg Glu Leu Leu Ser Phe Tyr Lys Phe Pro Gly Asp Glu Ile Pro
```

```
        210                 215                 220

    Ile Ile Arg Gly Ser Ala Leu Ser Ala Leu Gln Gly Thr Asn Asp Glu
    225                 230                 235                 240

    Ile Gly Lys Asn Ala Ile Leu Lys Leu Met Asp Ala Val Asp Glu Tyr
                    245                 250                 255

    Ile Pro Asp Pro Val Arg Gln Leu Asp Lys Ser Phe Leu Met Pro Ile
                260                 265                 270

    Glu Asp Val Phe Ser Ile Gln Gly Arg Gly Thr Val Val Thr Gly Arg
            275                 280                 285

    Val Glu Gln Gly Thr Ile Lys Thr Gly Glu Asp Val Glu Ile Leu Gly
        290                 295                 300

    Leu Thr Pro Ser Gly Pro Leu Lys Thr Thr Val Thr Gly Val Glu Met
    305                 310                 315                 320

    Phe Lys Lys Ile Leu Asp His Gly Glu Ala Gly Asp Asn Val Gly Leu
                    325                 330                 335

    Leu Leu Arg Gly Leu Lys Arg Gly Asp Val Gln Arg Gly Gln Val Val
                340                 345                 350

    Cys Lys Pro Gly Thr Val Lys Thr Tyr Gln Lys Phe Glu Ala Glu Ile
            355                 360                 365

    Tyr Val Leu Thr Lys Asp Glu Gly Gly Arg His Thr Ala Phe Leu Ser
        370                 375                 380

    Asn Tyr Ser Pro Gln Phe Tyr Phe Arg Thr Ala Asp Val Thr Gly Lys
    385                 390                 395                 400

    Val Val Leu Pro Asp Gly Val Glu Met Val Met Pro Gly Asp Asn Val
                    405                 410                 415

    Thr Ala Gly Phe Glu Leu Ile Ser Pro Val Pro Leu Glu Pro Gly Gln
                420                 425                 430

    Arg Phe Ala Leu Arg Glu Gly Gly Arg Thr Val Gly Ala Gly Val Val
            435                 440                 445

    Ser Lys Val Tyr Ser
        450
```

<210> 17
<211> 5993
<212> DNA
<213> Zea mays

<400> 17

```
ggatcctacg accttgtcca gcgttgcccg catctccggg ctcatagccg ccaggcaccg     60
caccgaccgg ctgcgctccg ggggtgagag ccacgcgcca cgcctgagag caaggcgctg    120
ggccggagca ggaggaatca gggagagcaa ggcgaccctg gcaggccgcg tccgcgtcga    180
gagcgggagc gggacggcgg tcccggcgag aggcctcgcg gccgtggcgg aggcagccat    240
cgcggtgcgc cggcggcgac ggatggtacg gaggcgcgtt tgtggtgggc tgagggctgt    300
ggacgggaaa aacggctgtg ggctcctcta gtagagctaa taaattagcg tttggttgag    360
```

```
ctagctaata aactaatagt tagttataag atagctaaca attaacagtg ttattaacgg    420
agactgtttc aaactaaaca actaagtttt agtagctaac tattaatttt agaggttcca    480
aatagagcct aaaaccgttc gatggaaaac actacatata tattttcaca atttcattta    540
aaagttgcta aaacagattt agaggtgctt tcagttttac catacgagaa aatcggcttt    600
taaaaaagct agtttctaaa tccacttttt tttgttaagc ttttgatttt tagggagcaa    660
aatccaaaac caaaagccat accaaatata cagttaggac ggcatggaat aattaattaa    720
accagtaata attattttc gtggccgtgg aggagaacgg aggaaggttg tcgaagggag    780
tcgagccgtc gaggcagcaa gccgagtcga taccggagcg gcccatgggc catgtatggt    840
gttgtcttgg gccgcaaatc tgtgccttcc agtccagcgt gccgcacggt atgcgtctgc    900
cggccgttct cattcccctc caaacttaaa gccctaaacc catcttgctc ccccctcgtc    960
tccactgctc tgctgctcct cgcgccgccg cagcgacgta atggcttccg ccgcggtgct   1020
ccggaacgcc ggctcccggc gcctcttctc ctaccctact ctccgcgccg ctgcgatata   1080
tgtaccttct gcgctacccg atgcgcccgc ggcggcggca gcgccggccc agccgccacc   1140
gacggccggg accctctggg cgaggtctat ggccaccttc acgcgcacgt gagtactcct   1200
tggaccagat ccgttccgtg cccctatttt ctcgttctag aatcactcat gagttcactt   1260
ccattgatcc gtgcaattca atatccgtct attgcttcgt gtgattcact ttgcaagtcg   1320
tgtcaatctg tggcaggaag ccccatgtga ccgtcggcac cattgggcac gtcgatcacg   1380
gcaaaaccac tctcactgct gccattacca aggttggatg tgttatactg ttcctgcttc   1440
acatctcgct tcatttccaa acggatgggc tgattctatg gcgtctattt cctcgttatt   1500
ataggtcctg gccgaggcag ggaaggccaa agccgttgct ttcgacgaga tcgacaaggc   1560
tccggaggag aaagccagag gaatcaccat tgcgacagtt gggtcctagc acgtctccta   1620
tagttgtgcc agaccgatag agcaattctc tttatatttt ttattcttca tttactgccc   1680
agtgtttaac catttggatt gtttttaggc tcacgtcgag tatgagacgg ctaaaaggca   1740
ttatgctcac gttgattgtc caggtcacgc agattatgtc aaggtttata cgaatcaaga   1800
tttttttctt tctcaaatgc attattatta ttttagtaat gaaggttgtt tgcacgtcaa   1860
attgtgaaaa atctattgaa agccaccatc tagagccatt acattccttt gtttatggtc   1920
caagtctttt tgtttttgtg aaaatggtag aacatgatca ctggagctgc tcaaatggat   1980
ggtggtattc ttgtcgtgtc agctcctgat ggcccgatgc cacaaactaa ggagcatatt   2040
cttcttgccc gtcaggtatg gaatgcttgt ttctattgtt gtttaaaatg cttctttcag   2100
gctctgcttg tatagtctcg acttgatcag ataaacatga gctttctttt taggttggtg   2160
taccttccct tgtgtgcttc ttaaacaaag ttgatgctgt tgatgatcca gagcttctgg   2220
aacttgtaga gatggagctt cggggtatgc taccattcat gtctagacat catgtttaga   2280
ttcctgtatg tcttctgtat tttttaaggt tgtcttcttg gtccccctcc ccctcttttt   2340
tctgcagagc tcctcagttt ctacaagttc cccggtgatg agattccgat catccgtggg   2400
tctgcgttgt ctgctttgca agggaacaat gatgagatag gaaagaatgc aattttgaaa   2460
ttaatggacg cagttgatga gtacatccct gatcctgtga ggcagcttga taagcctttc   2520
ttgatgccga tagaagatgt gttttctatt caggtgaatt tatttatgag ctatcactac   2580
tgcagtatgc attagctatt caattatgat gtttgatgtt tcttcgacgt tgattaggga   2640
tactagtttt gtttttcttg ggctactggg tccatcaaaa cttaccaagt cagaatactt   2700
ttgaattatg gctataggga tgcaatgcaa atatgcaatg aattaaagtg gtgacccaac   2760
taaatgctga gattttcagt accgctgtat taagcattgt atattcccta tttgtgtgaa   2820
taaatgtttc tattcagcat cttcttttgt taaactaatt aagattgcac caagaaacag   2880
tttaaagttt gcatagattg accgcttgca tttgctgttg aatgaaaaca cataaaagta   2940
ttagtttgtg ttgtttgaaa taaggttgtt aaagagatca ctggtaaatg tgttgccatg   3000
gagattgtgt caatatatga aaatgcatat attttatcgg attaatagaa aatgaaaagg   3060
aaaaacattg ataggactgt actgtcaatc aggtcatatg cttttttat atttagtaat    3120
tatttgtgtg gcggccattc ttgtttccag ttacatactg tacaacgagg attcttcttg   3180
ttcttatatt cctactgtct aagaaccaaa gggttgtttc tagtgacgcc gttgggcaaa   3240
aatttggtta cccagtcccc atacctgaaa atcatccacc ccgctttatc catccccaga   3300
actagttgat aatgcccaat gggcatggat gacccaatgc gtaagcagtg acggctatca   3360
ttaccctcac cgcaaccacc ggctgcacca tggtcttgtt ggacactctg ctgtgcacct   3420
ccttgttgca aattagcctg tgcgcagcgc agggactaaa cacagatgct gatgtgggtg   3480
tccacagcca ccttcaaagc ctgctgctgt tgccaccgtt gtcagctgtc acacaagcgc   3540
tcggcggcgg ccagggactt agacagtggc ggccccatta aacaggggtg gcactggcag   3600
gcaagtggac agtggacatc tgcaactcca tcaaaggtat caatgtctgg tgttggcgtg   3660
acttttggca gaagcacctg tgccggcatt gaggacttga aacgccttgc cgcccacttg   3720
ctgctggggc cgctgctgtc gttgtcggtg gcgactgggg ccttggtttg acagatgtag   3780
atggcccatg gtgcttggtg ctggtggctg cagggagggc cgtaagcaga atagaccagg   3840
tgattgggga agcgggtttc ccatgtttta tctacccata cccaatttta gatggataat   3900
```

```
ccataccata cccaaacaga atgggtatgg atttggttga agggtaacaa atggcaacat   3960
tagtgatttc agtagacatt ctttattata ccatatttgt ggttttgtgc tctttctgac   4020
atgttttgtt ttcttttagt attttaaagt tttttcgttt tcatttacaa tttcctccct   4080
tgtgctgtca tgttcttaaa gtaaacatga attgaattgc tgatggagct attaagcatt   4140
gcaatgcaat gcaatggcca tttaatcaaa tgtttttttg tgagggagtt attataagtt   4200
tttaagtagc ctgtctgatc tgcagggtcg tggaactgtt gttactgggc gtgttgaaca   4260
gggaacaatc aaaactggtg aagatgttga gatcttgggt ttggcacagg ttagatcctt   4320
tttgaactga attactgcat gtttatccta gtttgtagtg tttgaagtac ggattgaatg   4380
agtttgtggt ttataaaatt tagtatgtta gtactcctta attataaggt gtttttggct   4440
tttcaagata cattgttttt attgtgtatc tagacatagt gcatatctaa gtgcatagca   4500
aaaattgtat ttaaacatgg agctctaact tagtatacaa cctgccattt ccttgactgt   4560
ctaacttttg gatgatttat ttgtaaatgt aaggcaataa ctaatcacaa gagcaggagg   4620
ttctaatatt tctttttctg gtttagtacc cactgcacct atatatgttg tgacgaaata   4680
tattattggt tgcagactgg tcccttgaag actacagtta ctggtgttga gatgttcaaa   4740
aagatactgg accatgggaa agtaatgatt ctgaaagcat agtgtgtgag gctttctatt   4800
gatccaaatt atcatacatg aaacttcttg tgtacaggct ggtgacaacg ttgggcttct   4860
tctccgtggt cttaagcgtg gtgatgttga gcgtggccag gttagatttc ctgaattcga   4920
aattgtctga gtaagttttt cgaaatattc attgaactgt tttgcctgga aaacgccttg   4980
catttgatgg tacatggttg cgtgatcacc aaacaaatta tacagttctc ttaatcctga   5040
tcctctgtat gttcctcaat ttctgggtgt ccaatatcga aactgaacga tctcgaatgt   5100
tgacaccctg tcgtttccta ccgtttcagg tggtgtgcag acctgggtat tctgaagact   5160
gcaaaaagtt cgaggcggaa atatatgtcc tgacaaagga tgaaggtggc cggcacactg   5220
cctttgtcac aaattacagc ccccaattct acttcaggac agctgatgtc actggaaggg   5280
ttgagctgct tggggagatg aagatggttt tgcccggtga caatgtaact gcgaattttg   5340
acttgatatc acctgttcct cttgaacctg gtaacgatta cattctcaat ttctttcgac   5400
ggtatagaac tatagaccag gtcttctgac aacatgctgt tcttcgtgat gccattgcac   5460
gctccaagct acttcttttc attgaatact aaagaaattc atattggatc gtcaactccg   5520
cctttgatgg cagggcaaag atttgcgatt agggaaggag gaagaacggt cggtgctgga   5580
gtcgtctcca aagttcttag ctgatcagtt tccactttct tctccgtagc aattcagtga   5640
acaaacgagt gttgatatgt ccgaccgggt tagaaacgag cgcaaagcac cgggttttct   5700
tttgcatttt tcttggaatg cctgagcaaa ggcagagctg gcctggccag tggccaccat   5760
tttattgttg aggggcgtgg tgctgtgtac accacggtta gtttggttga cacgcaagtt   5820
gggcataact ttgattgaat aatactagtc tcatcataaa catattttct agcaattttg   5880
tcatgtcggt cttggaggcg ctttgtcaag tccgcgaaaa aaaatgctgg ttggactata   5940
tatgtgtatg tatttgtgat gtttaccgtc gctagcttct gatctggtga tgc          5993
```

<210> 18
<211> 452
<212> PRT
<213> Zea mays

<400> 18

```
Met Ala Ser Ala Ala Val Leu Arg Asn Ala Gly Ser Arg Arg Leu Phe
1               5                   10                  15

Ser Tyr Pro Thr Leu Arg Ala Ala Ala Ile Tyr Val Pro Ser Ala Leu
            20                  25                  30

Pro Asp Ala Pro Ala Ala Ala Ala Ala Pro Ala Gln Pro Pro Pro Thr
        35                  40                  45

Ala Gly Thr Leu Trp Ala Arg Ser Met Ala Thr Phe Thr Arg Thr Lys
    50                  55                  60

Pro His Val Thr Val Gly Thr Ile Gly His Val Asp His Gly Lys Thr
65                  70                  75                  80

Thr Leu Thr Ala Ala Ile Thr Lys Val Leu Ala Glu Ala Gly Lys Ala
                85                  90                  95
```

```
Lys Ala Val Ala Phe Asp Glu Ile Asp Lys Ala Pro Glu Glu Lys Ala
            100                 105                 110

Arg Gly Ile Thr Ile Ala Thr Ala His Val Glu Tyr Glu Thr Ala Lys
        115                 120                 125

Arg His Tyr Ala His Val Asp Cys Pro Gly His Ala Asp Tyr Val Lys
    130                 135                 140

Asn Met Ile Thr Gly Ala Ala Gln Met Asp Gly Gly Ile Leu Val Val
145                 150                 155                 160

Ser Ala Pro Asp Gly Pro Met Pro Gln Thr Lys Glu His Ile Leu Leu
                165                 170                 175

Ala Arg Gln Val Gly Val Pro Ser Leu Val Cys Phe Leu Asn Lys Val
            180                 185                 190

Asp Ala Val Asp Asp Pro Glu Leu Leu Glu Leu Val Glu Met Glu Leu
        195                 200                 205

Arg Glu Leu Leu Ser Phe Tyr Lys Phe Pro Gly Asp Glu Ile Pro Ile
    210                 215                 220

Ile Arg Gly Ser Ala Leu Ser Ala Leu Gln Gly Asn Asn Asp Glu Ile
225                 230                 235                 240

Gly Lys Asn Ala Ile Leu Lys Leu Met Asp Ala Val Asp Glu Tyr Ile
                245                 250                 255

Pro Asp Pro Val Arg Gln Leu Asp Lys Pro Phe Leu Met Pro Ile Glu
            260                 265                 270

Asp Val Phe Ser Ile Gln Gly Arg Gly Thr Val Val Thr Gly Arg Val
        275                 280                 285

Glu Gln Gly Thr Ile Lys Thr Gly Glu Asp Val Glu Ile Leu Gly Leu
    290                 295                 300

Ala Gln Thr Gly Pro Leu Lys Thr Thr Val Thr Gly Val Glu Met Phe
305                 310                 315                 320

Lys Lys Ile Leu Asp His Gly Lys Ala Gly Asp Asn Val Gly Leu Leu
                325                 330                 335

Leu Arg Gly Leu Lys Arg Gly Asp Val Glu Arg Gly Gln Val Val Cys
            340                 345                 350

Arg Pro Gly Tyr Ser Glu Asp Cys Lys Lys Phe Glu Ala Glu Ile Tyr
        355                 360                 365

Val Leu Thr Lys Asp Glu Gly Gly Arg His Thr Ala Phe Val Thr Asn
    370                 375                 380

Tyr Ser Pro Gln Phe Tyr Phe Arg Thr Ala Asp Val Thr Gly Arg Val
385                 390                 395                 400

Glu Leu Leu Gly Glu Met Lys Met Val Leu Pro Gly Asp Asn Val Thr
```

```
                    405                     410                     415
Ala Asn Phe Asp Leu Ile Ser Pro Val Pro Leu Glu Pro Gly Gln Arg
                420                     425                     430
Phe Ala Ile Arg Glu Gly Gly Arg Thr Val Gly Ala Gly Val Val Ser
            435                     440                     445
Lys Val Leu Ser
    450
```

<210> 19
<211> 1350
<212> DNA
<213> Arabidopsis thaliana

<400> 19

```
atgggtaaag agaagtttca catcaacatt gtggtcattg gccacgtcga ttctggaaag      60
tcgaccacca ctgggcactt gatctacaag ttgggtggta ttgacaagcg tgtcattgag     120
aggttcgaga aggaggctgc tgagatgaac aagaggtcct tcaagtacgc atgggttttg     180
gacaaactta aggctgagcg tgagcgtggt atcaccattg acattgctct ctggaagttc     240
gagaccacca agtactactg cactgtcatt gatgctcctg gccatcgtga tttcatcaag     300
aacatgatca ctggtacctc ccaggctgat tgtgctgtcc ttatcattga ctccaccact     360
ggtggttttg aggctggtat ctccaaggat ggtcagaccc gtgagcacgc tctacttgct     420
ttcacccttg gtgtcaagca gatgatctgc tgttgtaaca agatggatgc cactaccccc     480
aagtactcca aggccaggta cgatgaaatc atcaaggagg tgtcttccta cttgaagaag     540
gttggttaca accccgacaa aatcccattt gtgcccatct ctggatttga gggtgacaac     600
atgattgaga ggtccaccaa ccttgactgg tacaagggac caactctcct tgaggctctt     660
gaccagatca acgagcccaa gaggccgtca gacaagcccc ttcgtctccc acttcaggat     720
gtctacaaga ttggtggtat tggaacggtg ccagtgggac gtgttgagac tggtatgatc     780
aagcctggta tggttgtgac ctttgctccc acaggattga ccactgaggt caagtctgtt     840
gagatgcacc acgagtctct tcttgaggca cttccaggtg acaacgttgg gttcaatgtt     900
aagaatgttg ctgtcaagga tcttaagaga gggtacgtcg catccaactc caaggatgac     960
cctgccaagg gtgctgctaa cttcacctcc caggtcatca tcatgaacca ccctggtcag    1020
attggtaacg gttacgcccc agtcctggat tgccacacct ctcacattgc agtcaagttc    1080
tctgagatct tgaccaagat tgacaggcgt tctggtaagg agattgagaa ggagcctaaa    1140
ttcttgaaga atggtgatgc tggtatggtg aagatgactc caaccaagcc catggttgtg    1200
gagaccttct ctgagtaccc accacttgga cgtttcgctg tgagggacat gaggcagact    1260
gttgcagtcg gtgttatcaa gagtgttgac aagaaggacc caactggagc caaggttacc    1320
aaggctgcag tcaagaaggg tgccaagtga                                    1350
```

<210> 20
<211> 449
<212> PRT
<213> Arabidopsis thaliana

<400> 20

```
Met Gly Lys Glu Lys Phe His Ile Asn Ile Val Val Ile Gly His Val
1               5                   10                  15

Asp Ser Gly Lys Ser Thr Thr Thr Gly His Leu Ile Tyr Lys Leu Gly
            20                  25                  30

Gly Ile Asp Lys Arg Val Ile Glu Arg Phe Glu Lys Glu Ala Ala Glu
        35                  40                  45

Met Asn Lys Arg Ser Phe Lys Tyr Ala Trp Val Leu Asp Lys Leu Lys
    50                  55                  60
```

```
Ala Glu Arg Glu Arg Gly Ile Thr Ile Asp Ile Ala Leu Trp Lys Phe
65                  70                  75                  80

Glu Thr Thr Lys Tyr Tyr Cys Thr Val Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Ile Asp Ser Thr Thr Gly Gly Phe Glu Ala Gly Ile Ser
        115                 120                 125

Lys Asp Gly Gln Thr Arg Glu His Ala Leu Leu Ala Phe Thr Leu Gly
    130                 135                 140

Val Lys Gln Met Ile Cys Cys Cys Asn Lys Met Asp Ala Thr Thr Pro
145                 150                 155                 160

Lys Tyr Ser Lys Ala Arg Tyr Asp Glu Ile Ile Lys Glu Val Ser Ser
                165                 170                 175

Tyr Leu Lys Lys Val Gly Tyr Asn Pro Asp Lys Ile Pro Phe Val Pro
            180                 185                 190

Ile Ser Gly Phe Glu Gly Asp Asn Met Ile Glu Arg Ser Thr Asn Leu
        195                 200                 205

Asp Trp Tyr Lys Gly Pro Thr Leu Leu Glu Ala Leu Asp Gln Ile Asn
    210                 215                 220

Glu Pro Lys Arg Pro Ser Asp Lys Pro Leu Arg Leu Pro Leu Gln Asp
225                 230                 235                 240

Val Tyr Lys Ile Gly Gly Ile Gly Thr Val Pro Val Gly Arg Val Glu
                245                 250                 255

Thr Gly Met Ile Lys Pro Gly Met Val Val Thr Phe Ala Pro Thr Gly
            260                 265                 270

Leu Thr Thr Glu Val Lys Ser Val Glu Met His His Glu Ser Leu Leu
        275                 280                 285

Glu Ala Leu Pro Gly Asp Asn Val Gly Phe Asn Val Lys Asn Val Ala
    290                 295                 300

Val Lys Asp Leu Lys Arg Gly Tyr Val Ala Ser Asn Ser Lys Asp Asp
305                 310                 315                 320

Pro Ala Lys Gly Ala Ala Asn Phe Thr Ser Gln Val Ile Ile Met Asn
                325                 330                 335

His Pro Gly Gln Ile Gly Asn Gly Tyr Ala Pro Val Leu Asp Cys His
            340                 345                 350

Thr Ser His Ile Ala Val Lys Phe Ser Glu Ile Leu Thr Lys Ile Asp
        355                 360                 365

Arg Arg Ser Gly Lys Glu Ile Glu Lys Glu Pro Lys Phe Leu Lys Asn
    370                 375                 380
```

```
Gly Asp Ala Gly Met Val Lys Met Thr Pro Thr Lys Pro Met Val Val
385                 390                 395                 400

Glu Thr Phe Ser Glu Tyr Pro Pro Leu Gly Arg Phe Ala Val Arg Asp
                405                 410                 415

Met Arg Gln Thr Val Ala Val Gly Val Ile Lys Ser Val Asp Lys Lys
            420                 425                 430

Asp Pro Thr Gly Ala Lys Val Thr Lys Ala Ala Val Lys Lys Gly Ala
        435                 440                 445

Lys
```

<210> 21
<211> 1200
<212> DNA
<213> Synechocystis

<400> 21

```
atggcacgcg caaaatttga acggacgaaa gaccacgtaa acattggcac cattggtcac     60
gttgaccacg gtaaaaccac cctaacggcg gcgatcacca tgaccttggc ggaattgggt    120
ggtgccaaag cccggaaata tgaagatatt gacgcggctc ctgaggaaaa agcccggggc    180
attaccatca atactgccca cgttgagtat gaaaccgata gccgtcacta tgcccacgta    240
gactgtcctg gtcacgctga ctatgtgaaa aacatgatca ccggtgctgc ccagatggac    300
ggtgcgattc tggtggtttc cgccgctgat ggccccatgc cccaaacccg ggaacacatt    360
cttttggcta agcaagtagg ggttcccaaa ctggtggtct ttttgaataa gaaagacatg    420
gtggacgacg aagaactgct ggaattggtg gaactggaag ttcgtgaatt gctcagcgac    480
tacgatttcc ccggtgatga cattcccatc gttgctggct ctgccctcaa ggcgatcgaa    540
ggagaaaaag aatacaaaga tgcgattctt gaactcatga aagccgttga cgactacatc    600
gacacccctg agcgggaagt tgataagccc ttcttgatgg cggtggaaga cgtattctcc    660
atcactggtc gtggtaccgt tgccaccggt cggattgagc gcggaaaagt taaagttggc    720
gaagaaattt ccatcgtcgg cattaaagat acccgtaaag ccactgttac cggtgtggaa    780
atgttccaaa aaacccttga agagggaatg gctggggaca acgtgggtct gctcctccgg    840
gggattcaaa aagaggacat cgaacggggt atggttttgg ctaaacccgg ttccatcact    900
ccccacaccg aatttgaagg ggaagtttac gtgctcaaga aagaagaagg tggccgccac    960
actcctttct ttgccaacta ccgccctcag ttctatgtac ggaccactga cgtaaccggt   1020
accatcaaaa gctacaccgc tgacgacggc agtgctgtgg aaatggttat gcctggggat   1080
cgtatcaaaa tgaccgttga gctcattaac ccgatcgcca ttgaacaggg gatgcgtttt   1140
gctatccgcg aaggtggtcg taccatcggc gccggtgttg tttctaagat tttgaagtag   1200
```

<210> 22
<211> 399
<212> PRT
<213> Synechocystis

<400> 22

```
Met Ala Arg Ala Lys Phe Glu Arg Thr Lys Asp His Val Asn Ile Gly
1               5                   10                  15

Thr Ile Gly His Val Asp His Gly Lys Thr Thr Leu Thr Ala Ala Ile
            20                  25                  30

Thr Met Thr Leu Ala Glu Leu Gly Gly Ala Lys Ala Arg Lys Tyr Glu
        35                  40                  45

Asp Ile Asp Ala Ala Pro Glu Glu Lys Ala Arg Gly Ile Thr Ile Asn
```

50 55 60

Thr Ala His Val Glu Tyr Glu Thr Asp Ser Arg His Tyr Ala His Val
65 70 75 80

Asp Cys Pro Gly His Ala Asp Tyr Val Lys Asn Met Ile Thr Gly Ala
85 90 95

Ala Gln Met Asp Gly Ala Ile Leu Val Val Ser Ala Ala Asp Gly Pro
100 105 110

Met Pro Gln Thr Arg Glu His Ile Leu Leu Ala Lys Gln Val Gly Val
115 120 125

Pro Lys Leu Val Val Phe Leu Asn Lys Lys Asp Met Val Asp Asp Glu
130 135 140

Glu Leu Leu Glu Leu Val Glu Leu Glu Val Arg Glu Leu Leu Ser Asp
145 150 155 160

Tyr Asp Phe Pro Gly Asp Asp Ile Pro Ile Val Ala Gly Ser Ala Leu
165 170 175

Lys Ala Ile Glu Gly Glu Lys Glu Tyr Lys Asp Ala Ile Leu Glu Leu
180 185 190

Met Lys Ala Val Asp Asp Tyr Ile Asp Thr Pro Glu Arg Glu Val Asp
195 200 205

Lys Pro Phe Leu Met Ala Val Glu Asp Val Phe Ser Ile Thr Gly Arg
210 215 220

Gly Thr Val Ala Thr Gly Arg Ile Glu Arg Gly Lys Val Lys Val Gly
225 230 235 240

Glu Glu Ile Ser Ile Val Gly Ile Lys Asp Thr Arg Lys Ala Thr Val
245 250 255

Thr Gly Val Glu Met Phe Gln Lys Thr Leu Glu Glu Gly Met Ala Gly
260 265 270

Asp Asn Val Gly Leu Leu Leu Arg Gly Ile Gln Lys Glu Asp Ile Glu
275 280 285

Arg Gly Met Val Leu Ala Lys Pro Gly Ser Ile Thr Pro His Thr Glu
290 295 300

Phe Glu Gly Glu Val Tyr Val Leu Lys Lys Glu Glu Gly Gly Arg His
305 310 315 320

Thr Pro Phe Phe Ala Asn Tyr Arg Pro Gln Phe Tyr Val Arg Thr Thr
325 330 335

Asp Val Thr Gly Thr Ile Lys Ser Tyr Thr Ala Asp Asp Gly Ser Ala
340 345 350

Val Glu Met Val Met Pro Gly Asp Arg Ile Lys Met Thr Val Glu Leu
355 360 365

```
Ile Asn Pro Ile Ala Ile Glu Gln Gly Met Arg Phe Ala Ile Arg Glu
    370                 375                 380

Gly Gly Arg Thr Ile Gly Ala Gly Val Val Ser Lys Ile Leu Lys
385                 390                 395
```

<210> 23
<211> 32
<212> PRT
<213> Chlamydomonas

<400> 23

```
Met Ala Met Ala Met Arg Ser Thr Phe Ala Ala Arg Val Gly Ala Lys
1               5                   10                  15

Pro Ala Val Arg Gly Ala Arg Pro Ala Ser Arg Met Ser Cys Met Ala
            20                  25                  30
```

<210> 24
<211> 32
<212> PRT
<213> Chlamydomonas

<400> 24

```
Met Gln Val Thr Met Lys Ser Ser Ala Val Ser Gly Gln Arg Val Gly
1               5                   10                  15

Gly Ala Arg Val Ala Thr Arg Ser Val Arg Arg Ala Gln Leu Gln Val
            20                  25                  30
```

<210> 25
<211> 52
<212> PRT
<213> Arabidopsis thaliana

<400> 25

```
Met Ala Ser Leu Met Leu Ser Leu Gly Ser Thr Ser Leu Leu Pro Arg
1               5                   10                  15

Glu Ile Asn Lys Asp Lys Leu Lys Leu Gly Thr Ser Ala Ser Asn Pro
            20                  25                  30

Phe Leu Lys Ala Lys Ser Phe Ser Arg Val Thr Met Thr Val Ala Val
        35                  40                  45

Lys Pro Ser Arg
    50
```

<210> 26
<211> 54
<212> PRT

<213> Arabidopsis thaliana

<400> 26

```
Met Ala Thr Gln Phe Ser Ala Ser Val Ser Leu Gln Thr Ser Cys Leu
1               5                   10                  15

Ala Thr Thr Arg Ile Ser Phe Gln Lys Pro Ala Leu Ile Ser Asn His
            20                  25                  30

Gly Lys Thr Asn Leu Ser Phe Asn Leu Arg Arg Ser Ile Pro Ser Arg
        35                  40                  45

Arg Leu Ser Val Ser Cys
    50
```

<210> 27
<211> 70
<212> PRT
<213> Arabidopsis thaliana

<400> 27

```
Met Ala Ser Ile Ala Ala Ser Ala Ser Ile Ser Leu Gln Ala Arg Pro
1               5                   10                  15

Arg Gln Leu Ala Ile Ala Ala Ser Gln Val Lys Ser Phe Ser Asn Gly
            20                  25                  30

Arg Arg Ser Ser Leu Ser Phe Asn Leu Arg Gln Leu Pro Thr Arg Leu
        35                  40                  45

Thr Val Ser Cys Ala Ala Lys Pro Glu Thr Val Asp Lys Val Cys Ala
    50                  55                  60

Val Val Arg Lys Gln Leu
65                  70
```

<210> 28
<211> 74
<212> PRT
<213> Arabidopsis thaliana

<400> 28

```
Met Ala Ser Ile Ala Thr Ser Ala Ser Thr Ser Leu Gln Ala Arg Pro
1               5                   10                  15

Arg Gln Leu Val Ile Gly Ala Lys Gln Val Lys Ser Phe Ser Tyr Gly
            20                  25                  30

Ser Arg Ser Asn Leu Ser Phe Asn Leu Arg Gln Leu Pro Thr Arg Leu
        35                  40                  45

Thr Val Tyr Cys Ala Ala Lys Pro Glu Thr Val Asp Lys Val Cys Ala
    50                  55                  60

Val Val Arg Lys Gln Leu Ser Leu Lys Glu
65                  70
```

<210> 29
<211> 1236
<212> DNA
<213> Oryza sativa

<400> 29

```
ggtcagccaa tacattgatc cgttgccaat catgcaaagt attttggctg tggccgagtg     60
ccggaattga taattgtgtt ctgactaaat taaatgacca gaagtcgcta tcttccaatg    120
tatccgaaac ctggattaaa caatcctgtt ctgttctcta gcccctcctg catggccgga    180
ttgttttttt gacatgtttt cttgactgag gcctgtttgt tctaaacttt ttcttcaaac    240
ttttaacttt ttcatcacat cagaactttt ctacacatat aaacttttaa cttttccgtc    300
acatcgttcc aatttcaatc aaactttcaa ttttggcgtg aactaaacac accctgagtc    360
ttttattgct cctccgtacg ggttggctgg ttgagaatag gtattttcag agagaaaatc    420
tagatattgg gaggaacttg gcatgaatgg ccactatatt tagagcaatt ctacggtcct    480
tgaggaggta ccatgaggta ccaaaatttt agtgtaaatt ttagtatctc attataacta    540
ggtattatga ggtaccaaat ttacaataga aaaaatagta cttcatggta ctttcttaag    600
taccgtaaaa ttgctcctat atttaagggg atgtttatat ctatccatat ccataatttg    660
attttgataa gaaaaaatgt gagcacacca agcatgtcca tgaccttgca ctcttggctc    720
actcgtcaac tgtgaagaac ctcaaaaatg ctcaatatag ctacaggtgc ctgaaaaaat    780
aactttaaag ttttgaacat cgatttcact aaacaacaat tattatctcc ctctgaaaga    840
tgatagttta gaactctaga atcattgtcg gcggagaaag taaattattt tccccaaatt    900
tccagctatg aaaaaaccct caccaaacac catcaaacaa gagttcacca aaccgcccat    960
gcggccatgc tgtcacgcaa cgcaccgcat tgcctgatgg ccgctcgatg catgcatgct   1020
tccccgtgca catatccgac agacgcgccg tgtcagcgag ctcctcgacc gacctgtgta   1080
gcccatgcaa gcatccaccc ccgccacgta cacccccctcc tcctccctac gtgtcaccgc   1140
tctctccacc tatatatgcc cacctggccc ctctcctccc atctccactt cacccgatcg   1200
cttcttcttc ttcttcgttg cattcatctt gctagc                              1236
```

## Description

The present invention relates generally to the field of molecular biology and concerns a method for increasing yield in a plant relative to corresponding wild type plants. More specifically, the present invention relates to a method for increasing seed yield under non-stressed conditions by preferentially increasing activity in a plastid of a translation elongation factor (EFTu) or a homologue thereof. The present invention also relates to plants having preferentially increased activity in a plastid of an EFTu or a homologue thereof, which plants have increased seed yield under non-stressed conditions relative to corresponding wild type plants grown under comparable conditions.

The ever-increasing world population and the dwindling supply of arable land available for agriculture fuel research towards improving the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits. A trait of particular economic interest is yield. Yield is normally defined as the measurable produce of economic value from a crop. This may be defined in terms of quantity and/or quality. Yield is directly dependent on several factors, for example, the number and size of the organs, plant architecture (for example, the number of branches), seed production and more. Root development, nutrient uptake and stress tolerance may also be important factors in determining yield. Crop yield may therefore be increased by optimizing one of the abovementioned factors. Seed yield is also a trait of particular economic interest with plant seeds being an important source of human and animal nutrition. Crops such as, corn, rice, wheat, canola and soybean account for over half of total human caloric intake, whether through direct consumption of the seeds themselves or through consumption of meat products raised on processed seeds. They are also a source of sugars, oils and many kinds of metabolites used in industrial processes. Seeds contain an embryo, the source of new shoots and roots after germination, and an endosperm, the source of nutrients for embryo growth, during germination and early growth of seedlings. The development of a seed involves many genes, and requires the transfer of metabolites from roots, leaves and stems into the growing seed. The endosperm, in particular, assimilates the metabolic precursors of carbohydrate polymers, oil and proteins and synthesizes them into storage macromolecules to fill out the grain. The ability to increase plant seed yield, whether through seed number, seed biomass, seed development, seed filling, or any other seed-related trait would have many applications in agriculture, and even many non-agricultural uses such as in the biotechnological production of substances such as pharmaceuticals, antibodies or vaccines.

It has now been found that preferentially increasing activity in a plastid of a translation elongation factor (EFTu) gives plants grown under non-stressed conditions increased seed yield relative to corresponding wild type plants.

In plants, protein synthesis occurs in three sub cellular compartments, namely the cytoplasm, plastids and mitochondria. The mechanisms responsible for protein synthesis in the cytoplasm, plastids and mitochondria are distinct from each other. Plant cells therefore contain three different types of ribosomes, three groups of transfer RNAs (tRNAs), and three sets of auxiliary factors for protein synthesis. Plastids and mitochondria are thought to have arisen through the endosymbiosis of ancient prokaryotic organisms. Consistent with this theory, the protein synthetic machinery in plastids and mitochondria is more closely related to bacterial systems than to the translation apparatus in the surrounding plant cell cytoplasm.

Translation elongation factors (EFTus) are essential components of protein synthesis playing a role in polypeptide elongation. EFTu interacts with aminoacyl tRNA and transports the codon-specific tRNA to the aminoacyl site on the ribosome (ribosomal A-site) during the translation elongation step. EFTu is encoded in the chloroplast of lower photosynthetic eukaryotes such as *Chlamydomonas* and *Euglena,* whereas in higher plants an evolutionary transfer of these genes occurred from the chloroplast to the nucleus. Several cDNA clones encoding chloroplast and other EFTus have been identified in a number of higher plants.

US published patent application US 2003/0044972 A1 in the name of Ristic *et al.* describes a heat shock protein with high homology to chloroplast elongation factor EFTu and the temporal and spatial expression of the heat shock protein in a plant organ or tissue to enhance tolerance to heat and drought in female reproductive organs.

Bhadula et al. (Planta (2001) 212: 359-366) show the heat-stress induced synthesis of EFTu in a heat-tolerant maize line.

While it is apparent from the prior art that EFTu plays a protective role during heat stress, it is not apparent from the prior art that EFTu would give any beneficial effects under non-stressed or normal growth conditions.

It has now surprisingly been found that preferentially increasing activity in a plastid of an EFTu or a homologue thereof gives plants grown under non-stressed conditions increased yield relative to wild type plants grown under corresponding conditions.

Reference herein to "wild type plants" is taken to mean any suitable control plant(s), the choice of which would be within the capabilities of a person skilled in the art and may include, for example, corresponding wild type plants or corresponding plants without the gene of interest. A "control plant" or "wild type plant" as used herein refers not only to whole plants, but also to plant parts, including seeds and seed parts.

Reference herein to "non-stressed conditions" is taken to mean growth/cultivation of a plant at any stage in its life cycle (from seed to mature plant and back to seed again) under normal growth conditions which include the everyday mild stresses that every plant encounters, but which does not include severe stress. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. One example of a severe stress that is specifically excluded is temperatures above 35°C as measured in the shade by a thermometer housed in an instrument shelter that is away from materials that may absorb heat and affect an accurate air temperature reading. Another example of a severe stress that is specifically excluded is drought stress, which is defined herein as a continuous increase in the degree of dryness over a period of seven days in comparison to a "normal" or average amount. Such normal or average amounts will vary from region to region.

According to the present invention, there is provided a method for increasing plant yield under normal growth conditions relative to corresponding wild type plants grown under comparable conditions, comprising the steps as defined in claim 1.

Advantageously, performance of the methods according to the present invention result in plants having increased seed yield, especially increased seed yield.

The term "increased yield" as defined herein is taken to mean an increase in any one or more of the following obtained under non-stressed conditions, each relative to corresponding wild type plants: (i) increased biomass (weight) of one or more parts of a plant, particularly aboveground (harvestable) parts, increased root biomass or increased biomass of any other part; (ii) increased seed yield, which may result from an increase in seed biomass (seed weight) and which may be an increase in the seed weight per plant or on an individual seed basis, and which increase in seed weight may be due to altered seed dimensions, such as seed length and/or seed width and/or seed area and/or seed perimeter; (iii) increased number of flowers (florets) per panicle, which is expressed as a ratio of number of filled seeds over number of primary panicles; (iv) increased seed fill rate (which is the number of filled seeds divided by the total number of seeds and multiplied by 100); (v) increased number of (filled) seeds; (vi) increased seed size; (vii) increased seed volume; (viii) increased harvest index, which is expressed as a ratio of the yield of harvestable parts, such as seeds, over the total biomass; and (ix) increased thousand kernel weight (TKW), which is extrapolated from the number of filled seeds counted and their total weight; an increased TKW may result from an increased seed size and/or seed weight. An increased TKW may also result from an increase in embryo size and/or endosperm size.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, among others. Taking rice as an example, a yield increase may be manifested by an increase in one or more of the following: number of plants per hectare or acre, number of panicles per plant, number of spikelets per panicle, number of flowers per panicle, increase in the seed filling rate, increase in thousand kernel weight, among others. An increase in yield may also result in modified architecture, or may occur because of modified architecture.

According to the invention, the methods result in plants having increased seed yield, such as defined in (ii) to (ix) above. Therefore, according to the present invention, there is provided a method for increasing seed yield in a plant grown under non-stressed conditions, which method comprises preferentially increasing activity in a plastid of an EFTu polypeptide or a homologue thereof as defined by the steps according to claim 1.

Since the transgenic plants have increased yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may even exhibit early flowering. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible. If the growth rate is sufficiently increased, it may allow for the sowing of further seeds of the same plant species (for example sowing and harvesting of rice plants followed by sowing and harvesting of further rice plants all within one conventional growing period). Similarly, if the growth rate is sufficiently increased, it may allow for the sowing of further seeds of different plants species (for example the sowing and harvesting of rice plants followed by, for example, the sowing and optional harvesting of soybean, potato or any other suitable plant). Harvesting additional times from the same rootstock in the case of some plants may also be possible. Altering the harvest cycle of a plant may lead to an increase in annual biomass production per acre (due to an increase in the number of times (say

in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods gives plants having an increased growth rate. Therefore, also provided is a method for increasing the growth rate of plants grown under non-stressed conditions, which method comprises preferentially increasing activity in a plastid of an EFTU-like polypeptide or a homologue thereof.

The abovementioned growth characteristics may advantageously be modified in any plant.

The term "plant" as used herein encompasses whole plants, ancestors and progeny of plants and plant parts (such as plant cells, seeds, shoots, stems, leaves, roots, flowers and tubers), tissues and organs, wherein each of the aforementioned comprise the gene/nucleic acid of interest. The term "plant" also encompasses suspension cultures, callus tissue, embryos, meristematic regions, gametophytes, sporophytes, pollen, and microspores, again wherein each of the aforementioned comprise the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acacia* spp.,Acer spp., *Actinidia* spp., *Aesculus* spp., *Agathis australis, Albizia amara, Alsophila tricolor, Andropogon* spp., *Arachis* spp, *Areca catechu, Astelia fragrans, Astragalus cicer, Baikiaea plurijuga, Betula* spp., *Brassica* spp., *Bruguiera gymnorrhiza, Burkea africana, Butea frondosa, Cadaba farinosa, Calliandra* spp, *Camellia sinensis, Canna indica, Capsicum* spp., *Cassia* spp., *Centroema pubescens, Chaenomeles* spp., *Cinnamomum cassia, Coffea arabica, Colophospermum mopane, Coronillia varia, Cotoneaster serotina, Crataegus* spp., *Cucumis* spp., *Cupressus* spp., *Cyathea dealbata, Cydonia oblonga, Cryptomeria japonica, Cymbopogon* spp., *Cynthea dealbata, Cydonia oblonga, Dalbergia monetaria, Davallia divaricata, Desmodium* spp., *Dicksonia squarosa, Diheteropogon amplectens, Dioclea* spp, *Dolichos* spp., *Dorycnium rectum, Echinochloa pyramidalis, Ehrartia* spp., *Eleusine coracana, Eragrestis* spp., *Erythrina* spp., *Eucalyptus* spp., *Euclea schimperi, Eulalia villosa, Fagopyrum* spp., *Feijoa sellowiana, Fragaria* spp., *Flemingia* spp, *Freycinetia banksii, Geranium thunbergii, Ginkgo biloba, Glycine javanica, Gliricidia* spp, *Gossypium hirsutum, Grevillea* spp., *Guibourtia coleosperma, Hedysarum* spp., *Hemarthia altissima, Heteropogon contortus, Hordeum vulgare, Hyparrhenia rufa, Hypericum erectum, Hyperthelia dissoluta, Indigo incamata, Iris* spp., *Leptarrhena pyrolifolia, Lespediza* spp., *Lettuca* spp., *Leucaena leucocephala, Loudetia simplex, Lotonus bainesii, Lotus* spp., *Macrotyloma axillare, Malus* spp., *Manihot esculenta, Medicago sativa, Metasequoia glyptostroboides, Musa sapientum, Nicotianum* spp., *Onobrychis* spp., *Ornithopus* spp., *Oryza* spp., *Peltophorum africanum, Pennisetum* spp., *Persea gratissima, Petunia* spp., *Phaseolus* spp., *Phoenix canariensis, Phormium cookianum, Photinia* spp., *Picea glauca, Pinus* spp., *Pisum sativum, Podocarpus totara, Pogonarthria fleckii, Pogonarthria squarrosa, Populus* spp., *Prosopis cineraria, Pseudotsuga menziesii, Pterolobium stellatum, Pyrus communis, Quercus* spp., *Rhaphiolepsis umbellata, Rhopalostylis sapida, Rhus natalensis, Ribes grossularia, Ribes* spp., *Robinia pseudoacacia, Rosa* spp., *Rubus* spp., *Salix* spp., *Schyzachyrium sanguineum, Sciadopitys verticillata, Sequoia sempervirens, Sequoiadendron giganteum, Sorghum bicolor, Spinacia* spp., *Sporobolus fimbriatus, Stiburus alopecuroides, Stylosanthos humilis, Tadehagi* spp, *Taxodium distichum, Themeda triandra, Trifolium* spp., *Triticum* spp., *Tsuga heterophylla, Vaccinium* spp., *Vicia* spp., *Vitis vinifera, Watsonia pyramidata, Zantedeschia aethiopica, Zea mays,* amaranth, artichoke, asparagus, broccoli, Brussels sprouts, cabbage, canola, carrot, cauliflower, celery, collard greens, flax, kale, lentil, oilseed rape, okra, onion, potato, rice, soybean, strawberry, sugar beet, sugarcane, sunflower, tomato, squash, tea and algae, amongst others. According to a preferred embodiment of the present invention, the plant is a crop plant such as soybean, sunflower, canola, alfalfa, rapeseed, cotton, tomato, potato or tobacco. Further preferably, the plant is a monocotyledonous plant, such as sugarcane. More preferably, the plant is a cereal, such as rice, maize, wheat, barley, millet, rye, sorghum or oats.

The activity of an EFTu polypeptide may be preferentially increased by increasing levels of the polypeptide (in a plastid). Alternatively, activity may also be increased when there is no change in levels of an EFTu, or even when there is a reduction in levels of an EFTu polypeptide. This may occur when the intrinsic properties of the polypeptide are altered, for example, by making mutant versions that are more active than the wild type polypeptide. Reference herein to "preferentially" increasing activity is taken to mean a targeted increase in activity in a plastid above that found in plastids of wild type plants under non-stressed conditions.

The activity may be preferentially increased in a plastid using techniques well known in the art, such as by targeting activity to the plastid using transit peptide sequences or by transformation of a plastid. Activity may be increased in any plastid, however, preferred is preferentially increasing activity in a chloroplast.

The term "EFTu (polypeptide) or a homologue thereof" as defined herein refers to a polypeptide comprising: (i) the following GTP-binding domains in any order GXXXXGK and DXXG and NKXD and S/L/K/Q A/G L/V/F, where X

is any amino acid; and (ii) EFTu domain ALMANPAIKR or a domain having in increasing order of preference at least 50%, 55%, 60%, 65%, 70%, 75%, 80%, 85%, 90% or 95% identity thereto and/or K D/G EE S/A.

An "EFTu polypeptide or a homologue thereof" may readily be identified using routine techniques well known in the art. The motifs defined above are highly conserved, thereby allowing a person skilled in the art to readily identify EFTu sequences falling within the definition above.

The plant EFTu polypeptide sequence represented by SEQ ID NO: 2, encoded by the nucleic acid of SEQ ID NO: 1, was found on the basis of homology to a transcription factor in *Drosophila.* Examples of plant-derived polypeptides falling under the definition of an "EFTu or a homologue thereof" include: SEQ ID NO: 4 from *Nicotiana tabacum*; SEQ ID NO: 6 from *Arabidopsis thaliana*; SEQ ID NO: 8 from *Nicotiana sylvestris*; SEQ ID NO: 10 a chloroplast translational elongation factor from rice; SEQ ID NO: 12 a mitochondrial elongation factor from *Arabidopsis thaliana*; SEQ ID NO: 14 a mitochondrial elongation factor from *Arabidopsis thaliana*; SEQ ID NO: 16 from *Oryza sativa*; SEQ ID NO: 18 from *Zea mays* (encoded by a nuclear gene for a mitochondrial product); SEQ ID NO: 20 from *Arabidopsis thaliana*; and SEQ ID NO: 22 *Synechocystis.*

The table below shows the percentage homology of various EFTu polypeptide sequences compared to the amino acid sequence represented by SEQ ID NO: 2 based on overall global sequence alignment. The percentage identity was calculated using an NCBI Align program with default parameters.

**Table 1:** Homology of EFTU-like protein sequences with SEQ ID NO: 2 based on overall global sequence alignment

| EFTu Source and Type | SEQ ID NO:/NCBI Accession Number | Align full length EMBOSS |
|---|---|---|
| Tobacco (EFTu B) | SEQ ID NO: 2 | |
| Tobacco (EFTu A) | SEQ ID NO: 4 M94204 | Identity: 439/489 (89.8%)<br># Similarity: 457/489 (93.5%) |
| Tobacco (EFTu B) | SEQ ID NO: 8 D11470 | # Identity: 485/485 (100.0%)<br># Similarity: 485/485 (100.0%) |
| *Arabidopsis* (EFTu A) | SEQ ID NO: 6 X52256 | Identity: 398/487 (81.7%)<br># Similarity: 434/487 (89.1%) |
| Rice (EFTu A) | SEQ ID NO: 10 AF145053 | |
| *Arabidopsis* (radicle up regulated EFTu) | S09152 | Identity: 398/487 (81.7%)<br># Similarity: 434/487 (89.1%) |
| Rice (EFTu A) | AF327413 | Identity: 367/488 (75.2%)<br># Similarity: 411/488 (84.2%) |
| *Arabidopsis* (tufA) | CAA36498.1 At4g20360 X52256 | |
| *Arabidopsis* (EFTu M-type - mitochondrial) | SEQ ID NO: 12 At4g02930 | Identity: 297/494 (60.1%)<br># Similarity: 346/494 (70.0%) |
| *Arabidopsis* (EFTu M-type - mitochondrial) | SEQ ID NO: 14 X89227<br>(close to At4g02930) | # Identity: 295/507 (58.2%)<br># Similarity: 347/507 (68.4%) |
| Rice (EFTu M-type - mitochondrial) | SEQ ID NO: 16 AF327062; XM_470417 | Identity: 287/500 (57.4%)<br># Similarity: 332/500 (66.4%) |
| *Arabidopsis* EFTu M-type - mitochondrial) | AL161495, X89227 | |
| Maize (EFTu M-type - mitochondrial) | SEQ ID NO: 18 AF264877 | Identity: 280/489 (57.3%)<br># Similarity: 333/489 (68.1%) |
| Pea (EFTu) | CAA74893 | Identity: 405/495 (81.8%)<br># Similarity: 445/495 (89.9%) |
| Soybean (tuf A) | CAA46864 | Identity: 409/492 (83.1%)<br># Similarity: 444/492 (90.2%) |

(continued)

| EFTu Source and Type | SEQ ID NO:/NCBI Accession Number | Align full length EMBOSS |
|---|---|---|
| Tomato (EFTu) | BT014591 | Identity: 438/486 (90.1%)<br># Similarity: 456/486 (93.8%) |
| Soybean (EFTu) | CAA61444 | Identity: 411/489 (84.0%)<br># Similarity: 440/489 (90.0%) |
| Pelargonium (EFTu) | AAK08141 | Identity: 398/487 (81.7%)<br># Similarity: 428/487 (87.9%) |

An assay may also be carried out to determine EFTu activity. A first step would involve isolating plastids (for example, chloroplasts), followed by obtaining purified EFTu for determination of the specific activity (GDP exchange). A person skilled in the art would readily be able to isolate plastids using techniques well known in the art. For an example of chloroplast isolation, see Olsson et al., (J Biol Chem. 2003 Nov 7:278(45): 44439-47). Similarly, a person skilled in the art would also readily be able to purify EFTu. As an example, see Stanzel et al., (Eur J Biochem. 1994 Jan 15:219 (1-2):435-9) for a method for the purification of total EFTu. Furthermore, a person skilled in the art may also readily be able to determine the specific activity of EFTu. See for example Zhang et al., (J. Bacteriol. 176, 1184-1187) for a [$^3$H] GDP exchange assay for the determination of the activity of recombinant pre-EFTu.

It is to be understood that sequences falling under the definition of "EFTu polypeptide or homologue thereof" are not to be limited to the sequences represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20 and SEQ ID NO: 22, but that any polypeptide meeting the criteria of comprising motifs: (i) the following GTP-binding domains in any order GXXXXGK and DXXG and NKXD and S/L/K/Q A/G L/V/F, where X is any amino acid; and (ii) EFTu domain ALMANPAIKR or a domain having 50% identity thereto and/or K D/G EE S/A may be suitable for use in the methods of the invention.

The nucleic acid encoding an EFTu polypeptide or a homologue thereof may be any natural or synthetic nucleic acid. An EFTu polypeptide or a homologue thereof as defined hereinabove is one that is encoded by an EFTu nucleic acid/gene. Therefore, the term "EFTu nucleic acid/gene" as defined herein is any nucleic acid/gene encoding an EFTu-like polypeptide or a homologue thereof as defined hereinabove. Examples of EFTu nucleic acids include those represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21. EFTu nucleic acids/genes and variants thereof may be suitable in practising the methods of the invention. Variant EFTu nucleic acid/genes include portions of an EFTu nucleic acid/gene and/or nucleic acids capable of hybridising with an EFTu nucleic acid/gene.

The term portion as defined herein refers to a piece of DNA comprising at least enough nucleotides to encode a protein comprising: (i) the following GTP-binding domains in any order GXXXXGK and DXXG and NKXD and S/L/K/Q A/G L/V/F, where X is any amino acid; and (ii) EFTu domain ALMANPAIKR or a domain having 50% identity thereto and/or K D/G EE S/A. A portion may be prepared, for example, by making one or more deletions to an EFTu nucleic add. The portions may be used in isolated form or they may be fused to other coding (or noncoding) sequences in order to, for example, produce a protein that combines several activities. When fused to other coding sequences, the resulting polypeptide produced upon translation could be bigger than that predicted for the EFTu fragment. Preferably, the functional portion is a portion of a nucleic acid as represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21.

Another variant EFTu nucleic acid/gene is a nucleic acid capable of hybridising under reduced stringency conditions, preferably under stringent conditions, with an EFTu nucleic acid/gene as hereinbefore defined, which hybridising sequence encodes a polypeptide comprising: (i) the following GTP-binding domains in any order GXXXXGK and DXXG and NKXD and S/L/K/Q A/G L/V/F, where X is any amino acid; and (ii) EFTu domain ALMANPAIKR or a domain having 50% identity thereto and/or K D/G EE S/A. Preferably, the hybridising sequence is one that is capable of hybridising to a nucleic acid as represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21 or to a portion of any of the aforementioned sequences as defined hereinabove.

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration, ionic strength and hybridisation buffer composition.

"Stringent hybridisation conditions" and "stringent hybridisation wash conditions" in the context of nucleic acid hybridisation experiments such as Southern and Northern hybridisations are sequence dependent and are different under different environmental parameters. The skilled artisan is aware of various parameters which may be altered during hybridisation and washing and which will either maintain or change the stringency conditions.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M. Formamide reduces the melting temperature of DNA-DNA and DNA-RNA duplexes with 0.6 to 0.7°C for each percent formamide, and addition of 50% formamide allows hybridisaton to be performed at 30 to 45°C, though the rate of hybridisation will be lowered. Base pair mismatches reduce the hybridisation rate and the thermal stability of the duplexes. On average and for large probes, the $T_m$ decreases about 1°C per % base mismatch. The $T_m$ may be calculated using the following equations, depending on the types of hybrids:

1. DNA-DNA hybrids (Meinkoth and Wahl, Anal. Biochem., 138: 267-284, 1984):

$$T_m = 81.5°C + 16.6 x \log[Na^+]^a + 0.41 x \%[G/C^b] - 500 x [L^c]^{-1} - 0.61 x \% \text{ formamide}$$

2. DNA-RNA or RNA-RNA hybrids:

$$T_m = 79.8 + 18.5 (\log_{10}[Na^+]^a) + 0.58 (\%G/C^b) + 11.8 (\%G/C^b)^2 - 820/L^c$$

3. oligo-DNA or oligo-RNA[d] hybrids:

For <20 nucleotides: $T_m = 2 (l_n)$
For 20-35 nucleotides: $T_m = 22 + 1.46 (l_n)$

[a] or for other monovalent cation, but only accurate in the 0.01-0.4 M range.
[b] only accurate for %GC in the 30% to 75% range.
[c] L = length of duplex in base pairs.
[d] Oligo, oligonucleotide; $l_n$, effective length of primer = 2×(no. of G/C)+(no. of A/T).

Note: for each 1 % formamide, the $T_m$ is reduced by about 0.6 to 0.7°C, while the presence of 6 M urea reduces the $T_m$ by about 30°C

Specificity of hybridisation is typically the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. Generally, suitable stringent conditions for nucleic acid hybridisation assays or gene amplification detection procedures are as set forth above. Conditions of greater or less stringency may also be selected. Generally, low stringency conditions are selected to be about 50°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. For example, stringent conditions are those that are at least as stringent as, for example, conditions A-L; and reduced stringency conditions are at least as stringent as, for example, conditions M-R. Non-specific binding may be controlled using any one of a number of known techniques such as, for example, blocking the membrane with protein containing solutions, additions of heterologous RNA, DNA, and SDS to the hybridisation buffer, and treatment with Rnase. Examples of hybridisation and wash conditions are listed in Table 2 below.

**Table 2:** Examples of hybridisation and wash conditions

| Stringency Condition | Polynucleotide Hybrid ± | Hybrid Length (bp) ‡ | Hybridization Temperature and Buffer † | Wash Temperature and Buffer† |
|---|---|---|---|---|
| A | DNA:DNA | > or equal to 50 | 65°C 1×SSC; or 42°C, 1×SSC and 50% formamide | 65°C; 0.3×SSC |
| B | DNA:DNA | <50 | Tb*; 1×SSC | Tb*; 1×SSC |
| C | DNA:RNA | > or equal to 50 | 67°C 1×SSC; or 45°C, 1×SSC and 50% formamide | 67°C; 0.3×SSC |
| D | DNA:RNA | <50 | Td*; 1×SSC | Td*; 1×SSC |
| E | RNA:RNA | > or equal to 50 | 70°C 1×SSC; or 50°C, 1×SSC and 50% formamide | 70°C; 0.3×SSC |
| F | RNA:RNA | <50 | Tf*; 1×SSC | Tf*; 1×SSC |
| G | DNA:DNA | > or equal to 50 | 65°C 4×SSC; or 45°C, 4×SSC and 50% formamide | 65°C; 1×SSC |
| H | DNA:DNA | <50 | Th*; 4 ×SSC | Th*; 4×SSC |
| I | DNA:RNA | > or equal to 50 | 67°C 4×SSC; or 45°C, 4×SSC and 50% formamide | 67°C; 1×SSC |
| J | DNA:RNA | <50 | Tj*; 4 ×SSC | Tj*; 4 ×SSC |
| K | RNA:RNA | > or equal to 50 | 70°C 4×SSC; or 40°C, 6×SSC and 50% formamide | 67°C; 1×SSC |
| L | RNA:RNA | <50 | Tl*; 2 ×SSC | Tl*; 2×SSC |
| M | DNA:DNA | > or equal to 50 | 50°C 4×SSC; or 40°C, 6×SSC and 50% formamide | 50°C; 2×SSC |
| N | DNA:DNA | <50 | Tn*; 6 ×SSC | Tn*; 6×SSC |
| O | DNA:RNA | > or equal to 50 | 55°C 4×SSC; or 42°C, 6×SSC and 50% formamide | 55°C; 2×SSC |
| P | DNA:RNA | <50 | Tp*; 6 ×SSC | Tp*; 6×SSC |
| Q | RNA:RNA | > or equal to 50 | 60°C 4×SSC; or 45°C, 6×SSC and 50% formamide | 60°C.; 2×SSC |

(continued)

| Stringency Condition | Polynucleotide Hybrid ± | Hybrid Length (bp) ‡ | Hybridization Temperature and Buffer † | Wash Temperature and Buffer† |
|---|---|---|---|---|
| R | RNA:RNA | <50 | Tr*; 4 ×SSC | Tr*; 4×SSC |

‡ The "hybrid length" is the anticipated length for the hybridising nucleic acid. When nucleic acids of known sequence are hybridised, the hybrid length may be determined by aligning the sequences and identifying the conserved regions described herein.

† SSPE (1×SSPE is 0.15M NaCl, 10mM $NaH_2PO_4$, and 1.25mM EDTA, pH7.4) may be substituted for SSC (1×SSC is 0.15M NaCl and 15mM sodium citrate) in the hybridisation and wash buffers; washes are performed for 15 minutes after hybridisation is complete. The hybridisations and washes may additionally include 5 × Denhardt's reagent, . 5-1.0% SDS, 100 μg/ml denatured, fragmented salmon sperm DNA, 0.5% sodium pyrophosphate, and up to 50% formamide.

* Tb-Tr: The hybridisation temperature for hybrids anticipated to be less than 50 base pairs in length should be 5-10°C less than the melting temperature $T_m$ of the hybrids; the $T_m$ is determined according to the above-mentioned equations.

± Also disclosed is the substitution of any one, or more DNA or RNA hybrid partners with either a PNA, or a modified nucleic acid.

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989).

The EFTu nucleic acid or variant thereof may be derived from any natural or artificial source. The nucleic acid/ gene or variant thereof may be isolated from a microbial source, such as bacteria, yeast or fungi, or from a plant, algae or animal source. This nucleic acid may be modified from its native form in composition and/or genomic environment through deliberate human manipulation. The nucleic acid is preferably of plant origin, whether from the same plant species (for example to the one in which it is to be introduced) or whether from a different plant species. The nucleic acid may be isolated from a dicotyledonous plant, preferably from the family Solanaceae, further preferably from the genus *Nicotianae,* more preferably from tobacco. Most preferably, the EFTu nucleic acid isolated from tobacco is represented by SEQ ID NO: 1 and the EFTu amino acid sequence is as represented by SEQ ID NO: 2. The nucleic acid of plant origin is preferably a plastidic nucleic acid (i.e. derived from a plastid). The plastidic nucleic acids have a closer relationship to bacterial nucleic acids than to non-plastidic nucleic acids. Therefore, the invention may also be performed using bacterial EFTu nucleic acids or variants thereof. The bacterial nucleic acids are targeted to a plastid, preferably to a chloroplast. Furthermore, mitochondria also share a common origin to the plastidic and bacterial nucleic acids and therefore the invention may also be performed using a mitochondrial EFTu nucleic acid or variant thereof which may be from animal or fungal origin. The mitochondrial nucleic acids are targeted to a plastid, preferably to a chloroplast. Despite being less closely related to a plastidic nucleic acid than bacterial or mitochondrial nucleic acid, a cytosolic nucleic acid may also be suitable for use in the methods of the invention so long as the nucleic acid is targeted to a plastid, preferably to a chloroplast.

Methods for targeting to plastids are well known in the art and include the use of transit peptides. Table 3 below shows examples of transit peptides which can be used to target any EFTu protein to a plastid, which EFTu is not, in its natural form, normally targeted to a plastid, or which EFTu in its natural form is targeted to a plastid by virtue of a different transit peptide (for example, its natural transit peptide).

**Table 3:** Examples of transit peptide sequences useful in targeting amino acids to plastids

| NCBI Accession Number/SEQ ID NO | Source Organism | Protein Function | Transit Peptide Sequence |
|---|---|---|---|
| SEQ ID NO: P07839 | *Chlamydomonas* | Ferredoxin | MAMAMRSTFAARVGAKPAVRGARPASRMSCMA |
| SEQ ID NO: AAR23425 | *Chlamydomonas* | Rubisco activase | MQVTMKSSAVSGQRVGGARVATRSVRRAQLQV |
| SEQ ID NO: CAA56932 | *Arabidopsis thaliana* | asp Amino transferase | MASLMLSLGSTSLLPREINKDKLKLGTSASNPFLKAKSFSRVT MTVAVKPSR |
| SEQ ID NO: CAA31991 | *Arabidopsis thaliana* | Acyl carrier protein1 | MATQFSASVSLQTSCLATTRISFQKPALISNHGKTNLSFNLRR SIPSRRLSVSC |
| SEQ ID NO: CAB63798 | *Arabidopsis thaliana* | Acyl carrier protein2 | MASIAASASISLQARPRQLAIAASQVKSFSNGRRSSLSFNLRQ LPTRLTVSCAAKPETVDKVCAVVRKQL |
| SEQ ID NO: CAB63799 | *Arabidopsis thaliana* | Acyl carrier protein3 | MASIATSASTSLQARPRQLVIGAKQVKSFSYGSRSNLSFNLR QLPTRLTVYCAAKPETVDKVCAVVRKQLSLKE |

The activity of an EFTu polypeptide or a homologue thereof may be increased by introducing a genetic modification (preferably in the locus of an EFTu gene). The locus of a gene as defined herein is taken to mean a genomic region, which includes the gene of interest and 10KB up- or downstream of the coding region.

The genetic modification may be introduced, for example, by any one (or more) of the following methods: TDNA activation, TILLING, site-directed mutagenesis, directed evolution, homologous recombination and by introducing and expressing in a plant cell a nucleic acid encoding an EFTu polypeptide or a homologue thereof (the gene product may then be targeted to a plastid in the plant cell, unless the gene being expressed is a plastidic gene). Following introduction of the genetic modification, there follows a step of selecting for increased activity of an EFTu polypeptide, which increase in activity gives plants having increased yield.

T-DNA activation tagging (Hayashi et al. Science (1992) 1350-1353) involves insertion of T-DNA usually containing a promoter (may also be a translation enhancer or an intron), in the genomic region of the gene of interest or 10KB up- or down stream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to overexpression of genes near to the inserted T-DNA. The resulting transgenic plants show dominant phenotypes due to overexpression of genes dose to the introduced promoter. The promoter to be introduced may be any promoter capable of directing expression of a gene in the desired organism, in this case a plant. For example, constitutive, tissue-preferred, cell type-preferred and inducible promoters are all suitable for use in T-DNA activation. Preferred is the use of a seed-specific promoter, more particularly an embryo- and/or alerone-specific promoter.

A genetic modification may also be introduced in the locus of an EFTu gene using the technique of TILLING (Targeted Induced Local Lesions IN Genomes). This is a mutagenesis technology useful to generate and/or identify, and to eventually isolate mutagenised variants of an EFTu nucleic acid capable of exhibiting EFTu activity. TILLING also allows selection of plants carrying such mutant variants. These mutant variants may even exhibit higher EFTu activity than that exhibited by the gene in its natural form. TILLNG combines high-density mutagenesis with high-throughput screening methods. The steps typically followed in TILLING are: (a) EMS mutagenesis (Redei and Koncz, 1992; Feldmann *et al.,* 1994; Lightner and Caspar, 1998); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum Nat Biotechnol. 2000 Apr; 18(4):455-7, reviewed by Stemple 2004 (TILLING-a high-throughput harvest for functional genomics. Nat. Rev. Genet. 2004 Feb; 5(2):145-50.)).

Site-directed mutagenesis may be used to generate variants of EFTu nucleic acids. Several methods are available to achieve site-directed mutagenesis, the most common being PCR-based methods (current protocols in molecular biology. Wiley Eds. http://www.4ulr.com/products/currentprotocols/index.html).

Directed evolution (or gene shuffling) may also be used to generate variants of EFTu nucleic acids. This consists of iterations of DNA shuffling followed by appropriate screening and/or selection to generate and identify variants having an modified biological activity (Castle et al., (2004) Science 304(5674): 1151-4; US patents 5,811,238 and 6,395,547).

TDNA activation, TILLING, directed evolution and site-directed mutagenesis are examples of technologies that enable the generation of novel alleles and EFTu variants.

Homologous recombination allows introduction in a genome of a selected nucleic acid at a defined selected position. Homologous recombination is a standard technology used routinely in biological sciences for lower organisms such as yeast or the moss physcomitrella. Methods for performing homologous recombination in plants have been described not only for model plants (Offringa et al. Extrachromosomal homologous recombination and gene targeting in plant cells after Agrobacterium-mediated transformation. 1990 EMBO J. 1990 Oct; 9(10):3077-84) but also for crop plants, for example rice (Terada R, Urawa H, Inagaki Y, Tsugane K, Iida S. Efficient gene targeting by homologous recombination in rice. Nat Biotechnol. 2002. Iida and Terada: A tale of two integrations, transgene and T-DNA: gene targeting by homologous recombination in rice. Curr Opin Biotechnol. 2004 Apr; 15(2):132-8). The nucleic acid to be targeted (which may be an EFTu nucleic acid or variant thereof as hereinbefore defined) need not be targeted to the locus of an EFTu gene, but may be introduced in, for example, regions of high expression. The nucleic acid to be targeted may be an improved allele used to replace the endogenous gene or may be introduced in addition to the endogenous gene.

Plant seed yield may be increased in plants grown under non-stressed conditions by introducing and expressing in a plant, plant part or plant cell a nucleic acid encoding an EFTu polypeptide or a homologue thereof. The polypeptide may then be targeted to a plastid in the plant cell, unless the gene being expressed is a plastidic gene. An EFTu polypeptide or a homologue thereof as mentioned above is one comprising: (i) the following GTP-binding domains in any order GXXXXGK and DXXG and NKXD and S/L/K/Q A/G L/V/F, where X is any amino acid; and (ii) EFTu domain ALMANPAIKR or a domain having 50% identity thereto and/or K D/G EE S/A. The nucleic acid to be introduced into a plant may be a full-length nucleic acid or may be a portion or a hybridizing sequence as hereinbefore defined.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (such as similar hydrophobicity, hydrophilicity, antigenicity, propensity to form or break α-helical structures or β-sheet structures). Conservative substitution tables are well known in the art (see for example Creighton (1984) Proteins. W.H. Freeman and Company and Table 1 above).

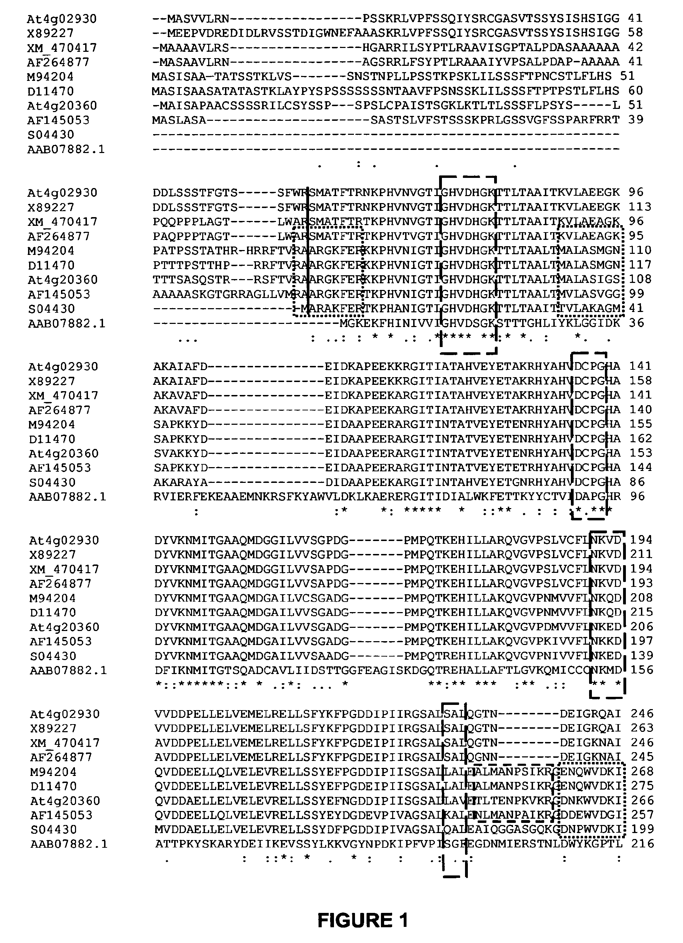

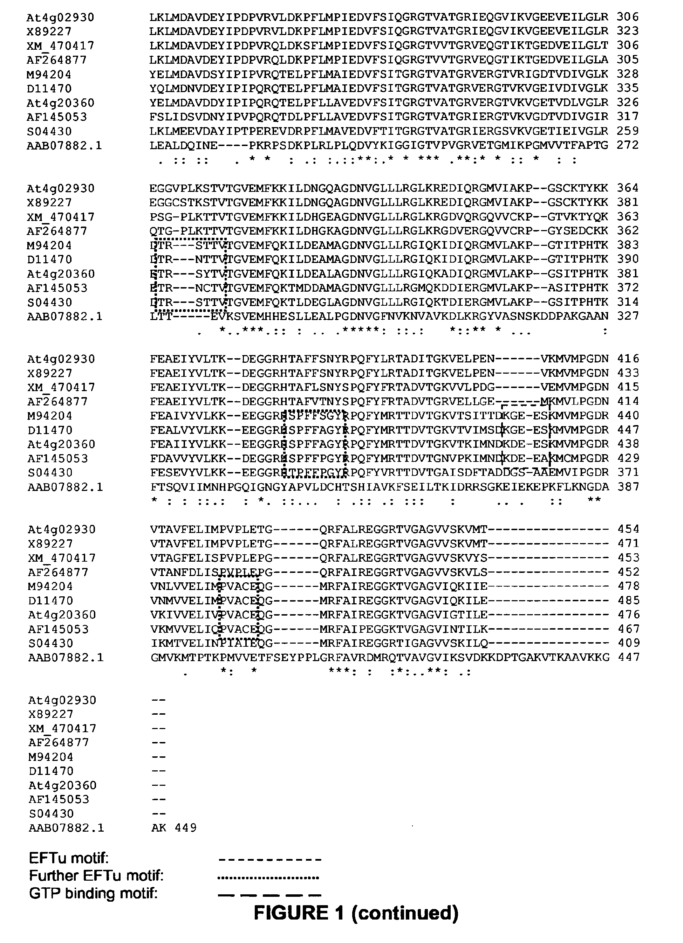

According to a preferred feature, the homologue has in increasing order of preference at least 45%, 50%, 55%, 60%, 65%, 70%, 75%, 80%, 85%, 90% or 95% sequence identity to the amino acid sequence represented by SEQ ID NO: 2. Whether a polypeptide has at least 45%, 50%, 55%, 60%, 65%, 70%, 75%, 80%, 85%, 90% or 95% identity to the amino acid represented by SEQ ID NO: 2 may readily be established by sequence alignment. Methods for the alignment of sequences for comparison are well known in the art, such methods include GAP, BESTFIT, BLAST, FASTA and TFASTA. GAP uses the algorithm of Needleman and Wunsch (J. Mol. Biol. 48: 443-453, 1970) to find the alignment of two complete sequences that maximises the number of matches and minimises the number of gaps. The BLAST algorithm calculates percent sequence identity and performs a statistical analysis of the similarity between the two sequences. The software for performing BLAST analysis is publicly available through the National Centre for Biotechnology Information. An EFTu polypeptide or a homologue thereof having at least 45%, 50%, 55%, 60%, 65%, 70%, 75%, 80%, 85%, 90% or 95% identity to the amino acid represented by SEQ ID NO: 2 may readily be identified by aligning a query sequence (preferably a protein sequence) with known EFTu protein sequences (see for example the alignment shown in Figure 1). The query sequence may be aligned (with known EFTU-like sequences) using, for example, the VNTI AlignX multiple alignment program, based on a modified clustal W algorithm (InforMax, Bethesda, MD, http: //www.informaxinc.com), with default settings for gap opening penalty of 10 and a gap extension of 0.05.

Also encompassed by the term "homologues" are two special forms of homology, which include orthologous sequences and paralogous sequences, which encompass evolutionary concepts used to describe ancestral relationships of genes. The term "paralogous" relates to gene-duplications within the genome of a species leading to paralogous genes. The term "orthologous" relates to homologous genes in different organisms due to speciation.

Othologues in, for example, monocot plant species may easily be found by performing a so-called reciprocal blast search. This may be done by a first blast involving blasting a query sequence (for example, SEQ ID NO: 1 or SEQ ID NO: 2) against any sequence database, such as the publicly available NCBI database which may be found at: http: //www.ncbi.nlm.nih.gov. BLASTN or TBLASTX (using standard default values) may be used when starting from a nucleotide sequence and BLASTP or TBLASTN (using standard default values) may be used when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2 the second blast would therefore be against tobacco sequences). The results of the first and second BLASTs are then compared. A paralogue is identified if a high-ranking hit from the second blast is from the same species as from which the query sequence is derived; an orthologue is identified if a high-ranking hit is not from the same species as from which the query sequence is derived. High-ranking hits are those having a low E-value. The lower the E-value, the more signficant the score (or in other words the lower the chance that the hit was found by chance). Computation of the E-value is well known in the art. In the case of large families, ClustalW may be used, followed by a neighbour joining tree, to help visualize clustering of related genes and to identify orthologues and paralogues.

A homologue may be in the form of a "substitutional variant" of a protein, i.e. where at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues. Preferably, amino acid substitutions comprise conservative amino acid substitutions.

A homologue may also be in the form of an "insertional variant" of a protein, i.e. where one or more amino acid residues are introduced into a predetermined site in a protein. Insertions may comprise amino-terminal and/or carboxy-terminal fusions as well as intra-sequence insertions of single or multiple amino acids. Generally, insertions within the amino acid sequence will be smaller than amino- or carboxy-terminal fusions, of the order of about 1 to 10 residues. Examples of amino- or carboxy-terminal fusion proteins or peptides include the binding domain or activation domain of a transcriptional activator as used in the yeast two-hybrid system, phage coat proteins, (histidine)6-tag, glutathione S-transferase-tag, protein A, maltose-binding protein, dihydrofolate reductase, Tag 100 epitope, c-myc epitope, FLAG®-epitope, lacZ, CMP (calmodulin-binding peptide), HA epitope, protein C epitope and VSV epitope.

Homologues in the form of "deletion variants" of a protein are characterised by the removal of one or more amino acids from a protein.

Amino acid variants of a protein may readily be made using peptide synthetic techniques well known in the art, such as solid phase peptide synthesis and the like, or by recombinant DNA manipulations. Methods for the manipulation

of DNA sequences to produce substitution, insertion or deletion variants of a protein are well known in the art. For example, techniques for making substitution mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols.

The EFTu polypeptide or homologue thereof may be a derivative. "Derivatives" include peptides, oligopeptides, polypeptides, proteins and enzymes which may comprise substitutions, deletions or additions of naturally and non-naturally occurring amino acid residues compared to the amino acid sequence of a naturally-occurring form of the protein, for example, as presented in SEQ ID NO: 2. "Derivatives" of a protein encompass peptides, oligopeptides, polypeptides, proteins and enzymes which may comprise naturally occurring altered, glycosylated, acylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

The EFTu polypeptide or homologue thereof may be encoded by an alternative splice variant of an EFTu nucleic acid/gene. The term "alternative splice variant" as used herein encompasses variants of a nucleic acid sequence in which selected introns and/or exons have been excised, replaced or added. Such variants will be ones in which the biological activity of the protein is retained, which may be achieved by selectively retaining functional segments of the protein. Such splice variants may be found in nature or may be manmade. Methods for making such splice variants are well known in the art. Preferred splice variants are splice variants of the nucleic acid represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21. Splice variants useful in the methods of the invention are those encoding polypeptides comprising: (i) the following GTP-binding domains in any order GXXXXGK and DXXG and NKXD and S/L/K/Q A/G L/V/F, where X is any amino acid; and (ii) EFTu domain ALMANPAIKR or a domain having 50% identity thereto and/or K D/G EE S/A.

The homologue may also be encoded by an allelic variant of a nucleic acid encoding an EFTu polypeptide or a homologue thereof, preferably an allelic variant of the nucleic acid represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21. Useful in the methods of the invention are allelic variants encoding polypeptides which comprise: (i) the following GTP-binding domains in any order GXXXXGK and DXXG and NKXD and S/L/K/Q A/G L/V/F, where X is any amino acid; and (ii) EFTu domain ALMANPAIKR or a domain having 50% identity thereto and/or K D/G EE S/A. Allelic variants exist in nature and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

As disclosed, enhanced or increased expression of the EFTu nucleic acid or variant thereof is envisaged. Methods for obtaining enhanced or increased expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of an EFTu nucleic acid or variant thereof. For example, endogenous promoters may be altered *in vivo* by mutation, deletion, and/or substitution (see, Kmiec, U.S. Pat. No. 5,565,350; Zarling et al., PCT/US93/03868), or isolated promoters may be introduced into a plant cell in the proper orientation and distance from a gene so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene. Methods for preferentially increasing activity of an EFTu polypeptide in a plastid are described hereinabove.

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold, Buchman and Berg, Mol. Cell biol. 8:4395-4405 (1988); Callis et al., Genes Dev. 1:1183-1200 (1987). Such intron enhancement of gene expression is typically greatest when placed near the 5' end of the transcription unit. Use of the maize introns Adh1-S intron 1, 2, and 6, the Bronze-1 intron are known in the art. See generally, The Maize Handbook, Chapter 116, Freeling and Walbot, Eds., Springer, N.Y. (1994).

Also disclosed are genetic constructs and vectors to facilitate introduction and/or expression of the nucleotide sequences useful in the methods according to the invention.

Therefore, there is provided a gene construct comprising:

(i) An EFTu nucleic acid or variant thereof encoding a polypeptide comprising: (a) the following GTP-binding domains in any order GXXXXGK and DXXG and NKXD and S/UK/Q A/G L/V/F, where X is any amino acid; and (b) EFTu domain ALMANPAIKR or a domain having 50% identity thereto and/or K D/G EE S/A; and
(ii) One or more control sequences capable of driving expression of the nucleic acid sequence of (i), preferably wherein the one or more control sequences comprise at least a seed-specific promoter; and optionally
(iii) A transcription termination sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells.

Plants are transformed with a vector comprising the sequence of interest (i.e., an EFTu nucleic acid or variant thereof). The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative which confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence, however our studies have revealed that some promoters outperform others in the methods of the invention. The promoter is preferrably a tissue-specific promoter, i.e. one that is capable of predominantly initiating transcription in certain tissues, such as the leaves, roots, seed tissue etc., substantially to the exclusion of initiating transcription elsewhere in the plant, but whilst still allowing for residual expression in other parts of a plant due to leaky promoters.

Our studies have revealed that use of seed-specific promoters, more particularly embryo-specific and/or alerone-specific promoters, perform better in the methods of the invention (i.e. give plants having better yield) than constitutive promoters in the same methods. It is therefore preferred that the EFTu nucleic acid or variant thereof is operably linked to a seed-specific promoter. A seed-specific promoter is transcriptionally active predominantly in seed tissue, but not necessarily exclusively in seed tissue (in case of leaky expression). Seed-specific promoters are well known in the art. Preferably, the seed-specific promoter is an embryo-specific and/or aleurone-specific promoter, more preferably an oleosin promoter (see for example, SEQ ID NO: 29 which represents the sequence of the oleosin promoter from rice). It should be clear that the applicability of the disclosed method is not restricted to the EFTu-like nucleic acid represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21, nor is the applicability of the disclosed method restricted to expression of an EFTu nucleic acid when driven by an oleosin promoter.

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences which may be suitable for use in performing the invention. Such sequences would be known or may readily be obtained by a person skilled in the art.

The genetic constructs of the invention may further include an origin of replication sequence which is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene that confers a phenotype on a cell in which it is expressed to facilitate the identification and/or selection of cells that are transfected or transformed with a nucleic acid construct of the invention. Suitable markers may be selected from markers that confer antibiotic or herbicide resistance, that introduce a new metabolic trait or that allow visual selection. Examples of selectable marker genes include genes conferring resistance to antibiotics (such as

nptII that phosphorylates neomycin and kanamycin, or hpt, phosphorylating hygromycin), to herbicides (for example bar which provides resistance to Basta; aroA or gox providing resistance against glyphosate), or genes that provide a metabolic trait (such as manA that allows plants to use mannose as sole carbon source). Expression of visual marker genes results in the formation of colour (for example β-glucuronidase, GUS), luminescence (such as luciferase) or fluorescence (Green Fluorescent Protein, GFP, and derivatives thereof).

Further disclosed are plants and plant parts obtainable by the methods according to the present invention which plants or plant parts comprise an EFTu nucleic acid or variant thereof (transgene).

The invention also provides a method for the production of transgenic plants having increased seed yield under non-stressed conditions, comprising introduction and expression in a plant or plant part (including plant cell) of an EFTu nucleic acid or a variant thereof as defined in claim 1. The gene product expression of the nucleic acid is targeted to a plastid in a plant cell if it is not already in the plastid.

More specifically, the present invention provides a method for the production of transgenic plants having increased seed yield under non-stressed conditions, which method comprises:

(i) introducing and expressing in a plant or plant part (including plant cell) an EFTu nucleic acid or variant thereof encoding a polypeptide comprising: (a) the following GTP-binding domains in any order GXXXXGK and DXXG and NKXD and S/L/K/Q A/G L/V/F, where X is any amino acid; and (b) EFTu domain ALMANPAIKR or a domain having 50% identity thereto and/or K D/G EE S/A, which the polypeptide is targeted to a plastid in a plant cell if it is not already in the plastid; and

(ii) cultivating the plant or plant part under non-stress growth conditions promoting plant growth and development.

The nucleic acid may be introduced directly into a plant cell or into the plant itself (including introduction into a tissue (such as a plastid), organ or any other part of a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., 1882, Nature 296, 72-74; Negrutiu I. et al., June 1987, Plant Mol. Biol. 8, 363-373); electroporation of protoplasts (Shillito R.D. et al., 1985 Bio/Technol 3, 1099-1102); microinjection into plant material (Crossway A. et al., 1986, Mol. Gen Genet 202, 179-185); DNA or RNA-coated particle bombardment (Klein T.M. et al., 1987, Nature 327, 70) infection with (non-integrative) viruses and the like. Transgenic rice plants expressing an EFTu nucleic acid/gene are preferably produced via *Agrobacterium*-mediated transformation using any of the well known methods for rice transformation, such as described in any of the following: published European patent application EP 1198985 A1, Aldemita and Hodges (Planta, 199, 612-617, 1996); Chan et al. (Plant Mol. Biol. 22 (3) 491-506, 1993), Hiei et al. (Plant J. 6 (2) 271-282, 1994), which disclosures are incorporated by reference herein as if fully set forth. In the case of corn transformation, the preferred method is as described in either Ishida et al. (Nat. Biotechnol. 1996 Jun; 14(6): 745-50) or Frame et al. (Plant Physiol. 2002 May; 129(1): 13-22), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding

techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention, relates to any plant cell or plant produced by any of the methods described herein, and to all plant parts and propagules thereof. The present invention relates to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to the invention. The invention also relates to host cells containing an isolated EFTu nucleic acid or variant thereof. Preferred host cells according to the invention are plant cells.

The invention relates to harvestable parts of a plant, such as but not limited to seeds, leaves, fruits, flowers, stem cultures, rhizomes, tubers and bulbs. The invention further relates to products derived (preferably directly) from a harvestable part of such a plant, such products including dry pellets or powders, oil, fat and fatty acids, starch or proteins.

Also disclosed are EFTu nucleic acids or variants thereof and to the use of EFTu polypeptides or homologues thereof in increasing seed yield, in plants grown under non-stress conditions. The seed yield is as defined hereinabove.

EFTu nucleic acids or variants thereof, or EFTu polypeptides or homologues thereof may find use in breeding programmes in which a DNA marker is identified which may be genetically linked to an EFTu gene or variant thereof. The EFTu nucleic acids/ genes or variants thereof, or EFTu polypeptides or homologues thereof may be used to define a molecular marker. This DNA or protein marker may then be used in breeding programs to select plants having increased yield. The EFTu gene or variant thereof may, for example, be a nucleic acid as represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21.

Allelic variants of an EFTu nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place by, for example, PCR. This is followed by a selection step for selection of superior allelic variants of the sequence in question and which give increased plant yield. Selection is typically carried out by monitoring growth performance of plants containing different allelic variants of the sequence in question, for example, different allelic variants of any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19 and SEQ ID NO: 21. Growth performance may be monitored in a greenhouse or in the field. Further optional steps include crossing plants, in which the superior allelic variant was identified, with another plant. This could be used, for example, to make a combination of interesting phenotypic features.

An EFTu nucleic acid or variant thereof may also be used as probes for genetically and physically mapping the genes that they are a part of, and as markers for traits linked to those genes. Such information may be useful in plant breeding in order to develop lines with desired phenotypes. Such use of EFTu nucleic acids or variants thereof requires only a nucleic acid sequence of at least 15 nucleotides in length. The EFTu nucleic acids or variants thereof may be used as restriction fragment length polymorphism (RFLP) markers. Southern blots (Maniatis) of restriction-digested plant genomic DNA may be probed with the EFTu nucleic acids or variants thereof. The resulting banding patterns may then be subjected to genetic analyses using computer programs such as MapMaker (Lander et al. (1987) Genomics 1:174-181) in order to construct a genetic map. In addition, the nucleic acids may be used to probe Southern blots containing restriction endonudease-treated genomic DNAs of a set of individuals representing parent and progeny of a defined genetic cross. Segregation of the DNA polymorphisms is noted and used to calculate the position of the EFTu nucleic acid or variant thereof in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4:37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

Nuclic acid probes may be used in direct fluorescence *in situ* hybridization (FISH) mapping (Trask (1991) Trends Genet. 7:149-154). Although current methods of FISH mapping favor use of large clones (several to several hundred KB; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Performance of the methods according to the present invention result in plants having increased seed yield. The trait of increased seed yield may be combined with other economically advantageous traits, such as further yield-enhancing traits, tolerance to various stresses, traits modifying various architectural features and/or biochemical and/or physiological features.

**Description of figures**

The present invention will now be described with reference to the following figures in which:

**Fig. 1** shows a CLUSTAL W multiple alignment of several plant EFTu polypeptides. The EFTu motif is represented by

a further EFTu motif is represented by

and a GTP binding motif is represented by

**Fig. 2** shows a binary vector for expression in *Oryza sativa* of a tobacco EFTu under the control of an oleosin promoter.

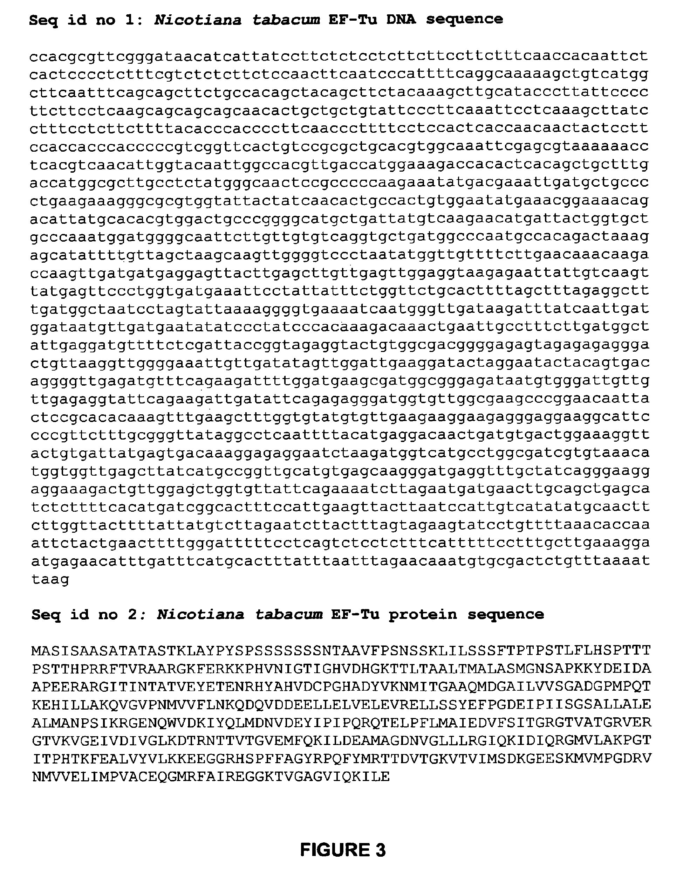

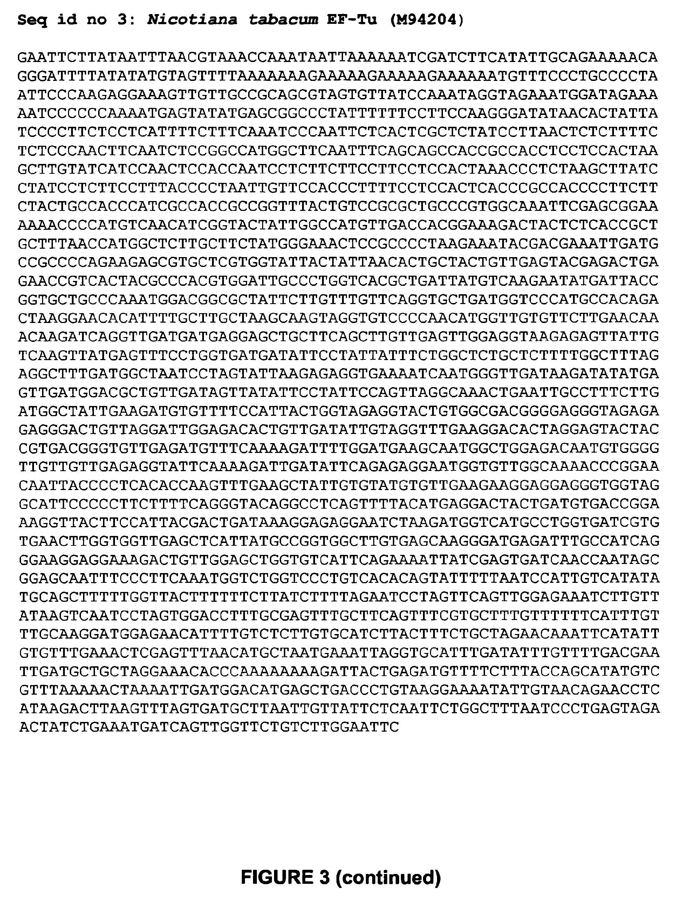

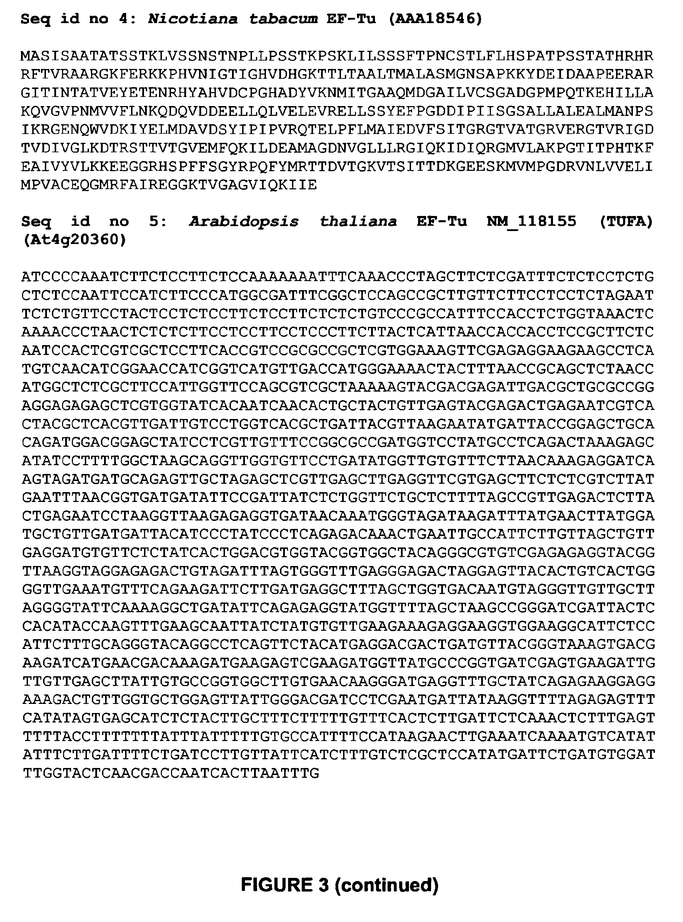

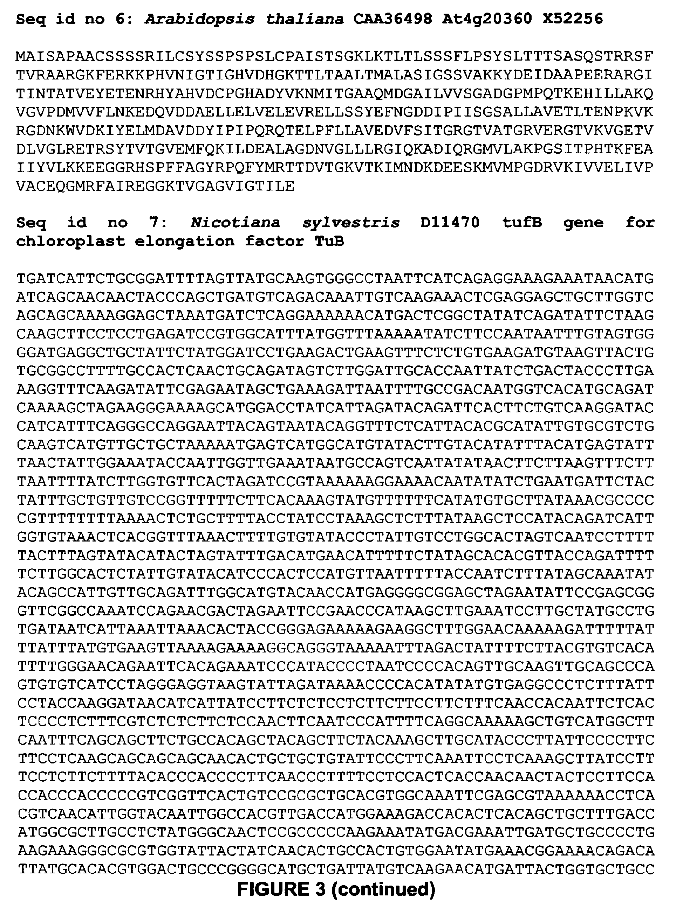

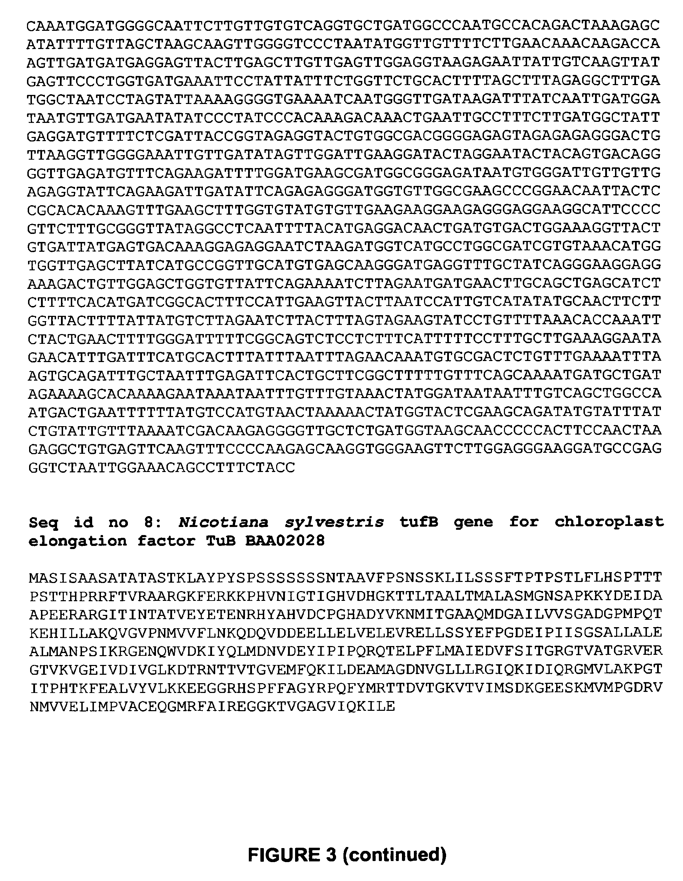

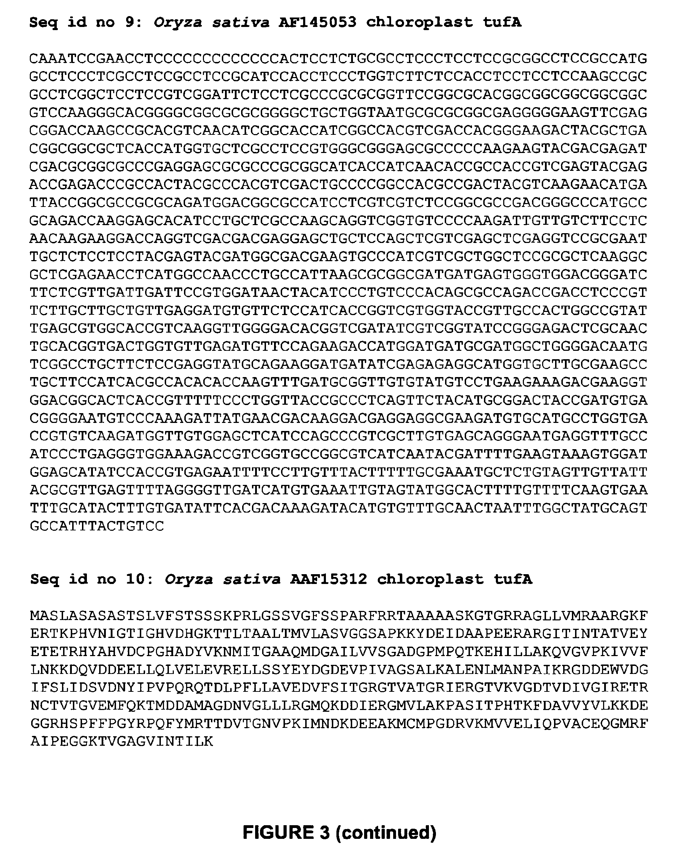

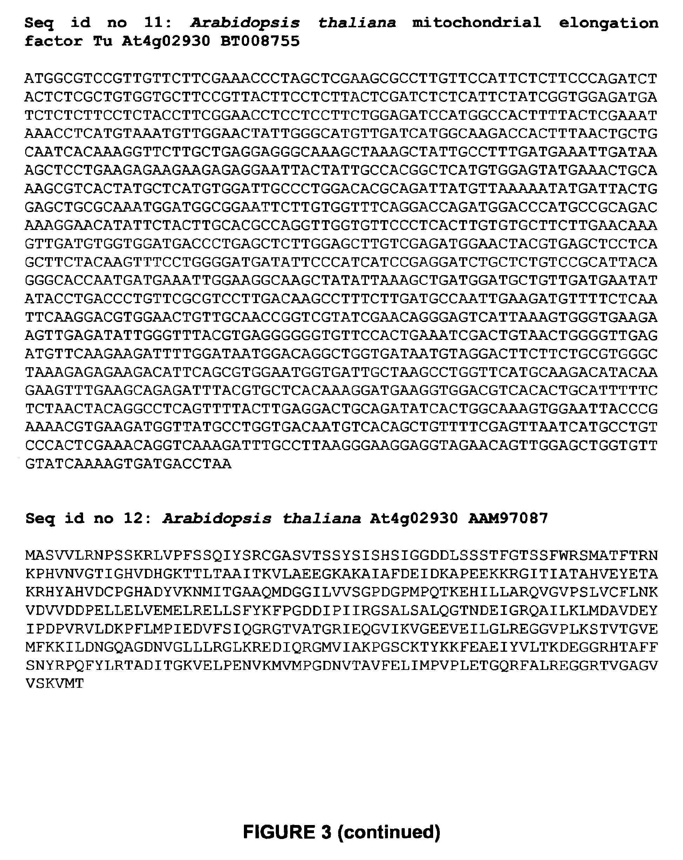

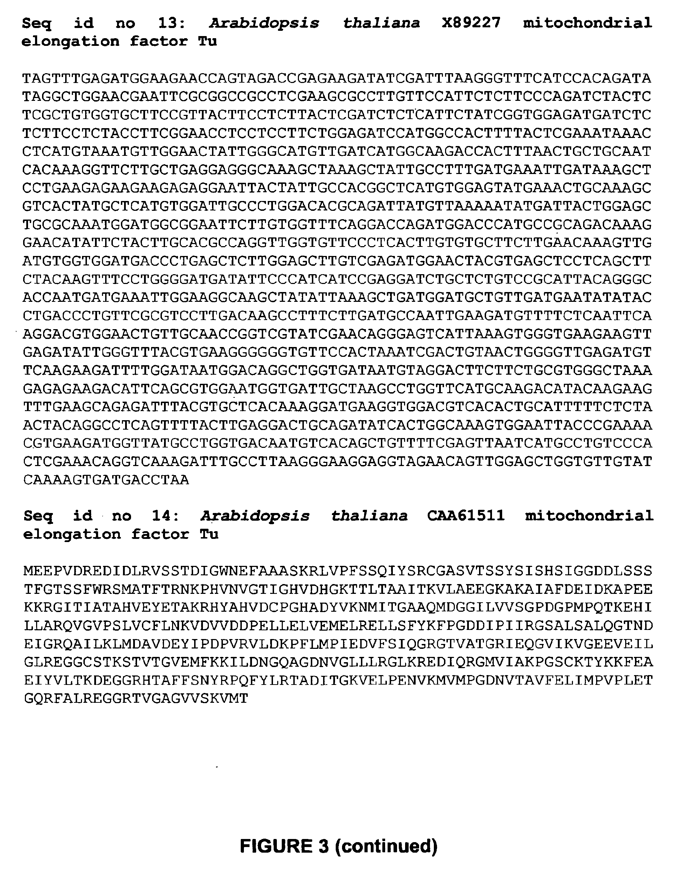

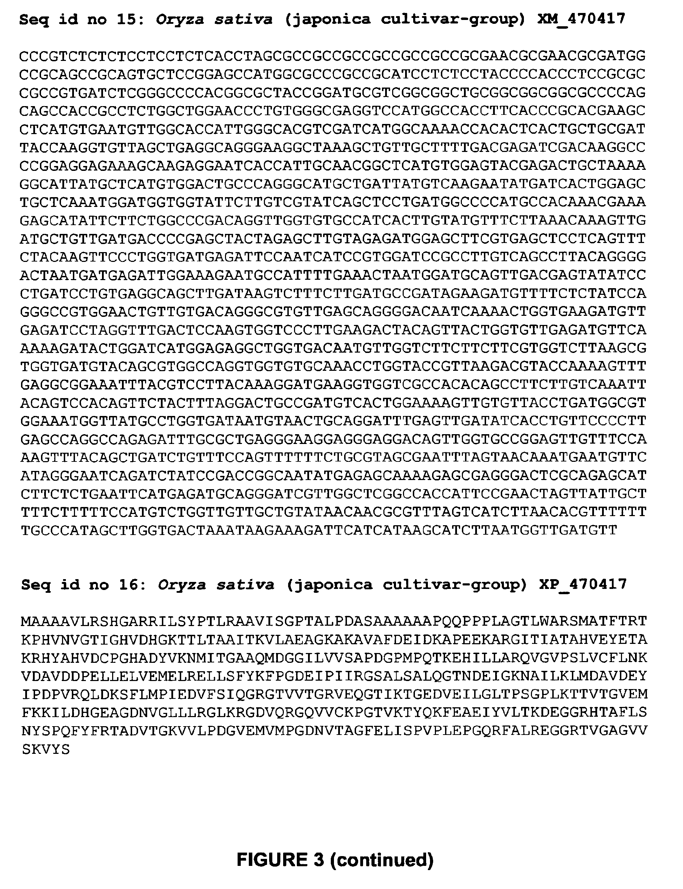

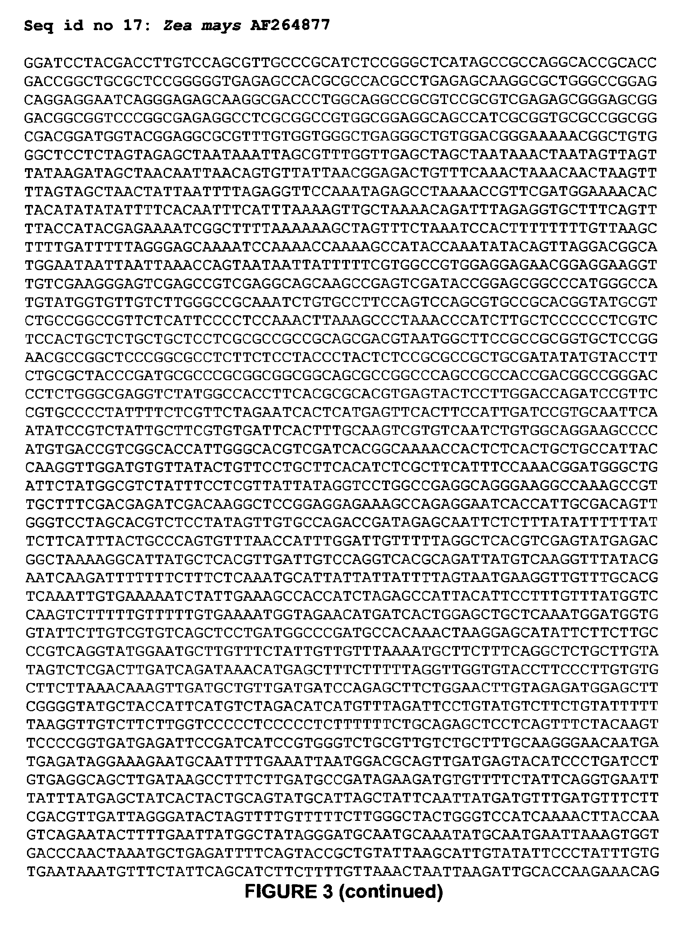

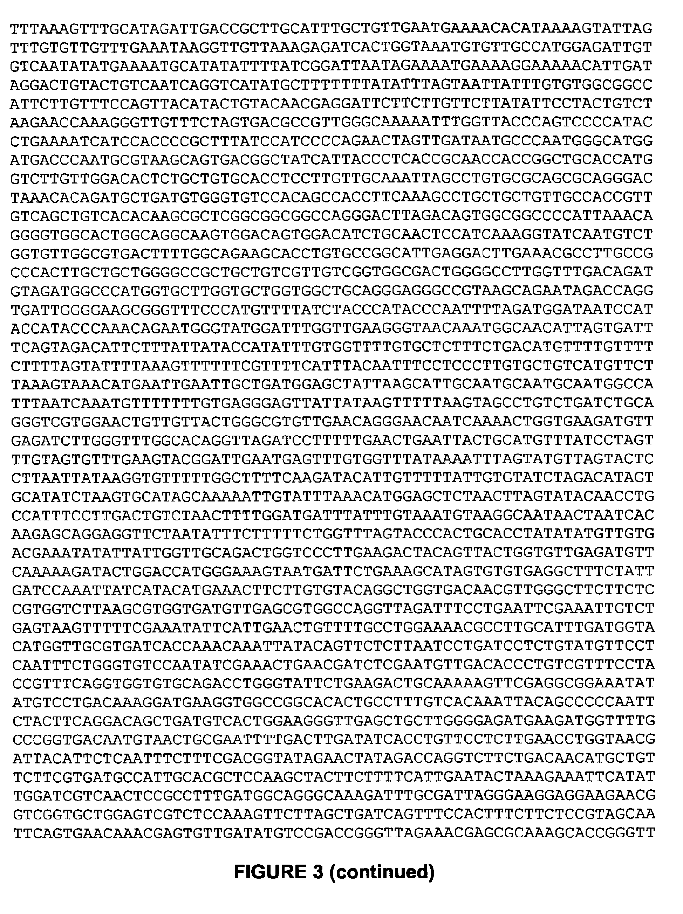

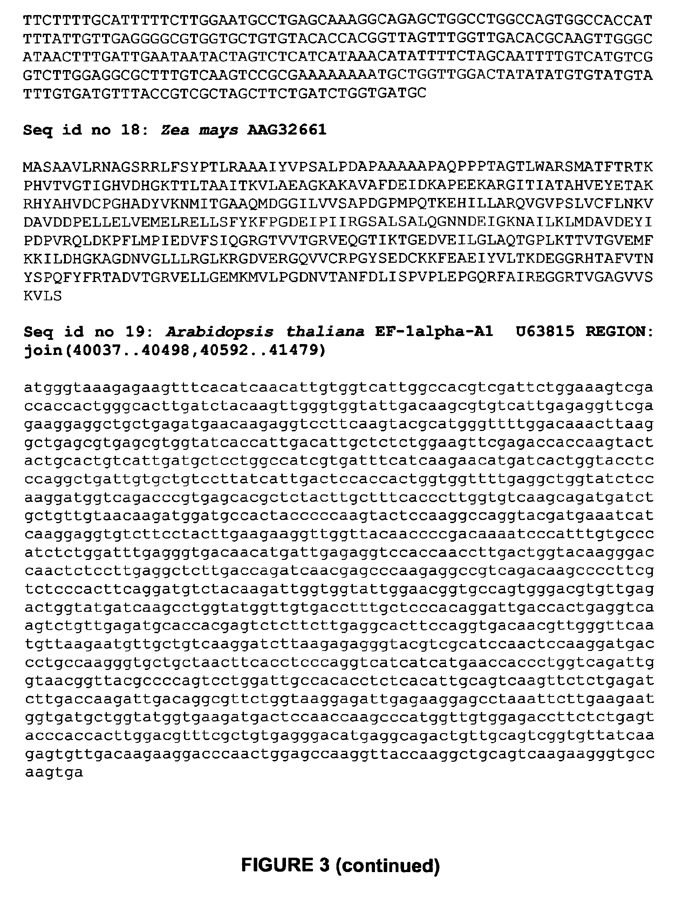

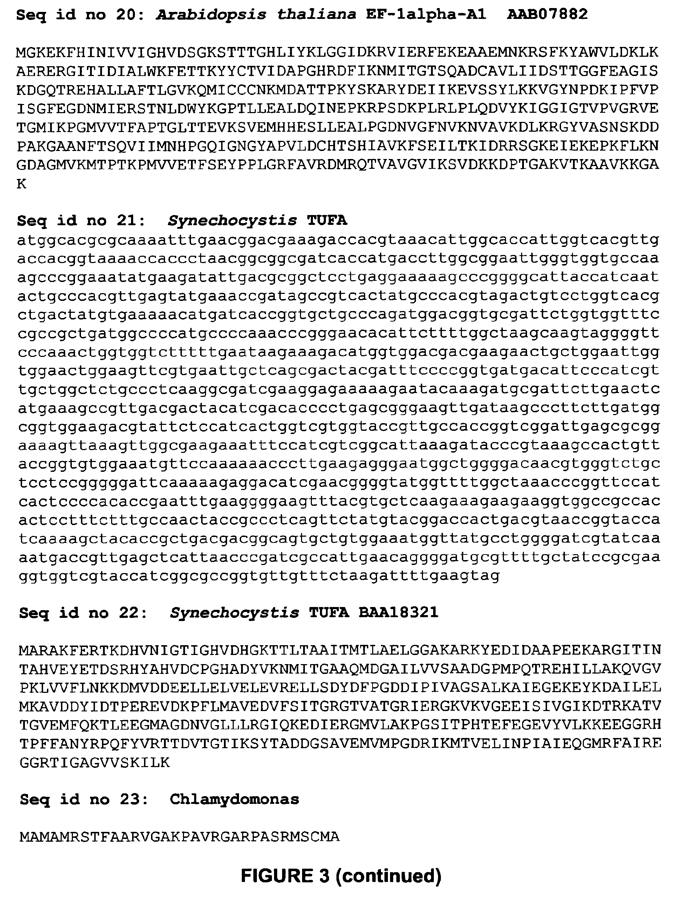

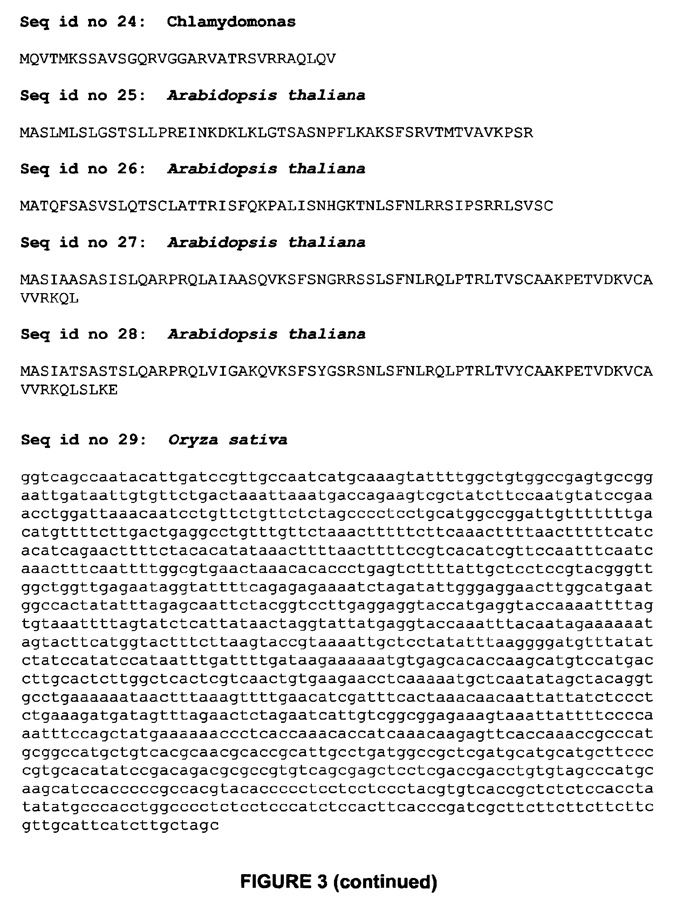

**Fig. 3** details examples of sequences useful in performing the methods according to the present invention.

**Examples**

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

***Example 1: Gene Cloning***

A gene encoding an EFTu protein was first identified as an expressed sequence tag from Tobacco BY2 cells and was isolated as a partial sequence in a CDNA-AFLP experiment performed with cDNA made from a synchronized tobacco BY2 cell culture (*Nicotiniana tabacum* L. cv. Bright Yellow-2). Based on this cDNA-AFLP experiment, BY2 tags that were cell cycle modulated were identified and selected for further cloning. The expressed sequence tags were used to screen a Tobacco cDNA library and to isolate the full length cDNA.

**Synchronization of BY2 cells**

Tobacco BY2 (*Nicotiana tabacum* L. cv. Bright Yellow-2) cultured cell suspension was synchronized by blocking cells in early S-phase with aphidicolin as follows. A cultured cell suspension of *Nicotiana tabacum* L. cv. Bright Yellow 2 was maintained as described (Nagata et al. Int. Rev. Cytol. 132, 1-30, 1992). For synchronization, a 7-day-old stationary culture was diluted 10-fold in fresh medium supplemented with aphidicolin (Sigma-Aldrich, St. Louis, MO; 5 mg/l), a DNA-polymerase $\alpha$ inhibiting drug. After 24 h, the cells were released from the block by several washings with fresh medium and they resumed their cell cycle progression.

**RNA extraction and cDNA synthesis**

Total RNA was prepared using LiCl precipitation (Sambrook *et al.,* 2001) and poly($A^+$) RNA was extracted from 500 pg of total RNA using Oligotex columns (Qiagen, Hilden, Germany) according to the manufacturer's instructions. Starting from 1 $\mu$g of poly($A^+$) RNA, first-strand cDNA was synthesized by reverse transcription with a biotinylated oligo-$dT_{25}$ primer (Genset, Paris, France) and Superscript II (Life Technologies, Gaithersburg, MD). Second-strand synthesis was done by strand displacement with *Escherichia coli* ligase (Life Technologies), DNA polymerase I (USB, Cleveland, OH) and RNAse-H (USB).

**cDNA-AFLP analysis**

Five hundred ng of double-stranded cDNA was used for AFLP analysis as described (Vos et al., Nucleic Acids Res. 23 (21) 4407-4414, 1995; Bachem et al., Plant J. 9 (5) 745-53, 1996) with modifications. The restriction enzymes used were *Bst*YI and *Mse*I (Biolabs) and the digestion was done in two separate steps. After the first restriction digest with one of the enzymes, the 3' end fragments were collected on Dyna beads (Dynal, Oslo, Norway) by means of their biotinylated tail, while the other fragments were washed away. After digestion with the second enzyme, the released restriction fragments were collected and used as templates in the subsequent AFLP steps. For pre-amplifications, a *Mse*I primer without selective nucleotides was combined with a *Bst*YI primer containing either a T or a C as 3' most nucleotide. PCR conditions were as described (Vos *et al.,* 1995). The obtained amplification mixtures were diluted 600-fold and 5 $\mu$l was used for selective amplifications using a $P^{33}$-labeled *Bst*YI primer and the Amplitaq-Gold polymerase (Roche Diagnostics, Brussels, Belgium). Amplification products were separated on 5% polyacrylamide gels using the Sequigel system (Biorad). Dried gels were exposed to Kodak Biomax films as well as scanned in a phospholmager (Amersham Pharmacia Biotech, Little Chalfont, UK).

**Characterization of AFLP fragments**

Bands corresponding to differentially expressed transcripts, among which was the transcript corresponding to SEQ ID NO 1, were isolated from the gel and eluted DNA was reamplified under the same conditions as for selective amplification. Sequence information was obtained either by direct sequencing of the reamplified polymerase chain reaction product with the selective *Bst*YI primer or after cloning the fragments in pGEM-T easy (Promega, Madison, WI) or by sequencing individual clones. The obtained sequences were compared against nucleotide and protein sequences present in the publicly available databases by BLAST sequence alignments (Altschul et al., Nucleic Acids Res. 25 (17) 3389-3402 1997). When available, tag sequences were replaced with longer EST or isolated cDNA sequences to increase the chance of finding significant homology. The physical cDNA clone corresponding to SEQ ID NO 1 was subsequently amplified from a commercial Tobacco cDNA library as follows.

**Gene Cloning**

A c-DNA library with average inserts of 1,400 bp was made with poly($A^+$) isolated from actively dividing, non-synchronized BY2 tobacco cells. These library-inserts were cloned in the vector pCMVSPORT6.0, comprising a attB gateway cassette (Life Technologies). From this library 46,000 clones were selected, arrayed in 384-well microtiter plates, and subsequently spotted in duplicate on nylon filters. The arrayed clones were screened by using pools of several hundreds of radioactively labeled tags as probes (among which was the BY2-tag corresponding to the sequence of SEQ ID NO 1). Positive clones were isolated (among which the clone reacting with the BY2-tag corresponding to the sequence of SEQ ID NO 1), sequenced, and aligned with the tag sequence. In cases where hybridisation with the tag failed, the full-length cDNA corresponding to the tag was selected by PCR amplification as follows. Tag-specific primers were designed using primer3 program (http://www-genome.wi.mit.edu/genome_software/other/primer3.html) and used in combination with the common vector primer to amplify partial cDNA inserts. Pools of DNA, from 50,000, 100,000, 150,000, and 300,000 cDNA clones were used as templates in PCR amplifications. Amplification products were isolated

from agarose gels, cloned, sequenced and aligned with tags.

Subsequently, the full-length cDNA corresponding to SEQ ID NO 1 was cloned from the pCMVsport6.0 library vector into a suitable plant expression vector via an LR Gateway reaction.

**LR gateway reaction to clone the gene into a plant expression vector**

The pCMV Sport 6.0 was subsequently used in an LR reaction with a Gateway destination vector suitable for rice transformation. This vector contains as functional elements within the T-DNA borders a plant selectable marker and a Gateway cassette intended for LR *in vivo* recombination with the sequence of interest already cloned in the donor vector. Upstream of this Gateway cassette is the oleosin promoter for seed-specific expression of the gene.

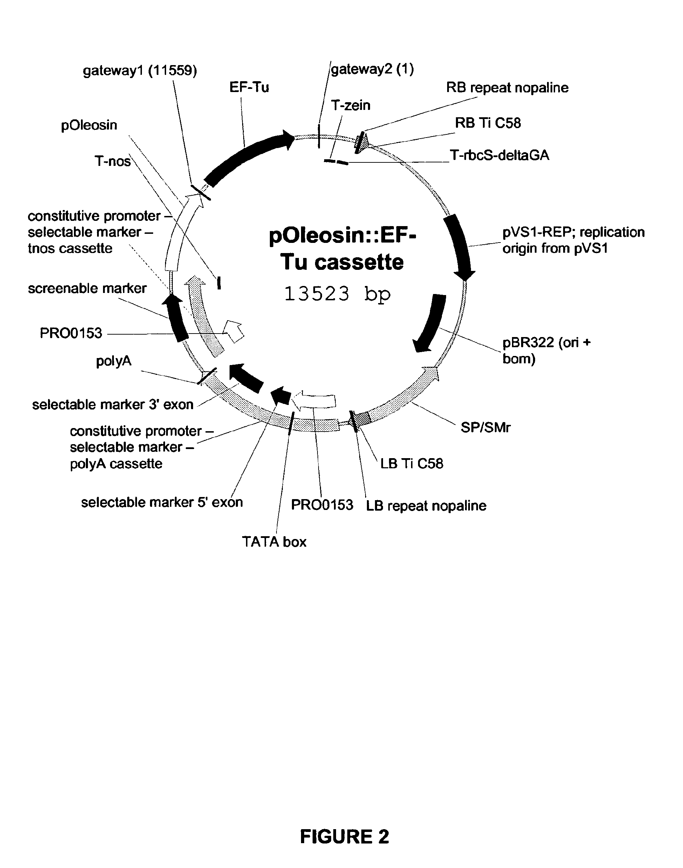

After the recombination step, the resulting expression vector (see Fig. 2) was transformed into *Agrobacterium* strain LBA4404 and subsequently into rice plants.

***Example 3: Evaluation and Results***

Approximately 15 to 20 independent T0 rice transformants were generated. The primary transformants were transferred from a tissue culture chamber to a greenhouse for growing and harvest of T1 seed. 5 events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. T1 events were further evaluated in the T2 generation following the same evaluation procedure as for the T1 generation but with more individuals per event.

**Statistical analysis: F-test**

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the presence or position of the gene that is causing the differences in phenotype.

**3.1 Seed-related parameter measurements**

The mature primary panicles were harvested, bagged, barcode-labeled and then dried for three days in an oven at 37°C. The panicles were then threshed and all the seeds were collected and counted. The filled husks were separated from the empty ones using an airblowing device. The empty husks were discarded and the remaining fraction was counted again. The filled husks were weighed on an analytical balance. The number of filled seeds was determined by counting the number of filled husks that remained after the separation step. The total seed yield (seed weight) was measured by weighing all filled husks harvested from a plant. Total seed number per plant was measured by counting the number of husks harvested from a plant. The harvest index in the present invention is defined as the ratio of total seed yield and the above ground area ($mm^2$) multiplied by a factor $10^6$.

**3.2 Aboveground Area**

Plant aboveground area (or area max) was determined by counting the total number of pixels from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground.

The Table of results below show the p values from the F test for the T1 and T2 evaluations. The percentage difference between the transgenics and the corresponding nullizygotes is also shown. For example, in the case of number of filled seeds, 2 lines in the T1 generation gave a greater than 59% difference in the number of filled seeds obtained from transgenic plants compared to the number of filled seeds obtained from corresponding nullizygotes; the p-value from the F test for these two lines was less than 0.12. Overall, 5 lines were evaluated for the number of filled seeds giving a percentage difference of 19% for the number of filled seeds of transgenics plants compared to the number of filled seeds of corresponding nullizygotes; a p value from the F test for these 5 lines gave a value of 0.05. Similarly, in the T2 generation, 1 line gave a 23% difference for the number of filled seeds obtained from transgenic plants compared to the number of filled seeds obtained from corresponding nullizygotes; the p-value from the F test for this line was 0.08.

Overall, in the T2 generation, 3 lines were evaluated for the number of filled seeds giving a percentage difference between the number of filled seeds of transgenics plants compared to the number of filled seeds of corresponding nullizygotes of 11 %; a p value from the F test for these 3 lines gave a value of 0.08.

| **EFTu: pOleosin Phenotype measured** | T1 Generation | % Difference | p-value from F test | | T2 Generation | % Difference | p-value from the F test |
|---|---|---|---|---|---|---|---|
| **Area max** | 2 lines | >17% | <0.1 | | / | / | / |
| **No. filled seeds** | 2 lines | >59% | <0.12 | | 1 line | 23% | 0.08 |
| | overall 5 lines | 19% | 0.05 | | overall 3 lines | 11% | 0.097 |
| **Total Weight Seeds** | 1 line | 62% | 0.091 | | 1 line | 22% | 0.094 |
| | overall 5 lines | 15% | 0.15 | | overall 3 lines | 11% | 0.123 |
| **Harvest Index** | 1 line | 46% | 0.019 | | 1 line | 24% | 0.043 |
| | overall 5 lines | 16% | 0.05 | | overall 3 lines | 10% | 0.1 |

## Claims

1. Method for increasing plant seed yield under normal growth conditions, relative to wild type plants grown under corresponding conditions, comprising:

(i) transforming a plant, plant part or plant cell by introducing a genetic construct comprising a nucleic acid encoding an EFTu polypeptide comprising: (i) the following GTP-binding domains: GXXXXGK and DXXG and NKXD and S/L/K/Q A/G UV/F, where X is any amino acid; and (ii) EFTu domain ALMANPAIKR or a domain

having at least 75% identity thereto and/or K D/G EE S/A;
(ii) expressing said nucleic acid; and
(iii) targeting the polypeptide to a plastid in the plant cell, unless the nucleic acid or variant thereof is a plastidic gene.

2. Method according to the previous claim, wherein said EFTu nucleic acid is overexpressed in a plant.

3. Method according to any of the previous claims , wherein said EFTu nucleic acid is of plant origin, preferably from a dicotyledonous plant, further preferably from the family *Solanaceae,* more preferably from the genus *Nicotianae.*

4. Method according to any of the previous claims, wherein said EFTu nucleic acid is operably linked to a seed-specific promoter, preferably to an embryo-specific and/or alerone-specific promoter.

5. Method according to the previous claim, wherein said promoter is an oleosin promoter.

6. Method according to any previous claim , wherein said increased seed yield is selected from any one or more of (i) increased seed biomass; (ii) increased number of (filled) seeds; (iii) increased seed size; (iv) increased seed volume; (v) increased harvest index; and (vi) increased thousand kernel weight (TKW).

7. Method for the production of a transgenic plant having increased seed yield under normal growth conditions relative to yield of corresponding wild type plants grown under corresponding conditions, which method comprises:

(i) transforming a plant or plant part (including plant cell) with a genetic construct comprising a nucleic acid encoding an EFTu polypeptide comprising: (a) the following GTP-binding domains GXXXXGK and DXXG and NKXD and S/L/K/Q A/G L/V/F, where X is any amino acid; and (b) EFTu domain ALMANPAIKR or a domain having at least 75% identity thereto and/or K D/G EE S/A, which polypeptide is targeted to a plastid in a plant cell if it is not already in the plastid;
(ii) expressing said nucleic acid; and
(iii) cultivating the plant or plant part under conditions promoting plant growth and development.

8. Use of a genetic construct comprising said EFTu nucleic acid/gene as defined in claim 1 in increasing seed yield, in plants grown under normal growth conditions.

9. Use according to claim 8, wherein said seed yield includes one or more of the following: increased number of (filled) seeds, increased seed weight, increased harvest index and increased TKW.

**Patentansprüche**

1. Verfahren zur Erhöhung des Samenertrags von Pflanzen unter normalen Wachstumsbedingungen im Vergleich zu Wildtyp-Pflanzen, die unter entsprechenden Bedingungen wachsen gelassen werden, umfassend:

(i) Transformieren einer Pflanze, eines Pflanzenteils oder einer Pflanzenzelle durch Einführen eines genetischen Konstrukts, welches eine Nukleinsäure umfasst, die ein EFTu-Polypeptid codiert, umfassend: (i) die folgenden GTP-Bindungsdomänen: GXXXXGK und DXXG und NKXD und S/L/K/Q A/G L/V/F, worin X jegliche Aminosäure ist; und (ii) EFTu-Domäne ALMANPAIKR oder eine Domäne mit mindestens 75 % Identität dazu und/oder K D/G EE S/A;
(ii) Exprimieren der Nukleinsäure; und
(iii) Targetieren des Polypeptids auf ein Plastid in der Pflanzenzelle, es sei denn die Nukleinsäure oder Variante davon ist ein plastidiäres Gen.

2. Verfahren gemäß dem vorausgehenden Anspruch, wobei die EFTu-Nukleinsäure in einer Pflanze überexprimiert ist.

3. Verfahren gemäß einem beliebigen der vorausgehenden Ansprüche, wobei die EFTu-Nukleinsäure von pflanzlichem Ursprung ist, vorzugsweise von einer dikotyledonen Pflanze, weiter bevorzugt aus der Familie *Solanaceae,* noch weiter bevorzugt von der Gattung *Nicotianae.*

4. Verfahren gemäß einem beliebigen der vorausgehenden Ansprüche, wobei die EFTu-Nukleinsäure funktionell mit

einem Samen-spezifischen Promotor, vorzugsweise mit einem Embryo-spezifischen und/oder Aleron-spezifischen Promotor verbunden ist.

**5.** Verfahren gemäß dem vorausgehenden Anspruch, wobei der Promotor ein Oleosin-Promotor ist.

**6.** Verfahren gemäß einem beliebigen der vorausgehenden Ansprüche, wobei die erhöhte, Samenausbeute aus einem beliebigen oder mehreren aus (i) einer erhöhten Samenbiomasse; (ii) einer erhöhten Anzahl an (gefüllten) Samen; (iii) einer erhöhten Samengröße; (iv) einem erhöhten Samenvolumen; (v) einem erhöhten Ernteindex; und (vi) einem erhöhten Tausendkorngewicht (TKW) gewählt ist.

**7.** Verfahren für die Herstellung einer transgenen Pflanze mit einem erhöhten Samenertrag unter normalen Wachstumsbedingungen im Vergleich zum Ertrag von entsprechenden Wildtyp-Pflanzen, die unter entsprechenden Bedingungen wachsen gelassen werden, wobei das Verfahren Folgendes umfasst:

(i) Transformieren einer Pflanze, eines Pflanzenteils (einschließlich einer Pflanzenzelle) mit einem genetischen Konstrukt, welches eine Nukleinsäure umfasst, die ein EFTu-Polypeptid codiert, umfassend: (a) die folgenden GTP-Bindungsdomänen: GXXXXGK und DXXG und NKXD und S/L/K/Q A/G L/V/F, worin X jegliche Aminosäure ist; und (b) EFTu-Domäne ALMANPAIKR oder eine Domäne mit mindestens 75 % Identität dazu und/oder K D/G EE S/A, wobei das Polypeptid auf ein Plastid in einer Pflanzenzelle targetiert ist bzw. darauf abzielt, wenn es sich nicht bereits in dem Plastid befindet;
(ii) Exprimieren der Nukleinsäure; und
(iii) Kultivieren der Pflanze oder eines Pflanzenteils unter Bedingungen, welche das Pflanzenwachstum und die Entwicklung fördert.

**8.** Verwendung eines genetischen Konstrukts, umfassend die/das EFTu-Nukleinsäure/Gen, wie in Anspruch 1 definiert, bei der Erhöhung des Samenertrags bei Pflanzen, die unter normalen wachstumsbedingungen wachsen gelassen werden.

**9.** Verwendung gemäß Anspruch 8, wobei der Samenertrag eines oder mehrere der Folgenden einschließt: eine erhöhte Anzahl an (gefüllten) Samen, ein erhöhtes Samengewicht, einen erhöhten Ernteindex und ein erhöhtes TKW.

## Revendications

**1.** Procédé destiné à accroître le rendement en graines de plantes, dans des conditions normales de croissance, par rapport à des plantes de type sauvage cultivées dans les conditions correspondantes, comprenant les étapes consistant à :

(i) transformer une plante, une partie de plante ou une cellule végétale en introduisant un construit génétique, comprenant un acide nucléique qui code pour un polypeptide EFTu comprenant : (i) les domaines de liaison au GTP suivants : GXXXXGK et DXXG et NKXD et S/L/K/Q A/G L/V/F, dans lesquels X est un acide aminé quelconque ; et (ii) un domaine ALMANPAIKR d'EFTu ou un domaine ayant au moins 75% d'identité avec celui-ci et/ou K D/G EE S/A ;
(ii) exprimer ledit acide nucléique ; et
(iii) cibler le polypeptide vers un plaste dans la cellule végétale, à moins que l'acide nucléique ou un variant de celui-ci soit un gène de plaste.

**2.** Procédé selon la revendication précédente, dans lequel ledit acide nucléique d'EFTu est surexprimé dans une plante.

**3.** Procédé selon l'une quelconque des revendications précédentes, dans lequel ledit acide nucléique d'EFTu est d'origine végétale, de préférence il provient d'une plante dicotylédone, de préférence en outre il provient de la famille des Solanacées, mieux préféré il provient du genre *Nicotianae.*

**4.** Procédé selon l'une quelconque des revendications précédentes, dans lequel ledit acide nucléique d'EFTu est lié, de manière opérationnelle, à un promoteur spécifique des graines, de préférence un promoteur spécifique d'un embryon et/ou d'une aleurone.

**5.** Procédé selon la revendication précédente, dans lequel ledit promoteur est un promoteur d'oléosine.

**6.** Procédé selon l'une quelconque des revendications précédentes, dans lequel ladite augmentation du rendement en graines est choisie parmi un quelconque élément suivant ou plusieurs d'entre eux : (i) une biomasse accrue de graines ; (ii) un nombre accru de graines (pleines) ; (iii) une taille accrue des graines ; (iv) un volume accru des graines ; (v) un indice de récolte accru ; et (vi) un poids de mille grains (PMG) accru.

**7.** Procédé pour la production d'une plante transgénique ayant un rendement accru en graines dans des conditions normales de croissance, par rapport au rendement de plantes de type sauvage correspondantes ayant été cultivées dans des conditions correspondantes, lequel procédé comprend les étapes consistant à :

(i) transformer une plante ou une partie de plante (y compris une cellule végétale) avec un construit génétique, comprenant un acide nucléique qui code pour un polypeptide EFTu comprenant : (a) les domaines de liaison au GTP suivants : GXXXXGK et DXXG et NKXD et S/L/K/Q A/G L/V/F, dans lesquels X est un acide aminé quelconque ; et (b) un domaine ALMANPAIKR d'EFTu ou un domaine ayant au moins 75% d'identité avec celui-ci et/ou K D/G EE S/A, lequel polypeptide est ciblé vers un plaste dans une cellule végétale s'il n'existe pas déjà dans le plaste ;
(ii) exprimer ledit acide nucléique ; et
(iii) cultiver la plante ou la partie de plante dans des conditions favorisant la croissance et le développement de la plante.

**8.** Utilisation d'un construit génétique comprenant ledit acide nucléique/gène d'EFTu, tel que défini dans la revendication 1, dans l'augmentation du rendement en graines pour des plantes cultivées dans des conditions normales de croissance.

**9.** Utilisation selon la revendication 8, dans laquelle ledit rendement en graines comprend un ou plusieurs des éléments suivants : un nombre accru de graines (pleines) ; un poids accru des graines ; un indice de récolte accru ; et un PMG accru.

```
At4g02930       --MASVVLRN-----------------PSSKRLVPFSSQIYSRCGASVTSSYSISHSIGG 41
X89227          --MEEPVDREDIDLRVSSTDIGWNEFAAASKRLVPFSSQIYSRCGASVTSSYSISHSIGG 58
XM_470417       -MAAAAVLRS-----------------HGARRILSYPTLRAAVISGPTALPDASAAAAAA 42
AF264877        -MASAAVLRN-----------------AGSRRLFSYPTLRAAAIYVPSALPDAP-AAAAA 41
M94204          MASISAA-TATSSTKLVS-------SNSTNPLLPSSTKPSKLILSSSFTPNCSTLFLHS 51
D11470          MASISAASATATASTKLAYPYSPSSSSSSSNTAAVFPSNSSKLILSSSFTPTPSTLFLHS 60
At4g20360       -MAISAPAACSSSSRILCSYSSP---SPSLCPAISTSGKLKTLTLSSSFLPSYS-----L 51
AF145053        MASLASA---------------------SASTSLVFSTSSSKPRLGSSVGFSSPARFRRT 39
S04430          ------------------------------------------------------------
AAB07882.1      ------------------------------------------------------------
                                     .    : .       .         .

At4g02930       DDLSSSTFGTS-----SFWRSMATFTRNKPHVNVGTIGHVDHGKTTLTAAITKVLAEEGK 96
X89227          DDLSSSTFGTS-----SFWRSMATFTRNKPHVNVGTIGHVDHGKTTLTAAITKVLAEEGK 113
XM_470417       PQQPPPLAGT------LWARSMATFTRTKPHVNVGTIGHVDHGKTTLTAAITKVLAEAGK 96
AF264877        PAQPPPTAGT------LWARSMATFTRTKPHVTVGTIGHVDHGKTTLTAAITKVLAEAGK 95
M94204          PATPSSTATHR-HRRFTVRAARGKFERKKPHVNIGTIGHVDHGKTTLTAALTMALASMGN 110
D11470          PTTTPSTTHP---RRFTVRAARGKFERKKPHVNIGTIGHVDHGKTTLTAALTMALASMGN 117
At4g20360       TTTSASQSTR---RSFTVRAARGKFERKKPHVNIGTIGHVDHGKTTLTAALTMALASIGS 108
AF145053        AAAAASKGTGRRAGLLVMRAARGKFERTKPHVNIGTIGHVDHGKTTLTAALTMVLASVGG 99
S04430          -------------------MARAKFERTKPHANIGTIGHVDHGKTTLTAAITTVLAKAGM 41
AAB07882.1      ------------------------MGKEKFHINIVVIGHVDSGKSTTTGHLIYKLGGIDK 36
                   ...              : ..: : * * .: .***** **:* *. :   *.  .

At4g02930       AKAIAFD---------------EIDKAPEEKKRGITIATAHVEYETAKRHYAHVDCPGHA 141
X89227          AKAIAFD---------------EIDKAPEEKKRGITIATAHVEYETAKRHYAHVDCPGHA 158
XM_470417       AKAVAFD---------------EIDKAPEEKARGITIATAHVEYETAKRHYAHVDCPGHA 141
AF264877        AKAVAFD---------------EIDKAPEEKARGITIATAHVEYETAKRHYAHVDCPGHA 140
M94204          SAPKKYD---------------EIDAAPEERARGITINTATVEYETENRHYAHVDCPGHA 155
D11470          SAPKKYD---------------EIDAAPEERARGITINTATVEYETENRHYAHVDCPGHA 162
At4g20360       SVAKKYD---------------EIDAAPEERARGITINTATVEYETENRHYAHVDCPGHA 153
AF145053        SAPKKYD---------------EIDAAPEERARGITINTATVEYETETRHYAHVDCPGHA 144
S04430          AKARAYA---------------DIDAAPEEKARGITINTAHVEYETGNRHYAHVDCPGHA 86
AAB07882.1      RVIERFEKEAAEMNKRSFKYAWVLDKLKAERERGITIDIALWKFETTKYYCTVIDAPGHR 96
                     :                 :*    *: *****  *  ::** . : : :*.***

At4g02930       DYVKNMITGAAQMDGGILVVSGPDG-------PMPQTKEHILLARQVGVPSLVCFLNKVD 194
X89227          DYVKNMITGAAQMDGGILVVSGPDG-------PMPQTKEHILLARQVGVPSLVCFLNKVD 211
XM_470417       DYVKNMITGAAQMDGGILVVSAPDG-------PMPQTKEHILLARQVGVPSLVCFLNKVD 194
AF264877        DYVKNMITGAAQMDGGILVVSAPDG-------PMPQTKEHILLARQVGVPSLVCFLNKVD 193
M94204          DYVKNMITGAAQMDGAILVCSGADG-------PMPQTKEHILLAKQVGVPNMVVFLNKQD 208
D11470          DYVKNMITGAAQMDGAILVVSGADG-------PMPQTKEHILLAKQVGVPNMVVFLNKQD 215
At4g20360       DYVKNMITGAAQMDGAILVVSGADG-------PMPQTKEHILLAKQVGVPDMVVFLNKED 206
AF145053        DYVKNMITGAAQMDGAILVVSGADG-------PMPQTKEHILLAKQVGVPKIVVFLNKKD 197
S04430          DYVKNMITGAAQMDGAILVVSAADG-------PMPQTREHILLAKQVGVPNIVVFLNKED 139
AAB07882.1      DFIKNMITGTSQADCAVLIIDSTTGGFEAGISKDGQTREHALLAFTLGVKQMICCQNKMD 156
                *::******::* * .:*: ... *          **:** ***  :**  .::  ** *

At4g02930       VVDDPELLELVEMELRELLSFYKFPGDDIPIIRGSALSALQGTN--------DEIGRQAI 246
X89227          VVDDPELLELVEMELRELLSFYKFPGDDIPIIRGSALSALQGTN--------DEIGRQAI 263
XM_470417       AVDDPELLELVEMELRELLSFYKFPGDEIPIIRGSALSALQGTN--------DEIGKNAI 246
AF264877        AVDDPELLELVEMELRELLSFYKFPGDEIPIIRGSALSALQGNN--------DEIGKNAI 245
M94204          QVDDEELLQLVELEVRELLSSYEFPGDDIPIISGSALLALHALMANPSIKRGENQWVDKI 268
D11470          QVDDEELLELVELEVRELLSSYEFPGDEIPIISGSALLALHALMANPSIKRGENQWVDKI 275
At4g20360       QVDDAELLELVELEVRELLSSYEFNGDDIPIISGSALLAVETLTENPKVKRGDNKWVDKI 266
AF145053        QVDDEELLQLVELEVRELLSSYEYDGDEVPIVAGSALKALENLMANPAIKRGDDEWVDGI 257
S04430          MVDDAELLELVELEVRELLSSYDFPGDDIPIVAGSALQALEAIQGGASGQKGDNPWVDKI 199
AAB07882.1      ATTPKYSKARYDEIIKEVSSYLKKVGYNPDKIPFVPISGFEGDNMIERSTNLDWYKGPTL 216
                 .         :  ::*: *  .  *  :   :   .: . :           :     :
```

FIGURE 1

```
At4g02930         LKLMDAVDEYIPDPVRVLDKPFLMPIEDVFSIQGRGTVATGRIEQGVIKVGEEVEILGLR 306
X89227            LKLMDAVDEYIPDPVRVLDKPFLMPIEDVFSIQGRGTVATGRIEQGVIKVGEEVEILGLR 323
XM_470417         LKLMDAVDEYIPDPVRQLDKSFLMPIEDVFSIQGRGTVVTGRVEQGTIKTGEDVEILGLT 306
AF264877          LKLMDAVDEYIPDPVRQLDKPFLMPIEDVFSIQGRGTVVTGRVEQGTIKTGEDVEILGLA 305
M94204            YELMDAVDSYIPIPVRQTELPFLMAIEDVFSITGRGTVATGRVERGTVRIGDTVDIVGLK 328
D11470            YQLMDNVDEYIPIPQRQTELPFLMAIEDVFSITGRGTVATGRVERGTVKVGEIVDIVGLK 335
At4g20360         YELMDAVDDYIPIPQRQTELPFLLAVEDVFSITGRGTVATGRVERGTVKVGETVDLVGLR 326
AF145053          FSLIDSVDNYIPVPQRQTDLPFLLAVEDVFSITGRGTVATGRIERGTVKVGDTVDIVGIR 317
S04430            LKLMEEVDAYIPTPEREVDRPFLMAVEDVFTITGRGTVATGRIERGSVKVGETIEIVGLR 259
AAB07882.1        LEALDQINE----PKRPSDKPLRLPLQDVYKIGGIGTVPVGRVETGMIKPGMVVTFAPTG 272
                   . :: ::   . * *  : .: :.::**:.* * *** .**:* * :: *  : :

At4g02930         EGGVPLKSTVTGVEMFKKILDNGQAGDNVGLLLRGLKREDIQRGMVIAKP--GSCKTYKK 364
X89227            EGGCSTKSTVTGVEMFKKILDNGQAGDNVGLLLRGLKREDIQRGMVIAKP--GSCKTYKK 381
XM_470417         PSG-PLKTTVTGVEMFKKILDHGEAGDNVGLLLRGLKRGDVQRGQVVCKP--GTVKTYQK 363
AF264877          QTG-PLKTTVTGVEMFKKILDHGKAGDNVGLLLRGLKRGDVERGQVVCRP--GYSEDCKK 362
M94204            DTR---STTVTGVEMFQKILDEAMAGDNVGLLLRGIQKIDIQRGMVLAKP--GTITPHTK 383
D11470            DTR---NTTVTGVEMFQKILDEAMAGDNVGLLLRGIQKIDIQRGMVLAKP--GTITPHTK 390
At4g20360         ETR---SYTVTGVEMFQKILDEALAGDNVGLLLRGIQKADIQRGMVLAKP--GSITPHTK 381
AF145053          ETR---NCTVTGVEMFQKTMDDAMAGDNVGLLLRGMQKDDIERGMVLAKP--ASITPHTK 372
S04430            DTR---STTVTGVEMFQKTLDEGLAGDNVGLLLRGIQKTDIERGMVLAKP--GSITPHTK 314
AAB07882.1        LTT-----EVKSVEMHHESLLEALPGDNVGFNVKNVAVKDLKRGYVASNSKDDPAKGAAN 327
                        .  *..***.:: : .. .*****: ::.:   *::** * ...         :

At4g02930         FEAEIYVLTK--DEGGRHTAFFSNYRPQFYLRTADITGKVELPEN------VKMVMPGDN 416
X89227            FEAEIYVLTK--DEGGRHTAFFSNYRPQFYLRTADITGKVELPEN------VKMVMPGDN 433
XM_470417         FEAEIYVLTK--DEGGRHTAFLSNYSPQFYFRTADVTGKVVLPDG------VEMVMPGDN 415
AF264877          FEAEIYVLTK--DEGGRHTAFVTNYSPQFYFRTADVTGRVELLGE------MKMVLPGDN 414
M94204            FEAIVYVLKK--EEGGRHSPFFSGYRPQFYMRTTDVTGKVTSITTDKGE-ESKMVMPGDR 440
D11470            FEALVYVLKK--EEGGRHSPFFAGYRPQFYMRTTDVTGKVTVIMSDKGE-ESKMVMPGDR 447
At4g20360         FEAIIYVLKK--EEGGRHSPFFAGYRPQFYMRTTDVTGKVTKIMNDKDE-ESKMVMPGDR 438
AF145053          FDAVVYVLKK--DEGGRHSPFFPGYRPQFYMRTTDVTGNVPKIMNDKDE-EAKMCMPGDR 429
S04430            FESEVYVLKK--EEGGRHTPFFPGYRPQFYVRTTDVTGAISDFTADDGS-AAEMVIPGDR 371
AAB07882.1        FTSQVIIMNHPGQIGNGYAPVLDCHTSHIAVKFSEILTKIDRRSGKEIEKEPKFLKNGDA 387
                  * : : ::.:  : *. ::...  : .:: .: :::   :     .. .   ::   **

At4g02930         VTAVFELIMPVPLETG------QRFALREGGRTVGAGVVSKVMT---------------- 454
X89227            VTAVFELIMPVPLETG------QRFALREGGRTVGAGVVSKVMT---------------- 471
XM_470417         VTAGFELISPVPLEPG------QRFALREGGRTVGAGVVSKVYS---------------- 453
AF264877          VTANFDLISPVPLEPG------QRFAIREGGRTVGAGVVSKVLS---------------- 452
M94204            VNLVVELIMPVACEQG------MRFAIREGGKTVGAGVIQKIIE---------------- 478
D11470            VNMVVELIMPVACEQG------MRFAIREGGKTVGAGVIQKILE---------------- 485
At4g20360         VKIVVELIVPVACEQG------MRFAIREGGKTVGAGVIGTILE---------------- 476
AF145053          VKMVVELIQPVACEQG------MRFAIPEGGKTVGAGVINTILK---------------- 467
S04430            IKMTVELINPIAIEQG------MRFAIREGGRTIGAGVVSKILQ---------------- 409
AAB07882.1        GMVKMTPTKPMVVETFSEYPPLGRFAVRDMRQTVAVGVIKSVDKKDPTGAKVTKAAVKKG 447
                      .    *:  *         ***: :  :*:..**: .:

At4g02930         --
X89227            --
XM_470417         --
AF264877          --
M94204            --
D11470            --
At4g20360         --
AF145053          --
S04430            --
AAB07882.1        AK 449
```

EFTu motif: -----------

Further EFTu motif: ........................

GTP binding motif: — — — — —

**FIGURE 1 (continued)**

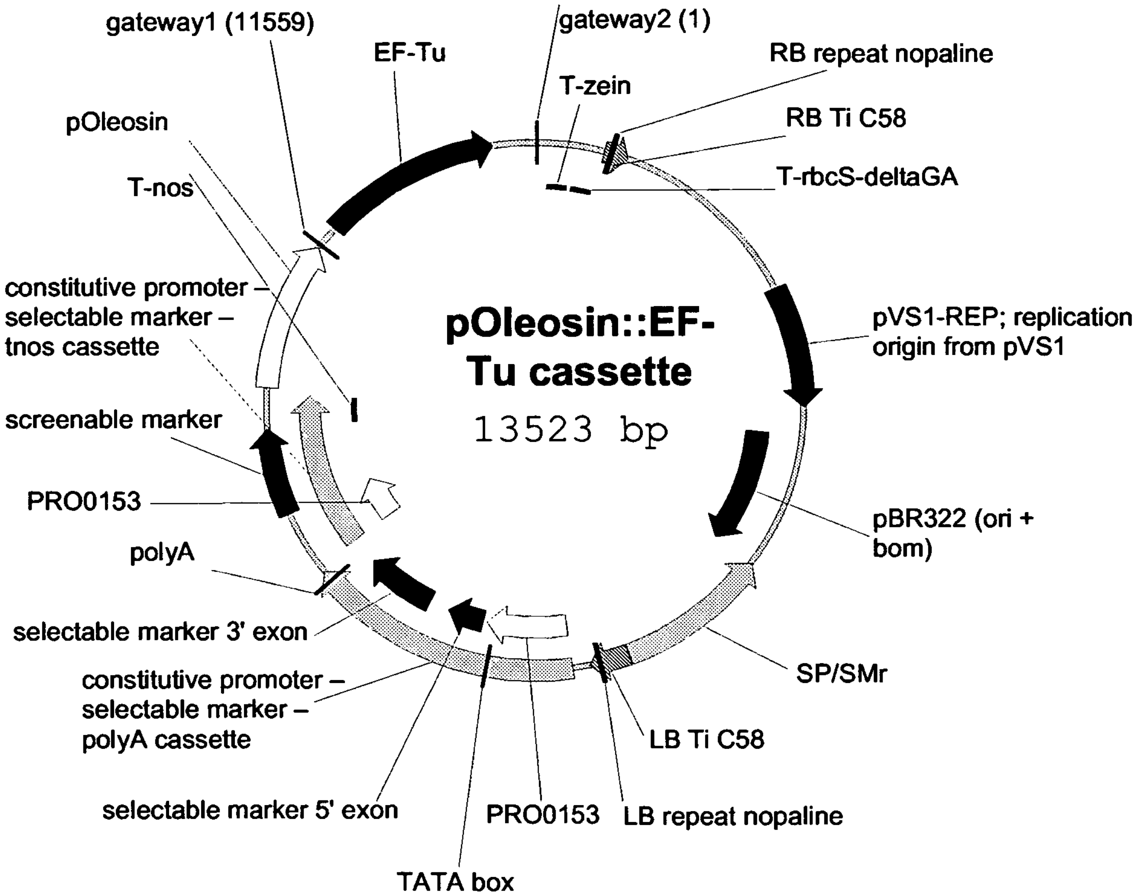
gateway1 (11559)
EF-Tu
gateway2 (1)
RB repeat nopaline
T-zein
RB Ti C58
pOleosin
T-rbcS-deltaGA
T-nos
constitutive promoter – selectable marker – tnos cassette
pOleosin::EF-Tu cassette
13523 bp
pVS1-REP; replication origin from pVS1
screenable marker
PRO0153
pBR322 (ori + bom)
polyA
selectable marker 3' exon
SP/SMr
constitutive promoter – selectable marker – polyA cassette
LB Ti C58
selectable marker 5' exon
PRO0153
LB repeat nopaline
TATA box

FIGURE 2

**Seq id no 1:** ***Nicotiana tabacum*** **EF-Tu DNA sequence**

ccacgcgttcgggataacatcattatccttctctcctcttcttccttctttcaaccacaattct
cactcccctctttcgtctctcttctccaacttcaatcccatttcaggcaaaagctgtcatgg
cttcaatttcagcagcttctgccacagctacagcttctacaaagcttgcatacccttattcccc
ttcttcctcaagcagcagcagcaacactgctgctgtattcccttcaaattcctcaaagcttatc
ctttcctcttcttttacacccaccccttcaacccttttcctccactcaccaacaactactcctt
ccaccacccacccccgtcggttcactgtccgcgctgcacgtggcaaattcgagcgtaaaaaacc
tcacgtcaacattggtacaattggccacgttgaccatggaaagaccacactcacagctgctttg
accatggcgcttgcctctatgggcaactccgcccccaagaaatatgacgaaattgatgctgccc
ctgaagaaagggcgcgtggtattactatcaacactgccactgtggaatatgaaacggaaaacag
acattatgcacacgtggactgcccggggcatgctgattatgtcaagaacatgattactggtgct
gcccaaatggatggggcaattcttgttgtgtcaggtgctgatggcccaatgccacagactaaag
agcatatttgttagctaagcaagttggggtccctaatatggttgttttcttgaacaaacaaga
ccaagttgatgatgaggagttacttgagcttgttgagttggaggtaagagaattattgtcaagt
tatgagttccctggtgatgaaattcctattatttctggttctgcacttttagctttagaggctt
tgatggctaatcctagtattaaaggggtgaaaatcaatgggttgataagatttatcaattgat
ggataatgttgatgaatatatccctatcccacaaagacaaactgaattgcctttcttgatggct
attgaggatgttttctcgattaccggtagaggtactgtggcgacggggagagtagagagaggga
ctgttaaggttggggaaattgttgatatagttggattgaaggatactaggaatactacagtgac
aggggttgagatgtttcagaagattttggatgaagcgatggcgggagataatgtgggattgttg
ttgagaggtattcagaagattgatattcagagagggatggtgttggcgaagcccggaacaatta
ctccgcacacaaagtttgaagctttggtgtatgtgttgaagaaggaagagggaggaaggcattc
cccgttctttgcgggttataggcctcaatttacatgaggacaactgatgtgactggaaaggtt
actgtgattatgagtgacaaaggagaggaatctaagatggtcatgcctggcgatcgtgtaaaca
tggtggttgagcttatcatgccggttgcatgtgagcaagggatgaggtttgctatcagggaagg
aggaaagactgttggagctggtgttattcagaaaatcttagaatgatgaacttgcagctgagca
tctcttttcacatgatcggcactttccattgaagttacttaatccattgtcatatatgcaactt
cttggttacttttattatgtcttagaatcttactttagtagaagtatcctgttttaaacaccaa
attctactgaacttttgggatttttcctcagtctcctctttcatttttcctttgcttgaaagga
atgagaacatttgatttcatgcactttatttaatttagaacaaatgtgcgactctgtttaaaat
taag

**Seq id no 2:** ***Nicotiana tabacum*** **EF-Tu protein sequence**

MASISAASATATASTKLAYPYSPSSSSSSSNTAAVFPSNSSKLILSSSFTPTPSTLFLHSPTTT
PSTTHPRRFTVRAARGKFERKKPHVNIGTIGHVDHGKTTLTAALTMALASMGNSAPKKYDEIDA
APEERARGITINTATVEYETENRHYAHVDCPGHADYVKNMITGAAQMDGAILVVSGADGPMPQT
KEHILLAKQVGVPNMVVFLNKQDQVDDEELLELVELEVRELLSSYEFPGDEIPIISGSALLALE
ALMANPSIKRGENQWVDKIYQLMDNVDEYIPIPQRQTELPFLMAIEDVFSITGRGTVATGRVER
GTVKVGEIVDIVGLKDTRNTTVTGVEMFQKILDEAMAGDNVGLLLRGIQKIDIQRGMVLAKPGT
ITPHTKFEALVYVLKKEEGGRHSPFFAGYRPQFYMRTTDVTGKVTVIMSDKGEESKMVMPGDRV
NMVVELIMPVACEQGMRFAIREGGKTVGAGVIQKILE

## FIGURE 3

**Seq id no 3: *Nicotiana tabacum* EF-Tu (M94204)**

GAATTCTTATAATTTAACGTAAACCAAATAATTAAAAAATCGATCTTCATATTGCAGAAAAACA
GGGATTTTATATATGTAGTTTTAAAAAAAGAAAAAGAAAAAGAAAAAATGTTTCCCTGCCCCTA
ATTCCCAAGAGGAAAGTTGTTGCCGCAGCGTAGTGTTATCCAAATAGGTAGAAATGGATAGAAA
AATCCCCCCAAAATGAGTATATGAGCGGCCCTATTTTTTCCTTCCAAGGGATATAACACTATTA
TCCCCTTCTCCTCATTTTCTTTCAAATCCCAATTCTCACTCGCTCTATCCTTAACTCTCTTTTC
TCTCCCAACTTCAATCTCCGGCCATGGCTTCAATTTCAGCAGCCACCGCCACCTCCTCCACTAA
GCTTGTATCATCCAACTCCACCAATCCTCTTCTTCCTTCCTCCACTAAACCCTCTAAGCTTATC
CTATCCTCTTCCTTTACCCCTAATTGTTCCACCCTTTTCCTCCACTCACCCGCCACCCCTTCTT
CTACTGCCACCCATCGCCACCGCCGGTTTACTGTCCGCGCTGCCCGTGGCAAATTCGAGCGGAA
AAAACCCCATGTCAACATCGGTACTATTGGCCATGTTGACCACGGAAAGACTACTCTCACCGCT
GCTTTAACCATGGCTCTTGCTTCTATGGGAAACTCCGCCCCTAAGAAATACGACGAAATTGATG
CCGCCCCAGAAGAGCGTGCTCGTGGTATTACTATTAACACTGCTACTGTTGAGTACGAGACTGA
GAACCGTCACTACGCCCACGTGGATTGCCCTGGTCACGCTGATTATGTCAAGAATATGATTACC
GGTGCTGCCCAAATGGACGGCGCTATTCTTGTTTGTTCAGGTGCTGATGGTCCCATGCCACAGA
CTAAGGAACACATTTTGCTTGCTAAGCAAGTAGGTGTCCCCAACATGGTTGTGTTCTTGAACAA
ACAAGATCAGGTTGATGATGAGGAGCTGCTTCAGCTTGTTGAGTTGGAGGTAAGAGAGTTATTG
TCAAGTTATGAGTTTCCTGGTGATGATATTCCTATTATTTCTGGCTCTGCTCTTTTGGCTTTAG
AGGCTTTGATGGCTAATCCTAGTATTAAGAGAGGTGAAAATCAATGGGTTGATAAGATATATGA
GTTGATGGACGCTGTTGATAGTTATATTCCTATTCCAGTTAGGCAAACTGAATTGCCTTTCTTG
ATGGCTATTGAAGATGTGTTTTCCATTACTGGTAGAGGTACTGTGGCGACGGGGAGGGTAGAGA
GAGGGACTGTTAGGATTGGAGACACTGTTGATATTGTAGGTTTGAAGGACACTAGGAGTACTAC
CGTGACGGGTGTTGAGATGTTTCAAAAGATTTTGGATGAAGCAATGGCTGGAGACAATGTGGGG
TTGTTGTTGAGAGGTATTCAAAAGATTGATATTCAGAGAGGAATGGTGTTGGCAAAACCCGGAA
CAATTACCCCTCACACCAAGTTTGAAGCTATTGTGTATGTGTTGAAGAAGGAGGAGGGTGGTAG
GCATTCCCCCTTCTTTTCAGGGTACAGGCCTCAGTTTTACATGAGGACTACTGATGTGACCGGA
AAGGTTACTTCCATTACGACTGATAAAGGAGAGGAATCTAAGATGGTCATGCCTGGTGATCGTG
TGAACTTGGTGGTTGAGCTCATTATGCCGGTGGCTTGTGAGCAAGGGATGAGATTTGCCATCAG
GGAAGGAGGAAAGACTGTTGGAGCTGGTGTCATTCAGAAAATTATCGAGTGATCAACCAATAGC
GGAGCAATTTCCCTTCAAATGGTCTGGTCCCTGTCACACAGTATTTTTAATCCATTGTCATATA
TGCAGCTTTTTGGTTACTTTTTTCTTATCTTTTAGAATCCTAGTTCAGTTGGAGAAATCTTGTT
ATAAGTCAATCCTAGTGGACCTTTGCGAGTTTGCTTCAGTTTCGTGCTTTGTTTTTTCATTTGT
TTGCAAGGATGGAGAACATTTTGTCTCTTGTGCATCTTACTTTCTGCTAGAACAAATTCATATT
GTGTTTGAAACTCGAGTTTAACATGCTAATGAAATTAGGTGCATTTGATATTTGTTTTGACGAA
TTGATGCTGCTAGGAAACACCCAAAAAAAAGATTACTGAGATGTTTTCTTTACCAGCATATGTC
GTTTAAAAACTAAAATTGATGGACATGAGCTGACCCTGTAAGGAAAATATTGTAACAGAACCTC
ATAAGACTTAAGTTTAGTGATGCTTAATTGTTATTCTCAATTCTGGCTTTAATCCCTGAGTAGA
ACTATCTGAAATGATCAGTTGGTTCTGTCTTGGAATTC

**FIGURE 3 (continued)**

**Seq id no 4: *Nicotiana tabacum* EF-Tu (AAA18546)**

MASISAATATSSTKLVSSNSTNPLLPSSTKPSKLILSSSFTPNCSTLFLHSPATPSSTATHRHR
RFTVRAARGKFERKKPHVNIGTIGHVDHGKTTLTAALTMALASMGNSAPKKYDEIDAAPEERAR
GITINTATVEYETENRHYAHVDCPGHADYVKNMITGAAQMDGAILVCSGADGPMPQTKEHILLA
KQVGVPNMVVFLNKQDQVDDEELLQLVELEVRELLSSYEFPGDDIPIISGSALLALEALMANPS
IKRGENQWVDKIYELMDAVDSYIPIPVRQTELPFLMAIEDVFSITGRGTVATGRVERGTVRIGD
TVDIVGLKDTRSTTVTGVEMFQKILDEAMAGDNVGLLLRGIQKIDIQRGMVLAKPGTITPHTKF
EAIVYVLKKEEGGRHSPFFSGYRPQFYMRTTDVTGKVTSITTDKGEESKMVMPGDRVNLVVELI
MPVACEQGMRFAIREGGKTVGAGVIQKIIE

**Seq id no 5: *Arabidopsis thaliana* EF-Tu NM_118155 (TUFA) (At4g20360)**

ATCCCCAAATCTTCTCCTTCTCCAAAAAAATTTCAAACCCTAGCTTCTCGATTTCTCTCCTCTG
CTCTCCAATTCCATCTTCCCATGGCGATTTCGGCTCCAGCCGCTTGTTCTTCCTCCTCTAGAAT
TCTCTGTTCCTACTCCTCTCCTTCTCCTTCTCTCTGTCCCGCCATTTCCACCTCTGGTAAACTC
AAAACCCTAACTCTCTCTTCCTCCTTCCTCCCTTCTTACTCATTAACCACCACCTCCGCTTCTC
AATCCACTCGTCGCTCCTTCACCGTCCGCGCCGCTCGTGGAAAGTTCGAGAGGAAGAAGCCTCA
TGTCAACATCGGAACCATCGGTCATGTTGACCATGGGAAAACTACTTTAACCGCAGCTCTAACC
ATGGCTCTCGCTTCCATTGGTTCCAGCGTCGCTAAAAAGTACGACGAGATTGACGCTGCGCCGG
AGGAGAGAGCTCGTGGTATCACAATCAACACTGCTACTGTTGAGTACGAGACTGAGAATCGTCA
CTACGCTCACGTTGATTGTCCTGGTCACGCTGATTACGTTAAGAATATGATTACCGGAGCTGCA
CAGATGGACGGAGCTATCCTCGTTGTTTCCGGCGCCGATGGTCCTATGCCTCAGACTAAAGAGC
ATATCCTTTTGGCTAAGCAGGTTGGTGTTCCTGATATGGTTGTGTTTCTTAACAAAGAGGATCA
AGTAGATGATGCAGAGTTGCTAGAGCTCGTTGAGCTTGAGGTTCGTGAGCTTCTCTCGTCTTAT
GAATTTAACGGTGATGATATTCCGATTATCTCTGGTTCTGCTCTTTTAGCCGTTGAGACTCTTA
CTGAGAATCCTAAGGTTAAGAGAGGTGATAACAAATGGGTAGATAAGATTTATGAACTTATGGA
TGCTGTTGATGATTACATCCCTATCCCTCAGAGACAAACTGAATTGCCATTCTTGTTAGCTGTT
GAGGATGTGTTCTCTATCACTGGACGTGGTACGGTGGCTACAGGGCGTGTCGAGAGAGGTACGG
TTAAGGTAGGAGAGACTGTAGATTTAGTGGGTTTGAGGGAGACTAGGAGTTACACTGTCACTGG
GGTTGAAATGTTTCAGAAGATTCTTGATGAGGCTTTAGCTGGTGACAATGTAGGGTTGTTGCTT
AGGGGTATTCAAAAGGCTGATATTCAGAGAGGTATGGTTTTAGCTAAGCCGGGATCGATTACTC
CACATACCAAGTTTGAAGCAATTATCTATGTGTTGAAGAAAGAGGAAGGTGGAAGGCATTCTCC
ATTCTTTGCAGGGTACAGGCCTCAGTTCTACATGAGGACGACTGATGTTACGGGTAAAGTGACG
AAGATCATGAACGACAAAGATGAAGAGTCGAAGATGGTTATGCCCGGTGATCGAGTGAAGATTG
TTGTTGAGCTTATTGTGCCGGTGGCTTGTGAACAAGGGATGAGGTTTGCTATCAGAGAAGGAGG
AAAGACTGTTGGTGCTGGAGTTATTGGGACGATCCTCGAATGATTATAAGGTTTTAGAGAGTTT
CATATAGTGAGCATCTCTACTTGCTTTCTTTTTGTTTCACTCTTGATTCTCAAACTCTTTGAGT
TTTTACCTTTTTTTATTTATTTTTGTGCCATTTTCCATAAGAACTTGAAATCAAAATGTCATAT
ATTTCTTGATTTTCTGATCCTTGTTATTCATCTTTGTCTCGCTCCATATGATTCTGATGTGGAT
TTGGTACTCAACGACCAATCACTTAATTTG

**FIGURE 3 (continued)**

**Seq id no 6: *Arabidopsis thaliana* CAA36498 At4g20360 X52256**

MAISAPAACSSSSRILCSYSSPSPSLCPAISTSGKLKTLTLSSSFLPSYSLTTTSASQSTRRSF
TVRAARGKFERKKPHVNIGTIGHVDHGKTTLTAALTMALASIGSSVAKKYDEIDAAPEERARGI
TINTATVEYETENRHYAHVDCPGHADYVKNMITGAAQMDGAILVVSGADGPMPQTKEHILLAKQ
VGVPDMVVFLNKEDQVDDAELLELVELEVRELLSSYEFNGDDIPIISGSALLAVETLTENPKVK
RGDNKWVDKIYELMDAVDDYIPIPQRQTELPFLLAVEDVFSITGRGTVATGRVERGTVKVGETV
DLVGLRETRSYTVTGVEMFQKILDEALAGDNVGLLLRGIQKADIQRGMVLAKPGSITPHTKFEA
IIYVLKKEEGGRHSPFFAGYRPQFYMRTTDVTGKVTKIMNDKDEESKMVMPGDRVKIVVELIVP
VACEQGMRFAIREGGKTVGAGVIGTILE

**Seq id no 7: *Nicotiana sylvestris* D11470 tufB gene for chloroplast elongation factor TuB**

TGATCATTCTGCGGATTTTAGTTATGCAAGTGGGCCTAATTCATCAGAGGAAAGAAATAACATG
ATCAGCAACAACTACCCAGCTGATGTCAGACAAATTGTCAAGAAACTCGAGGAGCTGCTTGGTC
AGCAGCAAAAGGAGCTAAATGATCTCAGGAAAAAACATGACTCGGCTATATCAGATATTCTAAG
CAAGCTTCCTCCTGAGATCCGTGGCATTTATGGTTTAAAAATATCTTCCAATAATTTGTAGTGG
GGATGAGGCTGCTATTCTATGGATCCTGAAGACTGAAGTTTCTCTGTGAAGATGTAAGTTACTG
TGCGGCCTTTTGCCACTCAACTGCAGATAGTCTTGGATTGCACCAATTATCTGACTACCCTTGA
AAGGTTTCAAGATATTCGAGAATAGCTGAAAGATTAATTTTGCCGACAATGGTCACATGCAGAT
CAAAAGCTAGAAGGGAAAAGCATGGACCTATCATTAGATACAGATTCACTTCTGTCAAGGATAC
CATCATTTCAGGGCCAGGAATTACAGTAATACAGGTTTCTCATTACACGCATATTGTGCGTCTG
CAAGTCATGTTGCTGCTAAAAATGAGTCATGGCATGTATACTTGTACATATTTACATGAGTATT
TAACTATTGGAAATACCAATTGGTTGAAATAATGCCAGTCAATATATAACTTCTTAAGTTTCTT
TAATTTTATCTTGGTGTTCACTAGATCCGTAAAAAAGGAAAACAATATATCTGAATGATTCTAC
TATTTGCTGTTGTCCGGTTTTTCTTCACAAAGTATGTTTTTTCATATGTGCTTATAAACGCCCC
CGTTTTTTTTAAAACTCTGCTTTTACCTATCCTAAAGCTCTTTATAAGCTCCATACAGATCATT
GGTGTAAACTCACGGTTTAAACTTTTGTGTATACCCTATTGTCCTGGCACTAGTCAATCCTTTT
TACTTTAGTATACATACTAGTATTTGACATGAACATTTTTCTATAGCACACGTTACCAGATTTT
TCTTGGCACTCTATTGTATACATCCCACTCCATGTTAATTTTTACCAATCTTTATAGCAAATAT
ACAGCCATTGTTGCAGATTTGGCATGTACAACCATGAGGGGCGGAGCTAGAATATTCCGAGCGG
GTTCGGCCAAATCCAGAACGACTAGAATTCCGAACCCATAAGCTTGAAATCCTTGCTATGCCTG
TGATAATCATTAAATTAAACACTACCGGGAGAAAAAGAAGGCTTTGGAACAAAAAGATTTTTAT
TTATTTATGTGAAGTTAAAAGAAAAGGCAGGGTAAAAATTTAGACTATTTTCTTACGTGTCACA
TTTTGGGAACAGAATTCACAGAAATCCCATACCCCTAATCCCCACAGTTGCAAGTTGCAGCCCA
GTGTGTCATCCTAGGGAGGTAAGTATTAGATAAAACCCCACATATATGTGAGGCCCTCTTTATT
CCTACCAAGGATAACATCATTATCCTTCTCTCCTCTTCTTCCTTCTTTCAACCACAATTCTCAC
TCCCCTCTTTCGTCTCTCTTCTCCAACTTCAATCCCATTTTCAGGCAAAAAGCTGTCATGGCTT
CAATTTCAGCAGCTTCTGCCACAGCTACAGCTTCTACAAAGCTTGCATACCCTTATTCCCCTTC
TTCCTCAAGCAGCAGCAGCAACACTGCTGCTGTATTCCCTTCAAATTCCTCAAAGCTTATCCTT
TCCTCTTCTTTTACACCCACCCCTTCAACCCTTTTCCTCCACTCACCAACAACTACTCCTTCCA
CCACCCACCCCCGTCGGTTCACTGTCCGCGCTGCACGTGGCAAATTCGAGCGTAAAAAACCTCA
CGTCAACATTGGTACAATTGGCCACGTTGACCATGGAAAGACCACACTCACAGCTGCTTTGACC
ATGGCGCTTGCCTCTATGGGCAACTCCGCCCCCAAGAAATATGACGAAATTGATGCTGCCCCTG
AAGAAAGGGCGCGTGGTATTACTATCAACACTGCCACTGTGGAATATGAAACGGAAAACAGACA
TTATGCACACGTGGACTGCCCGGGGCATGCTGATTATGTCAAGAACATGATTACTGGTGCTGCC

**FIGURE 3 (continued)**

CAAATGGATGGGGCAATTCTTGTTGTGTCAGGTGCTGATGGCCCAATGCCACAGACTAAAGAGC
ATATTTTGTTAGCTAAGCAAGTTGGGGTCCCTAATATGGTTGTTTTCTTGAACAAACAAGACCA
AGTTGATGATGAGGAGTTACTTGAGCTTGTTGAGTTGGAGGTAAGAGAATTATTGTCAAGTTAT
GAGTTCCCTGGTGATGAAATTCCTATTATTTCTGGTTCTGCACTTTTAGCTTTAGAGGCTTTGA
TGGCTAATCCTAGTATTAAAAGGGGTGAAAATCAATGGGTTGATAAGATTTATCAATTGATGGA
TAATGTTGATGAATATATCCCTATCCCACAAAGACAAACTGAATTGCCTTTCTTGATGGCTATT
GAGGATGTTTTCTCGATTACCGGTAGAGGTACTGTGGCGACGGGGAGAGTAGAGAGAGGGACTG
TTAAGGTTGGGGAAATTGTTGATATAGTTGGATTGAAGGATACTAGGAATACTACAGTGACAGG
GGTTGAGATGTTTCAGAAGATTTTGGATGAAGCGATGGCGGGAGATAATGTGGGATTGTTGTTG
AGAGGTATTCAGAAGATTGATATTCAGAGAGGGATGGTGTTGGCGAAGCCCGGAACAATTACTC
CGCACACAAAGTTTGAAGCTTTGGTGTATGTGTTGAAGAAGGAAGAGGGAGGAAGGCATTCCCC
GTTCTTTGCGGGTTATAGGCCTCAATTTTACATGAGGACAACTGATGTGACTGGAAAGGTTACT
GTGATTATGAGTGACAAAGGAGAGGAATCTAAGATGGTCATGCCTGGCGATCGTGTAAACATGG
TGGTTGAGCTTATCATGCCGGTTGCATGTGAGCAAGGGATGAGGTTTGCTATCAGGGAAGGAGG
AAAGACTGTTGGAGCTGGTGTTATTCAGAAAATCTTAGAATGATGAACTTGCAGCTGAGCATCT
CTTTTCACATGATCGGCACTTTCCATTGAAGTTACTTAATCCATTGTCATATATGCAACTTCTT
GGTTACTTTTATTATGTCTTAGAATCTTACTTTAGTAGAAGTATCCTGTTTTAAACACCAAATT
CTACTGAACTTTTGGGATTTTTCGGCAGTCTCCTCTTTCATTTTTCCTTTGCTTGAAAGGAATA
GAACATTTGATTTCATGCACTTTATTTAATTTAGAACAAATGTGCGACTCTGTTTGAAAATTTA
AGTGCAGATTTGCTAATTTGAGATTCACTGCTTCGGCTTTTTGTTTCAGCAAAATGATGCTGAT
AGAAAAGCACAAAAGAATAAATAATTTGTTTGTAAACTATGGATAATAATTTGTCAGCTGGCCA
ATGACTGAATTTTTTATGTCCATGTAACTAAAAACTATGGTACTCGAAGCAGATATGTATTTAT
CTGTATTGTTTAAAATCGACAAGAGGGGTTGCTCTGATGGTAAGCAACCCCCACTTCCAACTAA
GAGGCTGTGAGTTCAAGTTTCCCCAAGAGCAAGGTGGGAAGTTCTTGGAGGGAAGGATGCCGAG
GGTCTAATTGGAAACAGCCTTTCTACC

**Seq id no 8: *Nicotiana sylvestris* tufB gene for chloroplast elongation factor TuB BAA02028**

MASISAASATATASTKLAYPYSPSSSSSSSNTAAVFPSNSSKLILSSSFTPTPSTLFLHSPTTT
PSTTHPRRFTVRAARGKFERKKPHVNIGTIGHVDHGKTTLTAALTMALASMGNSAPKKYDEIDA
APEERARGITINTATVEYETENRHYAHVDCPGHADYVKNMITGAAQMDGAILVVSGADGPMPQT
KEHILLAKQVGVPNMVVFLNKQDQVDDEELLELVELEVRELLSSYEFPGDEIPIISGSALLALE
ALMANPSIKRGENQWVDKIYQLMDNVDEYIPIPQRQTELPFLMAIEDVFSITGRGTVATGRVER
GTVKVGEIVDIVGLKDTRNTTVTGVEMFQKILDEAMAGDNVGLLLRGIQKIDIQRGMVLAKPGT
ITPHTKFEALVYVLKKEEGGRHSPFFAGYRPQFYMRTTDVTGKVTVIMSDKGEESKMVMPGDRV
NMVVELIMPVACEQGMRFAIREGGKTVGAGVIQKILE

# FIGURE 3 (continued)

**Seq id no 9: *Oryza sativa* AF145053 chloroplast tufA**

CAAATCCGAACCTCCCCCCCCCCCCCACTCCTCTGCGCCTCCCTCCTCCGCGGCCTCCGCCATG
GCCTCCCTCGCCTCCGCCTCCGCATCCACCTCCCTGGTCTTCTCCACCTCCTCCTCCAAGCCGC
GCCTCGGCTCCTCCGTCGGATTCTCCTCGCCCGCGCGGTTCCGGCGCACGGCGGCGGCGGCGGC
GTCCAAGGGCACGGGGCGGCGCGCGGGGCTGCTGGTAATGCGCGCGGCGAGGGGGAAGTTCGAG
CGGACCAAGCCGCACGTCAACATCGGCACCATCGGCCACGTCGACCACGGGAAGACTACGCTGA
CGGCGGCGCTCACCATGGTGCTCGCCTCCGTGGGCGGGAGCGCCCCCAAGAAGTACGACGAGAT
CGACGCGGCGCCCGAGGAGCGCGCCCGCGGCATCACCATCAACACCGCCACCGTCGAGTACGAG
ACCGAGACCCGCCACTACGCCCACGTCGACTGCCCCGGCCACGCCGACTACGTCAAGAACATGA
TTACCGGCGCCGCGCAGATGGACGGCGCCATCCTCGTCGTCTCCGGCGCCGACGGGCCCATGCC
GCAGACCAAGGAGCACATCCTGCTCGCCAAGCAGGTCGGTGTCCCCAAGATTGTTGTCTTCCTC
AACAAGAAGGACCAGGTCGACGACGAGGAGCTGCTCCAGCTCGTCGAGCTCGAGGTCCGCGAAT
TGCTCTCCTCCTACGAGTACGATGGCGACGAAGTGCCCATCGTCGCTGGCTCCGCGCTCAAGGC
GCTCGAGAACCTCATGGCCAACCCTGCCATTAAGCGCGGCGATGATGAGTGGGTGGACGGGATC
TTCTCGTTGATTGATTCCGTGGATAACTACATCCCTGTCCCACAGCGCCAGACCGACCTCCCGT
TCTTGCTTGCTGTTGAGGATGTGTTCTCCATCACCGGTCGTGGTACCGTTGCCACTGGCCGTAT
TGAGCGTGGCACCGTCAAGGTTGGGGACACGGTCGATATCGTCGGTATCCGGGAGACTCGCAAC
TGCACGGTGACTGGTGTTGAGATGTTCCAGAAGACCATGGATGATGCGATGGCTGGGGACAATG
TCGGCCTGCTTCTCCGAGGTATGCAGAAGGATGATATCGAGAGAGGCATGGTGCTTGCGAAGCC
TGCTTCCATCACGCCACACACCAAGTTTGATGCGGTTGTGTATGTCCTGAAGAAAGACGAAGGT
GGACGGCACTCACCGTTTTTCCCTGGTTACCGCCCTCAGTTCTACATGCGGACTACCGATGTGA
CGGGGAATGTCCCAAAGATTATGAACGACAAGGACGAGGAGGCGAAGATGTGCATGCCTGGTGA
CCGTGTCAAGATGGTTGTGGAGCTCATCCAGCCCGTCGCTTGTGAGCAGGGAATGAGGTTTGCC
ATCCCTGAGGGTGGAAAGACCGTCGGTGCCGGCGTCATCAATACGATTTTGAAGTAAAGTGGAT
GGAGCATATCCACCGTGAGAATTTTCCTTGTTTACTTTTTGCGAAATGCTCTGTAGTTGTTATT
ACGCGTTGAGTTTTAGGGGTTGATCATGTGAAATTGTAGTATGGCACTTTTGTTTTCAAGTGAA
TTTGCATACTTTGTGATATTCACGACAAAGATACATGTGTTTGCAACTAATTTGGCTATGCAGT
GCCATTTACTGTCC

**Seq id no 10: *Oryza sativa* AAF15312 chloroplast tufA**

MASLASASASTSLVFSTSSSKPRLGSSVGFSSPARFRRTAAAAASKGTGRRAGLLVMRAARGKF
ERTKPHVNIGTIGHVDHGKTTLTAALTMVLASVGGSAPKKYDEIDAAPEERARGITINTATVEY
ETETRHYAHVDCPGHADYVKNMITGAAQMDGAILVVSGADGPMPQTKEHILLAKQVGVPKIVVF
LNKKDQVDDEELLQLVELEVRELLSSYEYDGDEVPIVAGSALKALENLMANPAIKRGDDEWVDG
IFSLIDSVDNYIPVPQRQTDLPFLLAVEDVFSITGRGTVATGRIERGTVKVGDTVDIVGIRETR
NCTVTGVEMFQKTMDDAMAGDNVGLLLRGMQKDDIERGMVLAKPASITPHTKFDAVVYVLKKDE
GGRHSPFFPGYRPQFYMRTTDVTGNVPKIMNDKDEEAKMCMPGDRVKMVVELIQPVACEQGMRF
AIPEGGKTVGAGVINTILK

**FIGURE 3 (continued)**

**Seq id no 11: *Arabidopsis thaliana* mitochondrial elongation factor Tu At4g02930 BT008755**

ATGGCGTCCGTTGTTCTTCGAAACCCTAGCTCGAAGCGCCTTGTTCCATTCTCTTCCCAGATCT
ACTCTCGCTGTGGTGCTTCCGTTACTTCCTCTTACTCGATCTCTCATTCTATCGGTGGAGATGA
TCTCTCTTCCTCTACCTTCGGAACCTCCTCCTTCTGGAGATCCATGGCCACTTTTACTCGAAAT
AAACCTCATGTAAATGTTGGAACTATTGGGCATGTTGATCATGGCAAGACCACTTTAACTGCTG
CAATCACAAAGGTTCTTGCTGAGGAGGGCAAAGCTAAAGCTATTGCCTTTGATGAAATTGATAA
AGCTCCTGAAGAGAAGAAGAGAGGAATTACTATTGCCACGGCTCATGTGGAGTATGAAACTGCA
AAGCGTCACTATGCTCATGTGGATTGCCCTGGACACGCAGATTATGTTAAAAATATGATTACTG
GAGCTGCGCAAATGGATGGCGGAATTCTTGTGGTTTCAGGACCAGATGGACCCATGCCGCAGAC
AAAGGAACATATTCTACTTGCACGCCAGGTTGGTGTTCCCTCACTTGTGTGCTTCTTGAACAAA
GTTGATGTGGTGGATGACCCTGAGCTCTTGGAGCTTGTCGAGATGGAACTACGTGAGCTCCTCA
GCTTCTACAAGTTTCCTGGGGATGATATTCCCATCATCCGAGGATCTGCTCTGTCCGCATTACA
GGGCACCAATGATGAAATTGGAAGGCAAGCTATATTAAAGCTGATGGATGCTGTTGATGAATAT
ATACCTGACCCTGTTCGCGTCCTTGACAAGCCTTTCTTGATGCCAATTGAAGATGTTTTCTCAA
TTCAAGGACGTGGAACTGTTGCAACCGGTCGTATCGAACAGGGAGTCATTAAAGTGGGTGAAGA
AGTTGAGATATTGGGTTTACGTGAGGGGGGTGTTCCACTGAAATCGACTGTAACTGGGGTTGAG
ATGTTCAAGAAGATTTTGGATAATGGACAGGCTGGTGATAATGTAGGACTTCTTCTGCGTGGGC
TAAAGAGAGAAGACATTCAGCGTGGAATGGTGATTGCTAAGCCTGGTTCATGCAAGACATACAA
GAAGTTTGAAGCAGAGATTTACGTGCTCACAAAGGATGAAGGTGGACGTCACACTGCATTTTTC
TCTAACTACAGGCCTCAGTTTTACTTGAGGACTGCAGATATCACTGGCAAAGTGGAATTACCCG
AAAACGTGAAGATGGTTATGCCTGGTGACAATGTCACAGCTGTTTTCGAGTTAATCATGCCTGT
CCCACTCGAAACAGGTCAAAGATTTGCCTTAAGGGAAGGAGGTAGAACAGTTGGAGCTGGTGTT
GTATCAAAAGTGATGACCTAA

**Seq id no 12: *Arabidopsis thaliana* At4g02930 AAM97087**

MASVVLRNPSSKRLVPFSSQIYSRCGASVTSSYSISHSIGGDDLSSSTFGTSSFWRSMATFTRN
KPHVNVGTIGHVDHGKTTLTAAITKVLAEEGKAKAIAFDEIDKAPEEKKRGITIATAHVEYETA
KRHYAHVDCPGHADYVKNMITGAAQMDGGILVVSGPDGPMPQTKEHILLARQVGVPSLVCFLNK
VDVVDDPELLELVEMELRELLSFYKFPGDDIPIIRGSALSALQGTNDEIGRQAILKLMDAVDEY
IPDPVRVLDKPFLMPIEDVFSIQGRGTVATGRIEQGVIKVGEEVEILGLREGGVPLKSTVTGVE
MFKKILDNGQAGDNVGLLLRGLKREDIQRGMVIAKPGSCKTYKKFEAEIYVLTKDEGGRHTAFF
SNYRPQFYLRTADITGKVELPENVKMVMPGDNVTAVFELIMPVPLETGQRFALREGGRTVGAGV
VSKVMT

**FIGURE 3 (continued)**

**Seq id no 13: *Arabidopsis thaliana* X89227 mitochondrial elongation factor Tu**

TAGTTTGAGATGGAAGAACCAGTAGACCGAGAAGATATCGATTTAAGGGTTTCATCCACAGATA
TAGGCTGGAACGAATTCGCGGCCGCCTCGAAGCGCCTTGTTCCATTCTCTTCCCAGATCTACTC
TCGCTGTGGTGCTTCCGTTACTTCCTCTTACTCGATCTCTCATTCTATCGGTGGAGATGATCTC
TCTTCCTCTACCTTCGGAACCTCCTCCTTCTGGAGATCCATGGCCACTTTTACTCGAAATAAAC
CTCATGTAAATGTTGGAACTATTGGGCATGTTGATCATGGCAAGACCACTTTAACTGCTGCAAT
CACAAAGGTTCTTGCTGAGGAGGGCAAAGCTAAAGCTATTGCCTTTGATGAAATTGATAAAGCT
CCTGAAGAGAAGAAGAGAGGAATTACTATTGCCACGGCTCATGTGGAGTATGAAACTGCAAAGC
GTCACTATGCTCATGTGGATTGCCCTGGACACGCAGATTATGTTAAAAATATGATTACTGGAGC
TGCGCAAATGGATGGCGGAATTCTTGTGGTTTCAGGACCAGATGGACCCATGCCGCAGACAAAG
GAACATATTCTACTTGCACGCCAGGTTGGTGTTCCCTCACTTGTGTGCTTCTTGAACAAAGTTG
ATGTGGTGGATGACCCTGAGCTCTTGGAGCTTGTCGAGATGGAACTACGTGAGCTCCTCAGCTT
CTACAAGTTTCCTGGGGATGATATTCCCATCATCCGAGGATCTGCTCTGTCCGCATTACAGGGC
ACCAATGATGAAATTGGAAGGCAAGCTATATTAAAGCTGATGGATGCTGTTGATGAATATATAC
CTGACCCTGTTCGCGTCCTTGACAAGCCTTTCTTGATGCCAATTGAAGATGTTTTCTCAATTCA
AGGACGTGGAACTGTTGCAACCGGTCGTATCGAACAGGGAGTCATTAAAGTGGGTGAAGAAGTT
GAGATATTGGGTTTACGTGAAGGGGGGTGTTCCACTAAATCGACTGTAACTGGGGTTGAGATGT
TCAAGAAGATTTTGGATAATGGACAGGCTGGTGATAATGTAGGACTTCTTCTGCGTGGGCTAAA
GAGAGAAGACATTCAGCGTGGAATGGTGATTGCTAAGCCTGGTTCATGCAAGACATACAAGAAG
TTTGAAGCAGAGATTTACGTGCTCACAAAGGATGAAGGTGGACGTCACACTGCATTTTTCTCTA
ACTACAGGCCTCAGTTTTACTTGAGGACTGCAGATATCACTGGCAAAGTGGAATTACCCGAAAA
CGTGAAGATGGTTATGCCTGGTGACAATGTCACAGCTGTTTTCGAGTTAATCATGCCTGTCCCA
CTCGAAACAGGTCAAAGATTTGCCTTAAGGGAAGGAGGTAGAACAGTTGGAGCTGGTGTTGTAT
CAAAAGTGATGACCTAA

**Seq id no 14: *Arabidopsis thaliana* CAA61511 mitochondrial elongation factor Tu**

MEEPVDREDIDLRVSSTDIGWNEFAAASKRLVPFSSQIYSRCGASVTSSYSISHSIGGDDLSSS
TFGTSSFWRSMATFTRNKPHVNVGTIGHVDHGKTTLTAAITKVLAEEGKAKAIAFDEIDKAPEE
KKRGITIATAHVEYETAKRHYAHVDCPGHADYVKNMITGAAQMDGGILVVSGPDGPMPQTKEHI
LLARQVGVPSLVCFLNKVDVVDDPELLELVEMELRELLSFYKFPGDDIPIIRGSALSALQGTND
EIGRQAILKLMDAVDEYIPDPVRVLDKPFLMPIEDVFSIQGRGTVATGRIEQGVIKVGEEVEIL
GLREGGCSTKSTVTGVEMFKKILDNGQAGDNVGLLLRGLKREDIQRGMVIAKPGSCKTYKKFEA
EIYVLTKDEGGRHTAFFSNYRPQFYLRTADITGKVELPENVKMVMPGDNVTAVFELIMPVPLET
GQRFALREGGRTVGAGVVSKVMT

**FIGURE 3 (continued)**

**Seq id no 15: *Oryza sativa* (japonica cultivar-group) XM_470417**

CCCGTCTCTCTCCTCCTCTCACCTAGCGCCGCCGCCGCCGCCGCCGCGAACGCGAACGCGATGG
CCGCAGCCGCAGTGCTCCGGAGCCATGGCGCCCGCCGCATCCTCTCCTACCCCACCCTCCGCGC
CGCCGTGATCTCGGGCCCCACGGCGCTACCGGATGCGTCGGCGGCTGCGGCGGCGGCGCCCCAG
CAGCCACCGCCTCTGGCTGGAACCCTGTGGGCGAGGTCCATGGCCACCTTCACCCGCACGAAGC
CTCATGTGAATGTTGGCACCATTGGGCACGTCGATCATGGCAAAACCACACTCACTGCTGCGAT
TACCAAGGTGTTAGCTGAGGCAGGGAAGGCTAAAGCTGTTGCTTTTGACGAGATCGACAAGGCC
CCGGAGGAGAAAGCAAGAGGAATCACCATTGCAACGGCTCATGTGGAGTACGAGACTGCTAAAA
GGCATTATGCTCATGTGGACTGCCCAGGGCATGCTGATTATGTCAAGAATATGATCACTGGAGC
TGCTCAAATGGATGGTGGTATTCTTGTCGTATCAGCTCCTGATGGCCCCATGCCACAAACGAAA
GAGCATATTCTTCTGGCCCGACAGGTTGGTGTGCCATCACTTGTATGTTTCTTAAACAAAGTTG
ATGCTGTTGATGACCCCGAGCTACTAGAGCTTGTAGAGATGGAGCTTCGTGAGCTCCTCAGTTT
CTACAAGTTCCCTGGTGATGAGATTCCAATCATCCGTGGATCCGCCTTGTCAGCCTTACAGGGG
ACTAATGATGAGATTGGAAAGAATGCCATTTTGAAACTAATGGATGCAGTTGACGAGTATATCC
CTGATCCTGTGAGGCAGCTTGATAAGTCTTTCTTGATGCCGATAGAAGATGTTTTCTCTATCCA
GGGCCGTGGAACTGTTGTGACAGGGCGTGTTGAGCAGGGGACAATCAAAACTGGTGAAGATGTT
GAGATCCTAGGTTTGACTCCAAGTGGTCCCTTGAAGACTACAGTTACTGGTGTTGAGATGTTCA
AAAAGATACTGGATCATGGAGAGGCTGGTGACAATGTTGGTCTTCTTCTTCGTGGTCTTAAGCG
TGGTGATGTACAGCGTGGCCAGGTGGTGTGCAAACCTGGTACCGTTAAGACGTACCAAAAGTTT
GAGGCGGAAATTTACGTCCTTACAAAGGATGAAGGTGGTCGCCACACAGCCTTCTTGTCAAATT
ACAGTCCACAGTTCTACTTTAGGACTGCCGATGTCACTGGAAAAGTTGTGTTACCTGATGGCGT
GGAAATGGTTATGCCTGGTGATAATGTAACTGCAGGATTTGAGTTGATATCACCTGTTCCCCTT
GAGCCAGGCCAGAGATTTGCGCTGAGGGAAGGAGGGAGGACAGTTGGTGCCGGAGTTGTTTCCA
AAGTTTACAGCTGATCTGTTTCCAGTTTTTTCTGCGTAGCGAATTTAGTAACAAATGAATGTTC
ATAGGGAATCAGATCTATCCGACCGGCAATATGAGAGCAAAAGAGCGAGGGACTCGCAGAGCAT
CTTCTCTGAATTCATGAGATGCAGGGATCGTTGGCTCGGCCACCATTCCGAACTAGTTATTGCT
TTTCTTTTTCCATGTCTGGTTGTTGCTGTATAACAACGCGTTTAGTCATCTTAACACGTTTTTT
TGCCCATAGCTTGGTGACTAAATAAGAAAGATTCATCATAAGCATCTTAATGGTTGATGTT

**Seq id no 16: *Oryza sativa* (japonica cultivar-group) XP_470417**

MAAAAVLRSHGARRILSYPTLRAAVISGPTALPDASAAAAAAPQQPPPLAGTLWARSMATFTRT
KPHVNVGTIGHVDHGKTTLTAAITKVLAEAGKAKAVAFDEIDKAPEEKARGITIATAHVEYETA
KRHYAHVDCPGHADYVKNMITGAAQMDGGILVVSAPDGPMPQTKEHILLARQVGVPSLVCFLNK
VDAVDDPELLELVEMELRELLSFYKFPGDEIPIIRGSALSALQGTNDEIGKNAILKLMDAVDEY
IPDPVRQLDKSFLMPIEDVFSIQGRGTVVTGRVEQGTIKTGEDVEILGLTPSGPLKTTVTGVEM
FKKILDHGEAGDNVGLLLRGLKRGDVQRGQVVCKPGTVKTYQKFEAEIYVLTKDEGGRHTAFLS
NYSPQFYFRTADVTGKVVLPDGVEMVMPGDNVTAGFELISPVPLEPGQRFALREGGRTVGAGVV
SKVYS

**FIGURE 3 (continued)**

**Seq id no 17:** ***Zea mays*** **AF264877**

GGATCCTACGACCTTGTCCAGCGTTGCCCGCATCTCCGGGCTCATAGCCGCCAGGCACCGCACC
GACCGGCTGCGCTCCGGGGGTGAGAGCCACGCGCCACGCCTGAGAGCAAGGCGCTGGGCCGGAG
CAGGAGGAATCAGGGAGAGCAAGGCGACCCTGGCAGGCCGCGTCCGCGTCGAGAGCGGGAGCGG
GACGGCGGTCCCGGCGAGAGGCCTCGCGGCCGTGGCGGAGGCAGCCATCGCGGTGCGCCGGCGG
CGACGGATGGTACGGAGGCGCGTTTGTGGTGGGCTGAGGGCTGTGGACGGGAAAAACGGCTGTG
GGCTCCTCTAGTAGAGCTAATAAATTAGCGTTTGGTTGAGCTAGCTAATAAACTAATAGTTAGT
TATAAGATAGCTAACAATTAACAGTGTTATTAACGGAGACTGTTTCAAACTAAACAACTAAGTT
TTAGTAGCTAACTATTAATTTTAGAGGTTCCAAATAGAGCCTAAAACCGTTCGATGGAAAACAC
TACATATATATTTTCACAATTTCATTTAAAAGTTGCTAAAACAGATTTAGAGGTGCTTTCAGTT
TTACCATACGAGAAAATCGGCTTTTAAAAAAGCTAGTTTCTAAATCCACTTTTTTTGTTAAGC
TTTTGATTTTTAGGGAGCAAAATCCAAAACCAAAAGCCATACCAAATATACAGTTAGGACGGCA
TGGAATAATTAATTAAACCAGTAATAATTATTTTTCGTGGCCGTGGAGGAGAACGGAGGAAGGT
TGTCGAAGGGAGTCGAGCCGTCGAGGCAGCAAGCCGAGTCGATACCGGAGCGGCCCATGGGCCA
TGTATGGTGTTGTCTTGGGCCGCAAATCTGTGCCTTCCAGTCCAGCGTGCCGCACGGTATGCGT
CTGCCGGCCGTTCTCATTCCCCTCCAAACTTAAAGCCCTAAACCCATCTTGCTCCCCCCTCGTC
TCCACTGCTCTGCTGCTCCTCGCGCCGCCGCAGCGACGTAATGGCTTCCGCCGCGGTGCTCCGG
AACGCCGGCTCCCGGCGCCTCTTCTCCTACCCTACTCTCCGCGCCGCTGCGATATATGTACCTT
CTGCGCTACCCGATGCGCCCGCGGCGGCGGCAGCGCCGGCCCAGCCGCCACCGACGGCCGGGAC
CCTCTGGGCGAGGTCTATGGCCACCTTCACGCGCACGTGAGTACTCCTTGGACCAGATCCGTTC
CGTGCCCCTATTTTCTCGTTCTAGAATCACTCATGAGTTCACTTCCATTGATCCGTGCAATTCA
ATATCCGTCTATTGCTTCGTGTGATTCACTTTGCAAGTCGTGTCAATCTGTGGCAGGAAGCCCC
ATGTGACCGTCGGCACCATTGGGCACGTCGATCACGGCAAAACCACTCTCACTGCTGCCATTAC
CAAGGTTGGATGTGTTATACTGTTCCTGCTTCACATCTCGCTTCATTTCCAAACGGATGGGCTG
ATTCTATGGCGTCTATTTCCTCGTTATTATAGGTCCTGGCCGAGGCAGGGAAGGCCAAAGCCGT
TGCTTTCGACGAGATCGACAAGGCTCCGGAGGAGAAAGCCAGAGGAATCACCATTGCGACAGTT
GGGTCCTAGCACGTCTCCTATAGTTGTGCCAGACCGATAGAGCAATTCTCTTTATATTTTTTAT
TCTTCATTTACTGCCCAGTGTTTAACCATTTGGATTGTTTTTAGGCTCACGTCGAGTATGAGAC
GGCTAAAAGGCATTATGCTCACGTTGATTGTCCAGGTCACGCAGATTATGTCAAGGTTTATACG
AATCAAGATTTTTTTCTTTCTCAAATGCATTATTATTATTTTAGTAATGAAGGTTGTTTGCACG
TCAAATTGTGAAAAATCTATTGAAAGCCACCATCTAGAGCCATTACATTCCTTTGTTTATGGTC
CAAGTCTTTTTGTTTTTGTGAAAATGGTAGAACATGATCACTGGAGCTGCTCAAATGGATGGTG
GTATTCTTGTCGTGTCAGCTCCTGATGGCCCGATGCCACAAACTAAGGAGCATATTCTTCTTGC
CCGTCAGGTATGGAATGCTTGTTTCTATTGTTGTTTAAAATGCTTCTTTCAGGCTCTGCTTGTA
TAGTCTCGACTTGATCAGATAAACATGAGCTTTCTTTTTAGGTTGGTGTACCTTCCCTTGTGTG
CTTCTTAAACAAAGTTGATGCTGTTGATGATCCAGAGCTTCTGGAACTTGTAGAGATGGAGCTT
CGGGGTATGCTACCATTCATGTCTAGACATCATGTTTAGATTCCTGTATGTCTTCTGTATTTTT
TAAGGTTGTCTTCTTGGTCCCCCTCCCCCTCTTTTTCTGCAGAGCTCCTCAGTTTCTACAAGT
TCCCCGGTGATGAGATTCCGATCATCCGTGGGTCTGCGTTGTCTGCTTTGCAAGGGAACAATGA
TGAGATAGGAAAGAATGCAATTTTGAAATTAATGGACGCAGTTGATGAGTACATCCCTGATCCT
GTGAGGCAGCTTGATAAGCCTTTCTTGATGCCGATAGAAGATGTGTTTTCTATTCAGGTGAATT
TATTTATGAGCTATCACTACTGCAGTATGCATTAGCTATTCAATTATGATGTTTGATGTTTCTT
CGACGTTGATTAGGGATACTAGTTTTGTTTTTCTTGGGCTACTGGGTCCATCAAAACTTACCAA
GTCAGAATACTTTTGAATTATGGCTATAGGGATGCAATGCAAATATGCAATGAATTAAAGTGGT
GACCCAACTAAATGCTGAGATTTTCAGTACCGCTGTATTAAGCATTGTATATTCCCTATTTGTG
TGAATAAATGTTTCTATTCAGCATCTTCTTTTGTTAAACTAATTAAGATTGCACCAAGAAACAG

**FIGURE 3 (continued)**

TTTAAAGTTTGCATAGATTGACCGCTTGCATTTGCTGTTGAATGAAAACACATAAAAGTATTAG
TTTGTGTTGTTTGAAATAAGGTTGTTAAAGAGATCACTGGTAAATGTGTTGCCATGGAGATTGT
GTCAATATATGAAAATGCATATATTTTATCGGATTAATAGAAAATGAAAAGGAAAAACATTGAT
AGGACTGTACTGTCAATCAGGTCATATGCTTTTTTATATTTAGTAATTATTTGTGTGGCGGCC
ATTCTTGTTTCCAGTTACATACTGTACAACGAGGATTCTTCTTGTTCTTATATTCCTACTGTCT
AAGAACCAAAGGGTTGTTTCTAGTGACGCCGTTGGGCAAAAATTTGGTTACCCAGTCCCCATAC
CTGAAAATCATCCACCCCGCTTTATCCATCCCCAGAACTAGTTGATAATGCCCAATGGGCATGG
ATGACCCAATGCGTAAGCAGTGACGGCTATCATTACCCTCACCGCAACCACCGGCTGCACCATG
GTCTTGTTGGACACTCTGCTGTGCACCTCCTTGTTGCAAATTAGCCTGTGCGCAGCGCAGGGAC
TAAACACAGATGCTGATGTGGGTGTCCACAGCCACCTTCAAAGCCTGCTGCTGTTGCCACCGTT
GTCAGCTGTCACACAAGCGCTCGGCGGCGGCCAGGGACTTAGACAGTGGCGGCCCCATTAAACA
GGGGTGGCACTGGCAGGCAAGTGGACAGTGGACATCTGCAACTCCATCAAAGGTATCAATGTCT
GGTGTTGGCGTGACTTTTGGCAGAAGCACCTGTGCCGGCATTGAGGACTTGAAACGCCTTGCCG
CCCACTTGCTGCTGGGGCCGCTGCTGTCGTTGTCGGTGGCGACTGGGGCCTTGGTTTGACAGAT
GTAGATGGCCCATGGTGCTTGGTGCTGGTGGCTGCAGGGAGGGCCGTAAGCAGAATAGACCAGG
TGATTGGGGAAGCGGGTTTCCCATGTTTTATCTACCCATACCCAATTTTAGATGGATAATCCAT
ACCATACCCAAACAGAATGGGTATGGATTTGGTTGAAGGGTAACAAATGGCAACATTAGTGATT
TCAGTAGACATTCTTTATTATACCATATTTGTGGTTTTGTGCTCTTTCTGACATGTTTTGTTTT
CTTTTAGTATTTTAAAGTTTTTTCGTTTTCATTTACAATTTCCTCCCTTGTGCTGTCATGTTCT
TAAAGTAAACATGAATTGAATTGCTGATGGAGCTATTAAGCATTGCAATGCAATGCAATGGCCA
TTTAATCAAATGTTTTTTTGTGAGGGAGTTATTATAAGTTTTTAAGTAGCCTGTCTGATCTGCA
GGGTCGTGGAACTGTTGTTACTGGGCGTGTTGAACAGGGAACAATCAAAACTGGTGAAGATGTT
GAGATCTTGGGTTTGGCACAGGTTAGATCCTTTTTGAACTGAATTACTGCATGTTTATCCTAGT
TTGTAGTGTTTGAAGTACGGATTGAATGAGTTTGTGGTTTATAAAATTTAGTATGTTAGTACTC
CTTAATTATAAGGTGTTTTTGGCTTTTCAAGATACATTGTTTTTATTGTGTATCTAGACATAGT
GCATATCTAAGTGCATAGCAAAAATTGTATTTAAACATGGAGCTCTAACTTAGTATACAACCTG
CCATTTCCTTGACTGTCTAACTTTTGGATGATTTATTTGTAAATGTAAGGCAATAACTAATCAC
AAGAGCAGGAGGTTCTAATATTTCTTTTTCTGGTTTAGTACCCACTGCACCTATATATGTTGTG
ACGAAATATATTATTGGTTGCAGACTGGTCCCTTGAAGACTACAGTTACTGGTGTTGAGATGTT
CAAAAAGATACTGGACCATGGGAAAGTAATGATTCTGAAAGCATAGTGTGTGAGGCTTTCTATT
GATCCAAATTATCATACATGAAACTTCTTGTGTACAGGCTGGTGACAACGTTGGGCTTCTTCTC
CGTGGTCTTAAGCGTGGTGATGTTGAGCGTGGCCAGGTTAGATTTCCTGAATTCGAAATTGTCT
GAGTAAGTTTTTCGAAATATTCATTGAACTGTTTTGCCTGGAAAACGCCTTGCATTTGATGGTA
CATGGTTGCGTGATCACCAAACAAATTATACAGTTCTCTTAATCCTGATCCTCTGTATGTTCCT
CAATTTCTGGGTGTCCAATATCGAAACTGAACGATCTCGAATGTTGACACCCTGTCGTTTCCTA
CCGTTTCAGGTGGTGTGCAGACCTGGGTATTCTGAAGACTGCAAAAAGTTCGAGGCGGAAATAT
ATGTCCTGACAAAGGATGAAGGTGGCCGGCACACTGCCTTTGTCACAAATTACAGCCCCCAATT
CTACTTCAGGACAGCTGATGTCACTGGAAGGGTTGAGCTGCTTGGGGAGATGAAGATGGTTTTG
CCCGGTGACAATGTAACTGCGAATTTTGACTTGATATCACCTGTTCCTCTTGAACCTGGTAACG
ATTACATTCTCAATTTCTTTCGACGGTATAGAACTATAGACCAGGTCTTCTGACAACATGCTGT
TCTTCGTGATGCCATTGCACGCTCCAAGCTACTTCTTTTCATTGAATACTAAAGAAATTCATAT
TGGATCGTCAACTCCGCCTTTGATGGCAGGGCAAAGATTTGCGATTAGGGAAGGAGGAAGAACG
GTCGGTGCTGGAGTCGTCTCCAAAGTTCTTAGCTGATCAGTTTCCACTTTCTTCTCCGTAGCAA
TTCAGTGAACAAACGAGTGTTGATATGTCCGACCGGGTTAGAAACGAGCGCAAAGCACCGGGTT

## FIGURE 3 (continued)

TTCTTTTGCATTTTTCTTGGAATGCCTGAGCAAAGGCAGAGCTGGCCTGGCCAGTGGCCACCAT
TTTATTGTTGAGGGGCGTGGTGCTGTGTACACCACGGTTAGTTTGGTTGACACGCAAGTTGGGC
ATAACTTTGATTGAATAATACTAGTCTCATCATAAACATATTTTCTAGCAATTTTGTCATGTCG
GTCTTGGAGGCGCTTTGTCAAGTCCGCGAAAAAAAATGCTGGTTGGACTATATATGTGTATGTA
TTTGTGATGTTTACCGTCGCTAGCTTCTGATCTGGTGATGC

**Seq id no 18:** ***Zea mays*** **AAG32661**

MASAAVLRNAGSRRLFSYPTLRAAAIYVPSALPDAPAAAAAPAQPPPTAGTLWARSMATFTRTK
PHVTVGTIGHVDHGKTTLTAAITKVLAEAGKAKAVAFDEIDKAPEEKARGITIATAHVEYETAK
RHYAHVDCPGHADYVKNMITGAAQMDGGILVVSAPDGPMPQTKEHILLARQVGVPSLVCFLNKV
DAVDDPELLELVEMELRELLSFYKFPGDEIPIIRGSALSALQGNNDEIGKNAILKLMDAVDEYI
PDPVRQLDKPFLMPIEDVFSIQGRGTVVTGRVEQGTIKTGEDVEILGLAQTGPLKTTVTGVEMF
KKILDHGKAGDNVGLLLRGLKRGDVERGQVVCRPGYSEDCKKFEAEIYVLTKDEGGRHTAFVTN
YSPQFYFRTADVTGRVELLGEMKMVLPGDNVTANFDLISPVPLEPGQRFAIREGGRTVGAGVVS
KVLS

**Seq id no 19:** ***Arabidopsis thaliana*** **EF-1alpha-A1 U63815 REGION: join(40037..40498,40592..41479)**

atgggtaaagagaagtttcacatcaacattgtggtcattggccacgtcgattctggaaagtcga
ccaccactgggcacttgatctacaagttgggtggtattgacaagcgtgtcattgagaggttcga
gaaggaggctgctgagatgaacaagaggtccttcaagtacgcatgggttttggacaaacttaag
gctgagcgtgagcgtggtatcaccattgacattgctctctggaagttcgagaccaccaagtact
actgcactgtcattgatgctcctggccatcgtgatttcatcaagaacatgatcactggtacctc
ccaggctgattgtgctgtccttatcattgactccaccactggtggttttgaggctggtatctcc
aaggatggtcagacccgtgagcacgctctacttgctttcacccttggtgtcaagcagatgatct
gctgttgtaacaagatggatgccactacccccaagtactccaaggccaggtacgatgaaatcat
caaggaggtgtcttcctacttgaagaaggttggttacaaccccgacaaaatcccatttgtgccc
atctctggatttgagggtgacaacatgattgagaggtccaccaaccttgactggtacaagggac
caactctccttgaggctcttgaccagatcaacgagcccaagaggccgtcagacaagccccttcg
tctcccacttcaggatgtctacaagattggtggtattggaacggtgccagtgggacgtgttgag
actggtatgatcaagcctggtatggttgtgacctttgctcccacaggattgaccactgaggtca
agtctgttgagatgcaccacgagtctcttcttgaggcacttccaggtgacaacgttgggttcaa
tgttaagaatgttgctgtcaaggatcttaagagagggtacgtcgcatccaactccaaggatgac
cctgccaagggtgctgctaacttcacctcccaggtcatcatcatgaaccaccctggtcagattg
gtaacggttacgccccagtcctggattgccacacctctcacattgcagtcaagttctctgagat
cttgaccaagattgacaggcgttctggtaaggagattgagaaggagcctaaattcttgaagaat
ggtgatgctggtatggtgaagatgactccaaccaagcccatggttgtggagaccttctctgagt
acccaccacttggacgtttcgctgtgagggacatgaggcagactgttgcagtcggtgttatcaa
gagtgttgacaagaaggacccaactggagccaaggttaccaaggctgcagtcaagaagggtgcc
aagtga

**FIGURE 3 (continued)**

**Seq id no 20:** ***Arabidopsis thaliana*** **EF-1alpha-A1 AAB07882**

MGKEKFHINIVVIGHVDSGKSTTTGHLIYKLGGIDKRVIERFEKEAAEMNKRSFKYAWVLDKLK
AERERGITIDIALWKFETTKYYCTVIDAPGHRDFIKNMITGTSQADCAVLIIDSTTGGFEAGIS
KDGQTREHALLAFTLGVKQMICCCNKMDATTPKYSKARYDEIIKEVSSYLKKVGYNPDKIPFVP
ISGFEGDNMIERSTNLDWYKGPTLLEALDQINEPKRPSDKPLRLPLQDVYKIGGIGTVPVGRVE
TGMIKPGMVVTFAPTGLTTEVKSVEMHHESLLEALPGDNVGFNVKNVAVKDLKRGYVASNSKDD
PAKGAANFTSQVIIMNHPGQIGNGYAPVLDCHTSHIAVKFSEILTKIDRRSGKEIEKEPKFLKN
GDAGMVKMTPTKPMVVETFSEYPPLGRFAVRDMRQTVAVGVIKSVDKKDPTGAKVTKAAVKKGA
K

**Seq id no 21:** ***Synechocystis*** **TUFA**
atggcacgcgcaaaatttgaacggacgaaagaccacgtaaacattggcaccattggtcacgttg
accacggtaaaaccaccctaacggcggcgatcaccatgaccttggcggaattgggtggtgccaa
agcccggaaatatgaagatattgacgcggctcctgaggaaaaagcccggggcattaccatcaat
actgcccacgttgagtatgaaaccgatagccgtcactatgcccacgtagactgtcctggtcacg
ctgactatgtgaaaaacatgatcaccggtgctgcccagatggacggtgcgattctggtggtttc
cgccgctgatggccccatgccccaaacccgggaacacattcttttggctaagcaagtaggggtt
cccaaactggtggtctttttgaataagaaagacatggtggacgacgaagaactgctggaattgg
tggaactggaagttcgtgaattgctcagcgactacgatttccccggtgatgacattcccatcgt
tgctggctctgccctcaaggcgatcgaaggagaaaaagaatacaaagatgcgattcttgaactc
atgaaagccgttgacgactacatcgacacccctgagcgggaagttgataagcccttcttgatgg
cggtggaagacgtattctccatcactggtcgtggtaccgttgccaccggtcggattgagcgcgg
aaaagttaaagttggcgaagaaatttccatcgtcggcattaaagatacccgtaaagccactgtt
accggtgtggaaatgttccaaaaaacccttgaagagggaatggctggggacaacgtgggtctgc
tcctccggggggattcaaaaagaggacatcgaacggggtatggttttggctaaacccggttccat
cactccccacaccgaatttgaaggggaagtttacgtgctcaagaaagaagaaggtggccgccac
actcctttctttgccaactaccgccctcagttctatgtacggaccactgacgtaaccggtacca
tcaaaagctacaccgctgacgacggcagtgctgtggaaatggttatgcctggggatcgtatcaa
aatgaccgttgagctcattaacccgatcgccattgaacaggggatgcgttttgctatccgcgaa
ggtggtcgtaccatcggcgccggtgttgtttctaagattttgaagtag

**Seq id no 22:** ***Synechocystis*** **TUFA BAA18321**

MARAKFERTKDHVNIGTIGHVDHGKTTLTAAITMTLAELGGAKARKYEDIDAAPEEKARGITIN
TAHVEYETDSRHYAHVDCPGHADYVKNMITGAAQMDGAILVVSAADGPMPQTREHILLAKQVGV
PKLVVFLNKKDMVDDEELLELVELEVRELLSDYDFPGDDIPIVAGSALKAIEGEKEYKDAILEL
MKAVDDYIDTPEREVDKPFLMAVEDVFSITGRGTVATGRIERGKVKVGEEISIVGIKDTRKATV
TGVEMFQKTLEEGMAGDNVGLLLRGIQKEDIERGMVLAKPGSITPHTEFEGEVYVLKKEEGGRH
TPFFANYRPQFYVRTTDVTGTIKSYTADDGSAVEMVMPGDRIKMTVELINPIAIEQGMRFAIRE
GGRTIGAGVVSKILK

**Seq id no 23: Chlamydomonas**

MAMAMRSTFAARVGAKPAVRGARPASRMSCMA

## FIGURE 3 (continued)

**Seq id no 24: Chlamydomonas**

MQVTMKSSAVSGQRVGGARVATRSVRRAQLQV

**Seq id no 25: *Arabidopsis thaliana***

MASLMLSLGSTSLLPREINKDKLKLGTSASNPFLKAKSFSRVTMTVAVKPSR

**Seq id no 26: *Arabidopsis thaliana***

MATQFSASVSLQTSCLATTRISFQKPALISNHGKTNLSFNLRRSIPSRRLSVSC

**Seq id no 27: *Arabidopsis thaliana***

MASIAASASISLQARPRQLAIAASQVKSFSNGRRSSLSFNLRQLPTRLTVSCAAKPETVDKVCA
VVRKQL

**Seq id no 28: *Arabidopsis thaliana***

MASIATSASTSLQARPRQLVIGAKQVKSFSYGSRSNLSFNLRQLPTRLTVYCAAKPETVDKVCA
VVRKQLSLKE

**Seq id no 29: *Oryza sativa***

ggtcagccaatacattgatccgttgccaatcatgcaaagtattttggctgtggccgagtgccgg
aattgataattgtgttctgactaaattaaatgaccagaagtcgctatcttccaatgtatccgaa
acctggattaaacaatcctgttctgttctctagcccctcctgcatggccggattgtttttttga
catgttttcttgactgaggcctgtttgttctaaactttttcttcaaacttttaactttttcatc
acatcagaacttttctacacatataaacttttaacttttccgtcacatcgttccaatttcaatc
aaactttcaattttggcgtgaactaaacacaccctgagtcttttattgctcctccgtacgggtt
ggctggttgagaataggtattttcagagagaaaatctagatattgggaggaacttggcatgaat
ggccactatatttagagcaattctacggtccttgaggaggtaccatgaggtaccaaaattttag
tgtaaattttagtatctcattataactaggtattatgaggtaccaaatttacaatagaaaaaat
agtacttcatggtactttcttaagtaccgtaaaattgctcctatatttaaggggatgtttatat
ctatccatatccataatttgattttgataagaaaaaatgtgagcacaccaagcatgtccatgac
cttgcactcttggctcactcgtcaactgtgaagaacctcaaaaatgctcaatatagctacaggt
gcctgaaaaaataactttaaagttttgaacatcgatttcactaaacaacaattattatctccct
ctgaaagatgatagtttagaactctagaatcattgtcggcggagaaagtaaattattttcccca
aatttccagctatgaaaaaccctcaccaaacaccatcaaacaagagttcaccaaaccgcccat
gcggccatgctgtcacgcaacgcaccgcattgcctgatggccgctcgatgcatgcatgcttccc
cgtgcacatatccgacagacgcgccgtgtcagcgagctcctcgaccgacctgtgtagcccatgc
aagcatccacccccgccacgtacacccccctcctcctccctacgtgtcaccgctctctccaccta
tatatgcccacctggcccctctcctcccatctccacttcacccgatcgcttcttcttcttcttc
gttgcattcatcttgctagc

**FIGURE 3 (continued)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030044972 A1
- US 5811238 A
- US 6395547 A
- US 5565350 A, Kmiec
- US 9303868 W, Zarling
- EP 1198985 A1
- EP 04106984 A
- US 60641657 B


### Non-patent literature cited in the description


- **Bhadula et al.** *Planta,* 2001, vol. 212, 359-366
- **Olsson et al.** *J Biol Chem.,* 07 November 2003, vol. 278 (45), 44439-47
- **Stanzel et al.** *Eur J Biochem.,* 15 January 1994, vol. 219 (1-2), 435-9
- **Zhang et al.** *J. Bacteriol.,* vol. 176, 1184-1187
- **Meinkoth ; Wahl.** *Anal. Biochem.,* 1984, vol. 138, 267-284
- **Sambrook et al.** Molecular Cloning: a laboratory manual. Cold Spring Harbor Laboratory Press, 2001
- Current Protocols in Molecular Biology. John Wiley & Sons, 1989
- **Hayashi et al.** *Science,* 1992, 1350-1353
- **McCallum.** *Nat Biotechnol.,* April 2000, vol. 18 (4), 455-7
- **Stemple.** TILLING-a high-throughput harvest for functional genomics. *Nat. Rev. Genet.,* February 2004, vol. 5 (2), 145-50
- **Castle et al.** *Science,* 2004, vol. 304 (5674), 1151-4
- **Offringa et al.** Extrachromosomal homologous recombination and gene targeting in plant cells after Agrobacterium-mediated transformation. *EMBO J.,* October 1990, vol. 9 (10), 3077-84
- **Terada R ; Urawa H ; Inagaki Y ; Tsugane K ; Iida S.** Efficient gene targeting by homologous recombination in rice. *Nat Biotechnol.,* 2002
- **Iida ; Terada.** A tale of two integrations, transgene and T-DNA: gene targeting by homologous recombination in rice. *Curr Opin Biotechnol.,* April 2004, vol. 15 (2), 132-8
- **Creighton.** Proteins. W.H. Freeman and Company, 1984
- **Needleman ; Wunsch.** *J. Mol. Biol.,* 1970, vol. 48, 443-453
- **Buchman ; Berg.** *Mol. Cell biol.,* 1988, vol. 8, 4395-4405
- **Callis et al.** *Genes Dev.,* 1987, vol. 1, 1183-1200
- The Maize Handbook. Springer, 1994
- **Krens, F.A. et al.** *Nature,* vol. 296, 72-74
- **Negrutiu I. et al.** *Plant Mol. Biol.,* June 1987, vol. 8, 363-373
- **Shillito R.D. et al.** *Bio/Technol,* 1985, vol. 3, 1099-1102
- **Crossway A. et al.** *Mol. Gen Genet,* 1986, vol. 202, 179-185
- **Klein T.M. et al.** *Nature,* 1987, vol. 327, 70
- **Aldemita ; Hodges.** *Planta,* 1996, vol. 199, 612-617
- **Chan et al.** *Plant Mol. Biol.,* 1993, vol. 22 (3), 491-506
- **Hiei et al.** *Plant J.,* 1994, vol. 6 (2), 271-282
- **Ishida et al.** *Nat. Biotechnol.,* June 1996, vol. 14 (6), 745-50
- **Frame et al.** *Plant Physiol.,* May 2002, vol. 129 (1), 13-22
- **Lander et al.** *Genomics,* 1987, vol. 1, 174-181
- **Botstein et al.** *Am. J. Hum. Genet.,* 1980, vol. 32, 314-331
- **Bematzky ; Tanksley.** *Plant Mol. Biol. Reporter,* 1986, vol. 4, 37-41
- **Hoheisel et al.** Non-mammalian Genomic Analysis: A Practical Guide. Academic press, 1996, 319-346
- **Trask.** *Trends Genet.,* 1991, vol. 7, 149-154
- **Laan et al.** *Genome Res.,* 1995, vol. 5, 13-20
- **Kazazian.** *J. Lab. Clin. Med,* 1989, vol. 11, 95-96
- **Sheffield et al.** *Genomics,* 1993, vol. 16, 325-332
- **Landegren et al.** *Science,* 1988, vol. 241, 1077-1080
- **Sokolov.** *Nucleic Acid Res.,* 1990, vol. 18, 3671
- **Walter et al.** *Nat. Genet.,* 1997, vol. 7, 22-28
- **Dear ; Cook.** *Nucleic Acid Res.,* 1989, vol. 17, 6795-6807
- **Sambrook.** Molecular Cloning: a laboratory manual. Cold Spring Harbor Laboratory Press, 2001

- **Ausubel et al.** Current Protocols in Molecular Biology. *Current Protocols,* 1994, vol. 1, 2
- **R.D.D. Croy.** Molecular Biology Labfase. BIOS Scientific Publications Ltd (UK) and Blackwell Scientific Publications (UK), 1993
- **Nagata et al.** *Int. Rev. Cytol.,* 1992, vol. 132, 1-30
- **Vos et al.** *Nucleic Acids Res.,* 1995, vol. 23 (21), 4407-4414
- **Bachem et al.** *Plant J.,* 1996, vol. 9 (5), 745-53
- **Altschul et al.** *Nucleic Acids Res.,* 1997, vol. 25 (17), 3389-3402